# EUROPEAN PATENT SPECIFICATION

(11) **EP 3 518 962 B1**
(45) Date of publication and mention of the grant of the patent: **09.06.2021**
(21) Application number: 17791485.0
(22) Date of filing: 28.09.2017
(51) Int. Cl.: A61K 38/46, A61P 1/16, A61P 3/00

(54) **SEBELIPASE ALFA FOR USE IN LIVER FIBROSIS AND TREATING LYSOSOMAL ACID LIPASE DEFICIENCY IN PATIENTS BASED ON ISHAK FIBROSIS STAGE**
SEBELIPASE ALFA ZUR REDUZIERUNG VON LEBERFIBROSE UND BEHANDLUNG VON MORBUS WOLMAN AUF BASIS DES ISHAK-FIBROSEGRADS
SEBELIPASE ALFA POUR LE RÉDUCTION DE LA FIBROSE HÉPATIQUE ET DU TRAITEMENT D'UN DÉFICIT EN LIPASE ACIDE LYSOSOMALE SUR LA BASE D'UN STADE DE FIBROSE D'ISHAK

(30) Priority: 30.09.2016 US 201662402183 P; 10.11.2016 US 201662420233 P; 08.02.2017 US 201762456511 P
(43) Date of publication of application: 07.08.2019
(73) Proprietor: Alexion Pharmaceuticals, Inc., Boston, MA 02210 (US)
(72) Inventor: QUINN, Anthony, Gloucester MA 01930 (US); GOODMAN, Zachary, Falls Church VA 22042 (US); BURTON, Barbara, Chicago IL 60611 (US); RANKIN, Andrew, Boston MA 02110 (US); FRIEDMAN, Mark, Newton MA 02461 (US); SONI, Paresh, Mystic CT 06355 (US)
(74) Representative: J A Kemp LLP
(86) International application number: PCT/US2017/053961
(87) International publication number: WO 2018/064303

(56) References cited:
- WO-A2-2011/133960
- BARBARA K. BURTON ET AL: "A Phase 3 Trial of Sebelipase Alfa in Lysosomal Acid Lipase Deficiency", NEW ENGLAND JOURNAL OF MEDICINE, THE - NEJM -, vol. 373, no. 11, 10 September 2015 (2015-09-10), pages 1010-1020, XP055440916, US ISSN: 0028-4793, DOI: 10.1056/NEJMoa1501365
- MANISHA BALWANI ET AL: "Clinical effect and safety profile of recombinant human lysosomal acid lipase in patients With cholesteryl ester storage disease", HEPATOLOGY, vol. 58, no. 3, 1 September 2013 (2013-09-01), pages 950-957, XP055399555, ISSN: 0270-9139, DOI: 10.1002/hep.26289
- VALAYANNOPOULOS VASSILI ET AL: "Sebelipase alfa over 52weeks reduces serum transaminases, liver volume and improves serum lipids in patients with lysosomal acid lipase deficiency", JOURNAL OF HEPATOLOGY, vol. 61, no. 5, 2014, pages 1135-1142, XP029077680, ISSN: 0168-8278, DOI: 10.1016/J.JHEP.2014.06.022

### SEQUENCE LISTING

<110> ALEXION PHARMACEUTICALS, INC.
<120> METHODS FOR REDUCING LIVER FIBROSIS AND TREATING LYSOSOMAL ACID LIPASE DEFICIENCY IN PATIENTS BASED ON ISHAK FIBROSIS STAGE
<130> AXJ-221PC
<140>
   <141>
<150> 62/456,511
   <151> 2017-02-08
<150> 62/420,233
   <151> 2016-11-10
<150> 62/402,183
   <151> 2016-09-30
<160> 2
<170> PatentIn version 3.5
<210> 1
   <211> 378
   <212> PRT
   <213> Homo sapiens
<400> 1
   Ser Gly Gly Lys Leu Thr Ala Val Asp Pro Glu Thr Asn Met Asn Val
   1               5                   10                  15
   Ser Glu Ile Ile Ser Tyr Trp Gly Phe Pro Ser Glu Glu Tyr Leu Val
               20                  25                  30
   Glu Thr Glu Asp Gly Tyr Ile Leu Cys Leu Asn Arg Ile Pro His Gly
           35                  40                  45
   Arg Lys Asn His Ser Asp Lys Gly Pro Lys Pro Val Val Phe Leu Gln
       50                  55                  60
   His Gly Leu Leu Ala Asp Ser Ser Asn Trp Val Thr Asn Leu Ala Asn
   65                  70                  75                  80
   Ser Ser Leu Gly Phe Ile Leu Ala Asp Ala Gly Phe Asp Val Trp Met
                   85                  90                  95
   Gly Asn Ser Arg Gly Asn Thr Trp Ser Arg Lys His Lys Thr Leu Ser
               100                 105                 110
   Val Ser Gln Asp Glu Phe Trp Ala Phe Ser Tyr Asp Glu Met Ala Lys
           115                 120                 125
   Tyr Asp Leu Pro Ala Ser Ile Asn Phe Ile Leu Asn Lys Thr Gly Gln
       130                 135                 140
   Glu Gln Val Tyr Tyr Val Gly His Ser Gln Gly Thr Thr Ile Gly Phe
   145                 150                 155                 160
   Ile Ala Phe Ser Gln Ile Pro Glu Leu Ala Lys Arg Ile Lys Met Phe
                   165                 170                 175
   Phe Ala Leu Gly Pro Val Ala Ser Val Ala Phe Cys Thr Ser Pro Met
               180                 185                 190
   Ala Lys Leu Gly Arg Leu Pro Asp His Leu Ile Lys Asp Leu Phe Gly
           195                 200                 205
   Asp Lys Glu Phe Leu Pro Gln Ser Ala Phe Leu Lys Trp Leu Gly Thr
       210                 215                 220
   His Val Cys Thr His Val Ile Leu Lys Glu Leu Cys Gly Asn Leu Cys
   225                 230                 235                 240
   Phe Leu Leu Cys Gly Phe Asn Glu Arg Asn Leu Asn Met Ser Arg Val
                   245                 250                 255
   Asp Val Tyr Thr Thr His Ser Pro Ala Gly Thr Ser Val Gln Asn Met
               260                 265                 270
   Leu His Trp Ser Gln Ala Val Lys Phe Gln Lys Phe Gln Ala Phe Asp
           275                 280                 285
   Trp Gly Ser Ser Ala Lys Asn Tyr Phe His Tyr Asn Gln Ser Tyr Pro
       290                 295                 300
   Pro Thr Tyr Asn Val Lys Asp Met Leu Val Pro Thr Ala Val Trp Ser
   305                 310                 315                 320
   Gly Gly His Asp Trp Leu Ala Asp Val Tyr Asp Val Asn Ile Leu Leu
                   325                 330                 335
   Thr Gln Ile Thr Asn Leu Val Phe His Glu Ser Ile Pro Glu Trp Glu
               340                 345                 350
   His Leu Asp Phe Ile Trp Gly Leu Asp Ala Pro Trp Arg Leu Tyr Asn
           355                 360                 365
   Lys Ile Ile Asn Leu Met Arg Lys Tyr Gln
       370                 375
<210> 2
   <211> 21
   <212> PRT
   <213> Homo sapiens
<400> 2
   Met Lys Met Arg Phe Leu Gly Leu Val Val Cys Leu Val Leu Trp Thr
   1               5                   10                  15
   Leu His Ser Glu Gly
               20

## Description

### RELATED APPLICATIONS

This application claims the benefit of provisional patent applications U.S. Serial No. 62/402183, filed September 30, 2016, U.S. Serial No. 62/420233, filed November 10, 2016 and U.S. Serial No. 62/456511, filed February 8, 2017.

### BACKGROUND OF THE INVENTION

Lysosomal Acid Lipase (LAL) Deficiency (LAL-D) is a rare lysosomal storage disease (LSD) characterized by a failure to breakdown cholesteryl esters (CE) and triglycerides (TAG) in lysosomes due to a deficiency of the enzyme. LAL deficiency resembles other lysosomal storage disorders with the accumulation of substrate in a number of tissues and cell types. In LAL deficiency substrate accumulation is most marked in cells of the reticuloendothelial system including Kupffer cells in the liver, histiocytes in the spleen and in the lamina propria of the small intestine. Reticuloendothelial cells express the macrophage mannose/N-acetyl glucosamine receptor (also known as macrophage mannose receptor, MMR, or CD206), which mediates binding, cell uptake and lysosomal internalization of proteins with GlcNAc or mannose terminated N-glycans, and provides a pathway for the potential correction of the enzyme deficiency in these key cell types.

LAL Deficiency is a multi-system disease that most commonly manifests with gastrointestinal, liver and cardiovascular complications and is associated with significant morbidity and mortality. The clinical effects of LAL deficiency are due to a massive accumulation of lipid material in the lysosomes in a number of tissues and a profound disturbance in cholesterol and lipid homeostatic mechanisms, including substantial increases in hepatic cholesterol synthesis. LAL deficiency presents as at least two phenotypes: Wolman Disease (WD) and Cholesteryl Ester Storage Disease (CESD).

Wolman Disease, named after the physician who first described it, is the most aggressive presentation of LAL deficiency. This phenotype is characterized by gastrointestinal and hepatic manifestations including growth failure, malabsorption, steatorrhea, profound weight loss, lymphadenopathy, splenomegaly, and hepatomegaly. Wolman Disease is rapidly progressive and invariably fatal usually within the first year of life. Case report review indicates that survival beyond 12 months of age is extremely rare for patients who present with growth failure due to severe LAL deficiency in the first year of life. In this most aggressive form, growth failure is the predominant clinical feature and is a key contributor to the early mortality. Hepatic involvement as evidenced by liver enlargement and elevation of transaminases is also common in infants.

The diagnosis of Wolman Disease is established through both physical findings and laboratory analyses. Infants are typically hospitalized within the first two months of life due to diarrhea, persistent vomiting, feeding difficulty, stunted growth, and failure to thrive. Physical findings include abdominal distention with hepatomegaly and splenomegaly, and radiographic examination often reveals calcification of the adrenal glands. Laboratory evaluations typically reveal elevated levels of serum transaminases and absent or markedly reduced endogenous LAL enzyme activity. Elevated blood levels of cholesterol and triglycerides are seen in some patients.

Patients with LAL deficiency can also present later in life with predominant liver and cardiovascular involvement, and this is often called Cholesteryl Ester Storage Disease (CESD). In CESD, the liver is severely affected with marked hepatomegaly, hepatocyte necrosis, elevation of transaminases, cirrhosis, and liver fibrosis. Due to increased levels of CE and TAG, the cardiovascular involvement can be characterized by hyperlipidemia. An accumulation of fatty deposits on the artery walls (atherosclerosis) has been reported in some subjects suffering from CESD. The deposits narrow the arterial lumen and can lead to vessel occlusion increasing the risk of significant cardiovascular events including myocardial infarction and strokes. However, not all subjects suffering from LAL deficiency develop atherosclerosis. For example, Wolman Disease patients are overwhelmed with other symptoms associated with the disease, including enlarged liver and spleen, lymphadenopathy, and the malabsorption by the small intestine, but WD is not generally characterized by atherosclerosis (The Metabolic and Molecular Bases of Inherited Disease (Scriver, C. R., Beaudet, A. L., Sly, W. S. and Valle D., eds) 7th ed., Volume 2 p.2570 McGraw-Hill, 1995). Likewise, not all CESD patients exhibit atherosclerosis See, Di Bisceglie et al., Hepatology 11: 764-772 (1990), Ameis et al., J. Lipid Res. 36: 241-250 (1995). The presentation of CESD is highly variable with some patients going undiagnosed until complications manifest in late adulthood, while others can have liver dysfunction presenting in early childhood. CESD is associated with shortened lifespan and significant ill health. The life expectancy of those with CESD depends on the severity of the associated complications.

In most cases, therapy for LAL deficiencies requires life-long treatment. Therefore, there is a strong need for an effective therapy with a minimized frequency of administration in order to improve the quality of life for patients.

### SUMMARY OF THE INVENTION

The present disclosure provides methods of reducing liver fibrosis in a human patient with a lysosomal acid lipase (LAL) deficiency comprising administering sebelipase alfa to the patient, wherein the patient has been determined to have at least a one point reduction (*e.g.,* a ≥ 1 point reduction or a ≥ 2 point reduction) in Ishak fibrosis stage (score) after administration of sebelipase alfa compared to a baseline Ishak fibrosis stage obtained from the patient prior to administration of sebelipase alfa.

In one embodiment, the method of reducing liver fibrosis in a human patient with a LAL deficiency comprises (a) administering sebelipase alfa to the patient and (b) determining whether the patient has at least a one point reduction (*e.g.,* a ≥ 1 point reduction or a ≥ 2 point reduction) in Ishak fibrosis stage after administration of sebelipase alfa compared to a baseline Ishak fibrosis stage obtained from the patient prior to administration of sebelipase alfa, wherein an at least one point reduction is indicative of reduced liver fibrosis.

In another embodiment, the method of reducing liver fibrosis in a human patient with LAL comprises (a) obtaining a liver biopsy from the patient and assigning a baseline Ishak fibrosis stage based on the biopsy, (b) administering sebelipase alfa to the patient subsequent to the biopsy, (c) obtaining a second liver biopsy from the patient and assigning an Ishak fibrosis stage after administration of sebelipase alfa; and (d) determining whether the patient has at least a one point reduction in Ishak fibrosis stage after administration of sebelipase alfa compared to the baseline Ishak fibrosis stage, wherein an at least one point reduction (*e.g.,* a ≥ 1 point reduction or a ≥ 2 point reduction) is indicative of reduced liver fibrosis.

In another embodiment, the method comprises diagnosing and treating a LAL patient with liver fibrosis by diagnosing the patient with liver fibrosis based on a baseline Ishak fibrosis stage of 1 or greater (*e.g.,* an Ishak fibrosis stage of 1, 2, 3, 4, 5, or 6) prior to administration of sebelipase alfa, wherein an at least one point reduction (*e.g.,* a ≥ 1 point reduction or a ≥ 2 point reduction) in Ishak fibrosis stage after treatment of sebelipase alfa compared to baseline is indicative of reduced liver fibrosis.

Also provided are methods of treating a human patient with a lysosomal acid lipase (LAL) deficiency comprising administering sebelipase alfa to the patient, wherein the patient has been determined to have at least a one point reduction (*e.g.,* a ≥ 1 point reduction or a ≥ 2 point reduction) in Ishak fibrosis stage after administration of sebelipase alfa compared to a baseline Ishak fibrosis stage obtained from the patient prior to administration of sebelipase alfa. In one embodiment, the method comprises (a) administering sebelipase alfa to the patient and (b) determining whether the patient has at least a one point reduction (*e.g.,* a ≥ 1 point reduction or a ≥ 2 point reduction) in Ishak fibrosis stage after administration of sebelipase alfa compared to a baseline Ishak fibrosis stage obtained from the patient prior to administration of sebelipase alfa, wherein an at least one point reduction is indicative of treatment.

In another embodiment, the method of treating a human patient with a LAL deficiency LAL comprises (a) obtaining a liver biopsy from the patient and assigning a baseline Ishak fibrosis stage based on the biopsy, (b) administering sebelipase alfa to the patient subsequent to the biopsy, (c) obtaining a second liver biopsy from the patient and assigning a second Ishak fibrosis stage after administration of sebelipase alfa; and (d) determining whether the patient has at least a one point reduction in Ishak fibrosis stage after administration of sebelipase alfa compared to the baseline Ishak fibrosis stage, wherein an at least one point reduction (*e.g.,* a ≥ 1 point reduction or a ≥ 2 point reduction) is indicative of treatment.

In another embodiment, the method comprises treating a LAL patient by diagnosing the patient with liver fibrosis based on a baseline Ishak fibrosis stage of 1 or greater (*e.g.,* an Ishak fibrosis stage of 1, 2, 3, 4, 5, or 6) prior to administration of sebelipase alfa, wherein an at least one point reduction (*e.g.,* a ≥ 1 point reduction or a ≥ 2 point reduction) in Ishak fibrosis stage after treatment of sebelipase alfa compared to baseline is indicative of treatment.

The methods described herein result in a one point reduction. For example, in one embodiment, the methods result in a reduction from Ishak fibrosis stage 6 to Ishak fibrosis stage 5. In another embodiment, the methods result in a reduction from Ishak fibrosis stage 5 to Ishak fibrosis stage 4. In another embodiment, the methods result in a reduction from Ishak fibrosis stage 4 to Ishak fibrosis stage 3. In another embodiment, the methods result in a reduction from Ishak fibrosis stage 3 to Ishak fibrosis stage 2. In another embodiment, the methods result in a reduction from Ishak fibrosis stage 2 to Ishak fibrosis stage 1. In another embodiment, the methods result in a reduction from Ishak fibrosis stage 0 to Ishak fibrosis stage 0. In another embodiment, the reduction is a ≥ 1 point reduction.

In another embodiment, the reduction is a two point reduction. For example, in one embodiment, the methods result in a reduction from Ishak fibrosis stage 6 to Ishak fibrosis stage 4. In another embodiment, the methods result in a reduction from Ishak fibrosis stage 5 to Ishak fibrosis stage 3. In another embodiment, the methods result in a reduction from Ishak fibrosis stage 4 to Ishak fibrosis stage 2. In another embodiment, the methods result in a reduction from Ishak fibrosis stage 3 to Ishak fibrosis stage 1. In another embodiment, the methods result in a reduction from Ishak fibrosis stage 2 to Ishak fibrosis stage 0. In another embodiment, the reduction is a ≥ 2 point reduction.

In another embodiment, the reduction is a three point reduction. For example, in one embodiment, the methods result in a reduction from Ishak fibrosis stage 6 to Ishak fibrosis stage 3. In another embodiment, the methods result in a reduction from Ishak fibrosis stage 5 to Ishak fibrosis stage 2. In another embodiment, the methods result in a reduction from Ishak fibrosis stage 4 to Ishak fibrosis stage 1. In another embodiment, the methods result in a reduction from Ishak fibrosis stage 3 to Ishak fibrosis stage 0. In another embodiment, the reduction is a ≥ 3 point reduction.

In one embodiment, the at least one point reduction occurs on or by week 20. In another embodiment, the at least one point reduction occurs on or by week 30. In another embodiment, the at least one point reduction occurs on or by week 52. In another embodiment, a ≥ 2 point reduction occurs on or by week 52.

The Ishak fibrosis stage can be assessed by any suitable technique known in the art. In one embodiment, the Ishak fibrosis stage is assessed via liver biopsy. In one embodiment, the patient has cirrhosis at baseline.

Sebelipase alfa can be administered to the patient by any suitable means known in the art. In one embodiment, sebelipase alfa is administered as an intravenous infusion. In one embodiment, sebelipase alfa is infused over at least two hours. In another embodiment, sebelipase alfa is administered to the patient at a dose of 1 mg/kg once every other week. In a particular embodiment wherein sebelipase alfa is administered to the patient at a dose of 1 mg/kg once every other week, sebelipase alfa is administered at a total infusion volume of: (a) 10 mL for a 1 to 10.9 kg patient, (b) 25 mL for a 11 to 24.9 kg patient, (c) 50 mL for a 25 to 49.9 kg patient, (d) 100 mL for a 50 to 99.9 kg patient, or (e) 250 mL for a 100 to 120.9 kg patient. In another embodiment, sebelipase alfa is administered to the patient at a dose of 3 mg/kg once weekly. In another embodiment, sebelipase alfa is administered to the patient at a dose of 0.35 mg/kg once every week or every other week (*e.g.,* to a pediatric patient or in the event of tolerance issues). In a particular embodiment, wherein sebelipase alfa is administered to the patient at a dose of 3 mg/kg once weekly, sebelipase alfa is administered at a total infusion volume of: (a) 25 mL for a 1 to 10.9 kg patient, (b) 50 mL for a 11 to 24.9 kg patient, (c) 100 mL for a 25 to 49.9 kg patient, (d) 250 mL for a 50 to 99.9 kg patient or (e) 500 mL for a 100 to 120.9 kg patient.

In another embodiment, the patient is administered a second therapeutic. The second therapeutic can include, for example, a cholesterol-reducing drug (*e.g*., statin or ezetimibe), an antihistamine (*e.g*., diphenhydramine), or an immunosuppressant.
The effectiveness of the methods described herein can be assessed by any suitable means. In another embodiment, the methods described herein result in a shift toward normal levels of low density lipoprotein cholesterol (LDL-C) (*e.g.,* a decrease in LDL-C levels compared to baseline). In another embodiment, the methods described herein result in at least about a 5%, 10%, 15%, 20%, 25%, 30%, or 35% decrease in LDL-C levels compared to baseline (*e.g*., after 20 or more weeks of treatment with SA). In a particular embodiment, the methods described herein result in at least about a 26%, 27%, 28%, 29%, or 30% decrease in LDL-C levels compared to baseline (*e.g*., after 20, 52, or 76 weeks of treatment with SA).

In another embodiment, the methods described herein result in a shift toward normal levels of high density lipoprotein cholesterol (HDL-C) (*e.g.,* an increase in HDL-C levels compared to baseline). In another embodiment, the methods described herein result in at least about a 5%, 10%, 15%, 20%, 25%, 30%, or 35% increase in HDL-C levels compared to baseline (*e.g.,* after 20 or more weeks of treatment with SA). In a particular embodiment, the methods described herein result in at least about a 18%, 19%, 20%, 21%, 22%, 23%, 24%, or 25% increase in HDL-C levels compared to baseline (*e.g*., after 20, 52, or 76 weeks of treatment with SA).

In another embodiment, the methods described herein result in a shift toward normal levels of non-high density lipoprotein cholesterol (non-HDL-C) (*e.g.,* a reduction in non-HDL-C levels compared to baseline). In another embodiment, the methods described herein result in at least about a 5%, 10%, 15%, 20%, 25%, 30%, or 35% decrease in non-HDL-C levels compared to baseline (*e.g*., after 20 or more weeks of treatment with SA). In a particular embodiment, the methods described herein result in at least about a 25%, 26%, 27%, 28%, 29%, or 30% decrease in non-HDL-C levels compared to baseline (*e.g.,* after 20, 52, or 76 weeks of treatment with SA).

In another embodiment, the methods result in reduction of (ALT), LDL-C, collagen, portal inflammation, lobular inflammation, macrovesicular steatosis, microvesicular steatosis, macrophages and/or overall liver fat content levels compared to baseline.

In another embodiment, the methods described herein result in an improvement in liver function. In another embodiment, the methods described herein are sufficient to normalize liver tests. In another embodiment, the methods described herein result in a shift toward normal serum levels of liver transaminases, such as alanine aminotransferase (ALT) and/or serum aspartate transaminase (AST). In another embodiment, the methods described herein are sufficient to decrease AST and/or ALT. In another embodiment, the methods described herein result in at least about a 35%, 40%, 45%, 50%, 55%, or 60% decrease in ALT levels compared to baseline (*e.g.,* after 20 or more weeks of treatment with SA). In a particular embodiment, the methods described herein result in at least about a 51%, 52%, 53%, 54% 55%, 56%, or 57% decrease in ALT levels compared to baseline (*e.g.,* after 20, 52, or 76 weeks of treatment with SA).

In another embodiment, the methods described herein result in at least about a 35%, 40%, 45%, 50%, 55%, or 60% decrease in AST levels compared to baseline (*e.g.,* after 20 or more weeks of treatment with SA). In a particular embodiment, the methods described herein result in at least about a 42%, 43%, 44%, 45%, 46%, 47%, 48%, 49%, 50%, or 51% decrease in AST levels compared to baseline (*e.g.,* after 20, 52, or 76 weeks of treatment with SA).

In another embodiment, the methods described herein are sufficient to minimize hepatomegaly. In another embodiment, the methods described herein are sufficient to decrease liver size of the patient. In another embodiment, the methods described herein result in a shift toward normal liver volume (*e.g.,* a reduction in liver volume compared to baseline). In another embodiment, the methods described herein result in at least about a 5%, 10%, 15%, or 20% decrease in liver volume compared to baseline (*e.g.,* after 20 or more weeks of treatment with SA). In a particular embodiment, the methods described herein result in at least about a 11%, 12%, or 13% decrease in liver volume compared to baseline (*e.g.,* after 20, 30, 52, or 76 weeks of treatment with SA).

In another embodiment, the methods described herein result in a shift toward normal levels of liver fat content (hepatic fat fraction) (*e.g.,* a reduction in liver fat content compared to baseline). In another embodiment, the methods described herein result in at least about a 10%, 15%, 20%, or 25% decrease in hepatic fat fraction compared to baseline (*e.g.,* after 20 or more weeks of treatment with SA). In a particular embodiment, the methods described herein result in at least about a 21%, 22%, 23%, 24%, 25%, 26%, 27%, 28%, or 29% decrease in hepatic fat fraction compared to baseline (*e.g.,* after 20, 30, 52, or 76 weeks of treatment with SA). In one embodiment, levels of liver fat content are assessed by magnetic resonance imaging (MRI).

In another embodiment, the methods described herein are sufficient to decrease serum ferritin levels. In another embodiment, the methods described herein are sufficient to decrease serum lipid levels, including, for example, cholesteryl ester (CE) and/or triglycerides (TG) levels. In another embodiment, the methods described herein result in a shift toward normal levels of TGs (*e.g.,* a reduction in TG levels compared to baseline). In another embodiment, the methods described herein result in at least about a 5%, 10%, 15%, 20%, 25%, 30%, or 35% decrease in TG levels compared to baseline (*e.g.,* after 20 or more weeks of treatment with SA). In a particular embodiment, the methods described herein result in at least about a 16%, 17%, 18%, 19%, 20%, 21%, 22%, 23%, 24%, or 25% decrease in TG levels compared to baseline (*e.g.,* after 20, 52, or 76 weeks of treatment with SA).

In another embodiment, the method results in reduction of: ALT by about 60% or more, LDL-C by about 40% or more, and/or liver fat content by about 30% or more, compared to baseline.

In another embodiment, the patient is a pediatric patient. In another embodiment, the patient is less than 8 months of age upon starting treatment with SA. In another embodiment, SA is administered to the pediatric patient at a dose of 1 mg/kg weekly or once every other week. In another embodiment, SA is administered to the pediatric patient at a dose of 3 mg/kg or 5 mg/kg once every other week. In another embodiment, SA is administered to the pediatric patient at a dose of 0.35 mg/kg weekly or once every other week. In another embodiment, the methods described herein result in a life expectancy of 40 months or greater (*e.g.,* 41, 42, 43, 44, 45, 46, 47, 48, 49, 50, 51, 52, 53, 54, 55, 56, 57, 58, 59, 60, 61, 62, 63, 64, 65, 66, 67, 68, 69, 70 months, or greater) for a pediatric patient. In another embodiment, the methods described herein result in an improvement in weight gain in a pediatric patient. For example, in one embodiment, the pediatric patient is in the 45% (*e.g.,* 50%, 55%, 60%, 65%, 70%, 75%, 80%, or 85%) or greater percentile on the weight-for-age growth curve after treatment with SA. In another embodiment, the methods described herein result in an improvement in gastrointestinal symptoms (*e.g.,* a decrease in vomiting and diarrhea) in a pediatric patient. In another embodiment, the methods described herein result in normal levels of albumin in a pediatric patient. In another embodiment, the methods described herein result in normal liver function in a pediatric patient. In another embodiment, the methods described herein result in a shift toward normal levels of ALT in a pediatric patient (*e.g.,* a reduction in ALT levels compared to baseline). In another embodiment, the methods described herein result in a shift toward normal levels of AST in a pediatric patient (*e.g.,* a reduction in AST levels compared to baseline). In another embodiment, the methods described herein result in a shift toward normal levels of hemoglobin in a pediatric patient (*e.g.,* an increase in hemoglobin levels compared to baseline). In another embodiment, the methods described herein result in a shift toward normal levels of albumin in a pediatric patient (*e.g.,* an increase in albumin levels compared to baseline). In another embodiments, the methods described herein result in normal development in a pediatric patient.

Also provided are kits for reducing liver fibrosis in a human patient with a lysosomal acid lipase (LAL) deficiency,the kit comprising (a) a dose of sebelipase alfa; and (b) instructions for using sebelipase alfa in any one of the methods described herein.

### BRIEF DESCRIPTION OF THE FIGURES

**Figure 1** depicts the overall study design.
**Figure 2** depicts the frequency of liver biopsy in ARISE.
**Figure 3** depicts the changes in percent hepatic steatosis from Baseline to Week 20 for subjects with paired liver biopsy data (H&E Stain).
**Figure 4** depicts the median change in percent steatosis for subjects with paired liver biopsy data at Baseline and Week 20.
**Figure 5** depicts the change in Ishak Stage in the SA Arm during the double-blind period (20 weeks of SA Exposure [n=16]).
**Figure 6** depicts the change in Ishak Stage in the SA Arm during the open-label period (52 weeks of SA exposure [n=12]).
**Figure 7** depicts the change in Ishak Stage in the PBO Arm during the double-blind period (0 weeks of SA exposure [n=10]).
**Figure 8** depicts the change in Ishak Stage once the PBO Arm started SA in the open-label period (30 weeks of SA Exposure [n=8]).
**Figure 9** sets forth a summary of the ALT, AST, LDL-C, HDL-C, Non-HDL-C, and TG data following 20 and 52 weeks of treatment with SA.
**Figure 10** depicts the mean change in ALT over time by treatment group (i.e., SA/SA and PBO/SA).
**Figure 11** depicts the mean change from baseline in lipid parameters at 76 weeks of treatment with SA.
**Figure 12** depicts the survival statistics for the pediatric LAL-CL03 clinical trial.
**Figure 13** shows the Kaplan-Meier plots of time from birth to death (LAL-CL03 clinical trial) and untreated LAL-D infants with growth failure (LAL--NH01 clinical trial).
**Figure 14** shows the weight-for-age growth curve for patient D.
**Figures 15A****-F** show the weight-for-age growth curve for patient B (Figure 15A), patient C (Figure 15B), patient D (Figure 15C), patient E (Figure 15D), patient F (Figure 15E), and patient G (Figure 15F).
**Figures 16A****-F** show liver and hematology parameters (ALT (Figure 16A), AST (Figure 16B), hemoglobin (Figure 16C), ferritin (Figure 16D), albumin (Figure 16E), and platelets (Figure 16F)) over time.

### DETAILED DESCRIPTION OF THE INVENTION

The present disclosure provides methods of reducing liver fibrosis in a human patient with a lysosomal acid lipase (LAL) deficiency comprising administering sebelipase alfa to the patient, wherein the patient has been determined to have at least a one point reduction (*e.g.,* a ≥ 1 point reduction or a ≥ 2 point reduction) in Ishak fibrosis stage after administration compared to a baseline Ishak fibrosis stage obtained from the patient prior to administration. Also provided are methods of treating a human patient with a lysosomal acid lipase (LAL) deficiency comprising administering sebelipase alfa to the patient, wherein the patient has been determined to have at least a one point reduction (*e.g.,* a ≥ 1 point reduction or a ≥ 2 point reduction) in Ishak fibrosis stage after administration compared to a baseline Ishak fibrosis stage obtained from the patient prior to administration.

### Definitions

For convenience, certain terms employed in the specification, examples, and appended claims are set forth herein to illustrate and define the meaning and scope of the various terms used to describe the present invention.

"LAL" as used herein refers to "lysosomal acid lipase," and the two terms are used interchangeably throughout the specification. The LAL can be a human protein, *i.e.,* human lysosomal acid lipase. The term "SBC-102," as used herein, refers to a recombinant human lysosomal acid lipase. LAL is also referred to in the literature as acid cholesteryl ester hydrolase, cholesteryl esterase, Lipase A, LIPA, and sterol esterase.

LAL catalyzes the hydrolysis of cholesterol esters and triglycerides to free cholesterol, glycerol, and free fatty acids. Thus, "LAL activity" can be measured, for example, by the cleavage of the fluorogenic substrate, 4-methylumbelliferyl oleate (4MUO). Cleavage of 4MUO can be detected, for example, by excitation at about 360 nm and emission at 460 nm of the released flurophore, 4-methylumbelliferone (4MU). Results can be reported in relative fluorescence units (RFU). For example, the amount of substrate cleaved in a 30 minute endpoint assay can be quantified relative to a 4MU standard curve, and one unit (U) of activity can be defined as the amount of enzyme required to cleave 1 micromole of 4MUO per minute at 37 °C. Accordingly, functional fragments or variants of LAL include fragments or variants that have LAL activity, *e.g.,* the ability to hydrolyze cholesterol esters and/or triglycerides.

As used herein "exogenous LAL" refers to LAL that is not naturally produced by a patient. For example, exogenous LAL includes recombinant LAL protein that is administered to a patient, LAL protein that is isolated from a person or animal and administered to a patient, and LAL protein that is produced (*i.e.,* expressed) in a patient as a result of administration of LAL-encoding RNA and/or DNA or another treatment that increases expression of endogenous LAL protein. In one embodiment, the exogenous LAL is sebelipase alfa.

As used herein, sebelipase alfa (KANUMA®) is a recombinant human lysosomal acid lipase (rhLAL). Lysosomal acid lipase is a lysosomal glycoprotein enzyme that catalyzes the hydrolysis of cholesteryl esters to free cholesterol and fatty acids and the hydrolysis of triglycerides to glycerol and free fatty acids. Sebelipase alfa is produced by recombinant DNA technology in the egg white of eggs laid by genetically engineered chickens. Purified sebelipase alfa is a monomeric glycoprotein containing 6 N-linked glycosylation sites and has a molecular mass of approximately 55,000 daltons. The amino acid sequence for sebelipase alfa is the same as the amino acid sequence for human LAL. The specific activity of sebelipase alfa is 195 to 345 units/mg. One unit is the amount of enzyme activity that catalyzes the hydrolysis of 1 micromole of the synthetic substrate 4-methylumbelliferyl oleate per minute at 37°C under specified assay conditions. KANUMA® is supplied as a sterile, preservative-free, non-pyrogenic aqueous solution in single-use vials for intravenous infusion. Each vial contains sebelipase alfa 20 mg/10 mL. Each mL of solution contains sebelipase alfa (2 mg), citric acid monohydrate (1.57 mg), Human Serum Albumin (10 mg), and trisodium citrate dihydrate (13.7 mg) at pH 5.9.

LAL deficiency is an autosomal recessive lysosomal storage disorder characterized by a genetic defect resulting in a marked decrease or loss in activity of the lysosomal acid lipase (LAL) enzyme. The primary site of action of the LAL enzyme is the lysosome, where the enzyme normally causes the breakdown of lipid particles including LDL-c. Deficient LAL enzyme activity results in progressive complications due to the lysosomal accumulation of cholesteryl esters and triglycerides in multiple organs, including the liver, spleen, intestine, and the walls of blood vessels. The resulting lipid accumulation in the liver may lead to increased liver fat content and progression of liver disease,including fibrosis and cirrhosis. Lipid accumulation in the intestinal wall leads to malabsorption and growth failure. In parallel, dyslipidemia due to impaired degradation of lysosomal lipid is common with elevated LDL-c and triglycerides and low HDL-cholesterol (HDL-c).

Sebelipase alfa binds to cell surface receptors via glycans expressed on the protein and is subsequently internalized into lysosomes. Sebelipase alfa catalyzes the lysosomal hydrolysis of cholesteryl esters and triglycerides to free cholesterol, glycerol and free fatty acids.

"Intravenous injection," often medically referred to as IV push or bolus injection, refers to a route of administration in which a syringe is connected to the IV access device and the medication is injected directly, typically rapidly and occasionally up to a period of 15 minutes if it might cause irritation of the vein or a too-rapid effect. Once a medicine has been injected into the fluid stream of the IV tubing, there must be some means of ensuring that it gets from the tubing to the patient. Usually this is accomplished by allowing the fluid stream to flow normally and thereby carry the medicine into the bloodstream. However, in some cases a second fluid injection, sometimes called a "flush," is used following the first injection to facilitate the entering of the medicine into the bloodstream.

"Intravenous infusion" refers to a route of administration in which medication is delivered over an extended period of time. For example, the medication can be delivered to a patient over a period of time between 1 and 8 hours. The medication can also be delivered to a patient over a period of about 1, about 2, about 3, about 4, about 5, about 6, about 7, or about 8 hours. To accomplish an intravenous infusion, an IV gravity drip or an IV pump can be used. IV infusion is typically used when a patient requires medications only at certain times and does not require additional intravenous fluids (*e.g*., water solutions which can contain sodium, chloride, glucose, or any combination thereof) such as those that restore electrolytes, blood sugar, and water loss.

The term "avian" as used herein refers to any species, subspecies or race of organism of the taxonomic class aves, such as, but not limited to chicken, turkey, duck, goose, quail, pheasants, parrots, finches, hawks, crows, and ratites including ostrich, emu and cassowary. The term includes the various known strains of *Gallus gallus,* or chickens (for example, White Leghorn, Brown Leghorn, Barred-Rock, Sussex, New Hampshire, Rhode Island, Australorp, Minorca, Amrox, California Gray), as well as strains of turkeys, pheasants, quails, duck, ostriches, and other poultry commonly bred in commercial quantities. It also includes an individual avian organism in all stages of development, including embryonic and fetal stages.

The term "poultry derived" or "avian derived" refers to a composition or substance produced by or obtained from poultry. "Poultry" refers to avians that can be kept as livestock, including but not limited to, chickens, duck, turkey, quail and ratites. For example, "poultry derived" may refer to chicken derived, turkey derived and/or quail derived.

The term "patient" as used herein refers to any person receiving or who has received or is to receive medical care or treatment, *e.g*., as directed by a medical care provider.

"Therapeutically effective dose" as used herein refers to the dose (*e.g*., amount and/or interval) of drug required to produce an intended therapeutic response. A therapeutically effective dose refers to a dose that, as compared to a corresponding subject who has not received such a dose, results in improved treatment, healing, prevention, or amelioration of a disease, disorder, or side effect, or a decrease in the rate of the occurrence or advancement of a disease or disorder. The term also includes within its scope, doses effective to enhance physiological functions.

The terms "treat," "treating," and "treatment" refer to methods of alleviating, abating, or ameliorating a disease or symptom, preventing an additional symptom, ameliorating or preventing an underlying cause of a symptom, inhibiting a disease or condition, arresting the development of a disease or condition, relieving a disease or condition, causing regression of a disease or condition, relieving a condition caused by the disease or condition, or stopping a symptom of the disease or condition either prophylactically and/or after the symptom has occurred.

As used herein with reference to a particular dose, "kg⁻¹", "per kg", "/kg," and "per kilogram" represent "per kilogram of body weight" of the mammal, and thus the terms can be used interchangeably.

As used herein, a "body surface area (BSA)-based dose" refers to a dose of an agent that is adjusted to the body-surface area (BSA) of the individual patient. A BSA-based dose may be provided as mg/kg body weight. Various calculations have been published to arrive at the BSA without direct measurement, the most widely used of which is the Du Bois formula (see Du Bois D, Du Bois EF (Jun 1916) Archives of Internal Medicine 17 (6): 863-71; and Verbraecken, J. et al. (Apr 2006). Metabolism - Clinical and Experimental 55 (4): 515-24). Other exemplary BSA formulas include the Mosteller formula (Mosteller RD. N Engl J Med., 1987; 317:1098), the Haycock formula (Haycock GB, et al., J Pediatr 1978, 93:62-66), the Gehan and George formula (Gehan EA, George SL, Cancer Chemother Rep 1970, 54:225-235), the Boyd formula (Current, JD (1998), The Internet Journal of Anesthesiology 2 (2); and Boyd, Edith (1935), University of Minnesota. The Institute of Child Welfare, Monograph Series, No. x. London: Oxford University Press), the Fujimoto formula (Fujimoto S, et al., Nippon Eiseigaku Zasshi 1968;5:443-50), the Takahira formula (Fujimoto S, et al., Nippon Eiseigaku Zasshi 1968;5:443-50), and the Schlich formula (Schlich E, et al., Ernährungs Umschau 2010;57:178-183).

As used herein, the terms "fixed dose", "flat dose" and "flat-fixed dose" are used interchangeably and refer to a dose that is administered to a patient without regard for the weight or body surface area (BSA) of the patient. The fixed or flat dose is therefore not provided as a mg/kg dose, but rather as an absolute amount of the agent.

As used herein, the term "polypeptide" is intended to encompass a singular "polypeptide" as well as plural "polypeptides," and refers to a molecule composed of monomers (amino acids) linearly linked by amide bonds (also known as peptide bonds). The term "polypeptide" refers to any chain or chains of two or more amino acids, and does not refer to a specific length of the product. Thus, peptides, dipeptides, tripeptides, oligopeptides, "proteins," "amino acid chains," or any other term used to refer to a chain or chains of two or more amino acids, are included within the definition of "polypeptide," and the term "polypeptide" may be used instead of, or interchangeably with any of these terms. The term "polypeptide" is also intended to refer to the products of post-expression modifications of the polypeptide, including without limitation glycosylation, acetylation, phosphorylation, amidation, derivatization by known protecting/blocking groups, proteolytic cleavage, or modification by non-naturally occurring amino acids. A polypeptide may be derived from a natural biological source or produced by recombinant technology, but is not necessarily translated from a designated nucleic acid sequence. It may be generated in any manner, including by chemical synthesis.

As used herein, the percent homology between two amino acid sequences or two nucleotide sequences is equivalent to the percent identity between the two sequences. The percent identity between the two sequences is a function of the number of identical positions shared by the sequences (i.e., % homology= # of identical positions/total # of positions X 100), taking into account the number of gaps, and the length of each gap, which need to be introduced for optimal alignment of the two sequences. The comparison of sequences and determination of percent identity between two sequences can be accomplished using a mathematical algorithm, as described in the non-limiting examples below.

The percent identity between two amino acid sequences can be determined using the algorithm of E. Meyers and W. Miller (Comput. Appl. Biosci., 4:11-17 (1988)), which has been incorporated into the ALIGN program (version 2.0), using a PAM120 weight residue table, a gap length penalty of 12 and a gap penalty of 4. In addition, the percent identity between two amino acid sequences can be determined using the Needleman and Wunsch (J. Mol, Biol. 48:444-453 (1970)) algorithm which has been incorporated into the GAP program in the GCG software package (available at http://www.gcg.com), using either a Blossom 62 matrix or a PAM250 matrix, and a gap weight of 16, 14, 12, 10, 8, 6, or 4 and a length weight of 1, 2, 3, 4, 5, or 6.

By an "isolated" polypeptide or a fragment, variant, or derivative thereof is intended a polypeptide that is not in its natural milieu. No particular level of purification is required. For example, an isolated polypeptide can be removed from its native or natural environment. Recombinantly produced polypeptides and proteins expressed in host cells are considered isolated as disclosed herein, as are native or recombinant polypeptides which have been separated, fractionated, or partially or substantially purified by any suitable technique.

Other polypeptides disclosed herein are fragments, derivatives, analogs, or variants of the foregoing polypeptides, and any combination thereof. The terms "fragment," "variant," "derivative" and "analog" when referring to any of the polypeptides disclosed herein include any polypeptides which retain at least some of the activity of the corresponding native polypeptide (*e.g.,* LAL polypeptide fragments, variants, derivatives, and analogs that retain the ability to hydrolyze cholesterol esters and/or triglycerides). Fragments of polypeptides include, for example, proteolytic fragments, as well as deletion fragments. Variants of a polypeptide include fragments as described above, and also polypeptides with altered amino acid sequences due to amino acid substitutions, deletions, or insertions. Variants can occur naturally or be non-naturally occurring. Non-naturally occurring variants can be produced using art-known mutagenesis techniques. Variant polypeptides can comprise conservative or non-conservative amino acid substitutions, deletions, or additions. Derivatives are polypeptides which have been altered so as to exhibit additional features not found on the native polypeptide. Examples include fusion proteins. Variant polypeptides can also be referred to herein as "polypeptide analogs." As used herein, a "derivative" of a subject polypeptide can contain one or more residues chemically derivatized by reaction of a functional side group. Also included as "derivatives" are those peptides which contain one or more naturally occurring amino acid derivatives of the twenty standard amino acids. For example, 4-hydroxyproline can be substituted for proline; 5-hydroxylysine can be substituted for lysine; 3-methylhistidine can be substituted for histidine; homoserine can be substituted for serine; and/or ornithine can be substituted for lysine.

The term "polynucleotide" is intended to encompass a singular nucleic acid as well as plural nucleic acids, and refers to an isolated nucleic acid molecule or construct, *e.g*., messenger RNA (mRNA) or plasmid DNA (pDNA). A polynucleotide may comprise a conventional phosphodiester bond or a non-conventional bond (*e.g.,* an amide bond, such as found in peptide nucleic acids (PNA)). The term "nucleic acid" refers to any one or more nucleic acid segments, *e.g.,* DNA or RNA fragments, present in a polynucleotide. By "isolated" nucleic acid or polynucleotide is intended a nucleic acid molecule, DNA or RNA, which has been removed from its native environment. For example, a recombinant polynucleotide encoding LAL contained in a vector is considered isolated for the purposes of the present invention. Further examples of an isolated polynucleotide include recombinant polynucleotides maintained in heterologous host cells or purified (partially or substantially) polynucleotides in solution. Isolated RNA molecules include *in vivo* or *in vitro* RNA transcripts of polynucleotides of the present invention. Isolated polynucleotides or nucleic acids according to the present invention further include such molecules produced synthetically. In addition, a polynucleotide or a nucleic acid can be or can include a regulatory element such as a promoter, ribosome binding site, or a transcription terminator.

As used herein, a "coding region" is a portion of nucleic acid which consists of codons translated into amino acids. Although a "stop codon" (TAG, TGA, or TAA) is not translated into an amino acid, it may be considered to be part of a coding region, but any flanking sequences, for example promoters, ribosome binding sites, transcriptional terminators, introns, and the like, are not part of a coding region. Two or more coding regions of the present invention can be present in a single polynucleotide construct, *e.g*., on a single vector, or in separate polynucleotide constructs, *e.g*., on separate (different) vectors. Furthermore, any vector can contain a single coding region, or can comprise two or more coding regions. In addition, a vector, polynucleotide, or nucleic acid of the invention can encode heterologous coding regions, either fused or unfused to a nucleic acid encoding a LAL polypeptide or fragment, variant, or derivative thereof. Heterologous coding regions include without limitation specialized elements or motifs, such as a secretory signal peptide or a heterologous functional domain.

A variety of transcription control regions are known to those skilled in the art. These include, without limitation, transcription control regions which function in vertebrate cells, such as, but not limited to, promoter and enhancer segments from cytomegaloviruses (the immediate early promoter, in conjunction with intron-A), simian virus 40 (the early promoter), and retroviruses (such as Rous sarcoma virus). Other transcription control regions include those derived from vertebrate genes such as actin, heat shock protein, bovine growth hormone and rabbit β-globin, as well as other sequences capable of controlling gene expression in eukaryotic cells. Additional suitable transcription control regions include tissue-specific promoters and enhancers as well as lymphokine-inducible promoters (*e.g*., promoters inducible by interferons or interleukins).

Similarly, a variety of translation control elements are known to those of ordinary skill in the art. These include, but are not limited to ribosome binding sites, translation initiation and termination codons, and elements derived from picornaviruses (particularly an internal ribosome entry site, or IRES, also referred to as a CITE sequence).

In other embodiments, a polynucleotide of the present invention is RNA, for example, in the form of messenger RNA (mRNA).

Polynucleotide and nucleic acid coding regions of the present invention may be associated with additional coding regions which encode secretory or signal peptides, which direct the secretion of a polypeptide encoded by a polynucleotide of the present invention. According to the signal hypothesis, proteins secreted by mammalian cells have a signal peptide or secretory leader sequence which is cleaved from the mature protein once export of the growing protein chain across the rough endoplasmic reticulum has been initiated. Those of ordinary skill in the art are aware that polypeptides secreted by vertebrate cells generally have a signal peptide fused to the N-terminus of the polypeptide, which is cleaved from the complete or "full length" polypeptide to produce a secreted or "mature" form of the polypeptide. In certain embodiments, the native signal peptide, *e.g.,* the MKMRFLGLVVCLVLWTLHSEG (SEQ ID NO:2) signal peptide of human LAL is used, or a functional derivative of that sequence that retains the ability to direct the secretion of the polypeptide that is operably associated with it. Alternatively, a heterologous signal peptide (*e.g.,* a heterologous mammalian or avian signal peptide), or a functional derivative thereof, may be used. For example, the wild-type leader sequence may be substituted with the leader sequence of human tissue plasminogen activator (TPA) or mouse β-glucuronidase.

"Vector" means a polynucleotide comprised of single strand, double strand, circular, or supercoiled DNA or RNA. A typical vector can be comprised of the following elements operatively linked at appropriate distances for allowing functional gene expression: replication origin, promoter, enhancer, 5' mRNA leader sequence, ribosomal binding site, nucleic acid cassette, termination and polyadenylation sites, and selectable marker sequences. One or more of these elements can be omitted in specific applications. The nucleic acid cassette can include a restriction site for insertion of the nucleic acid sequence to be expressed. In a functional vector the nucleic acid cassette contains the nucleic acid sequence to be expressed including translation initiation and termination sites. An intron optionally can be included in the construct, for example, 5" to the coding sequence. A vector is constructed so that the particular coding sequence is located in the vector with the appropriate regulatory sequences, the positioning and orientation of the coding sequence with respect to the control sequences being such that the coding sequence is transcribed under the "control" of the control or regulatory sequences. Modification of the sequences encoding the particular protein of interest can be desirable to achieve this end. For example, in some cases it can be necessary to modify the sequence so that it can be attached to the control sequences with the appropriate orientation, or to maintain the reading frame. The control sequences and other regulatory sequences can be ligated to the coding sequence prior to insertion into a vector. Alternatively, the coding sequence can be cloned directly into an expression vector which already contains the control sequences and an appropriate restriction site which is in reading frame with and under regulatory control of the control sequences.

The term "expression" as used herein refers to a process by which a gene produces a biochemical, for example, a polypeptide. The process includes any manifestation of the functional presence of the gene within the cell including, without limitation, gene knockdown as well as both transient expression and stable expression. It includes without limitation transcription of the gene into messenger RNA (mRNA), and the translation of such mRNA into polypeptide(s). Expression of a gene produces a "gene product." As used herein, a gene product can be either a nucleic acid, *e.g.,* a messenger RNA produced by transcription of a gene, or a polypeptide which is translated from a transcript. Gene products described herein further include nucleic acids with post transcriptional modifications, *e.g*., polyadenylation, or polypeptides with post translational modifications, *e.g*., methylation, glycosylation, the addition of lipids, association with other protein subunits, proteolytic cleavage, and the like.

As used herein, "host cells" refers to cells that harbor vectors constructed using recombinant DNA techniques and encoding at least one heterologous gene.

As used herein the terms "N-glycan," "oligosaccharide," "oligosaccharide structure," "glycosylation pattern," "glycosylation profile," and "glycosylation structure" have essentially the same meaning and each refer to one or more structures which are formed from sugar residues and are attached to glycosylated proteins.

As used herein, the term "pharmaceutical composition" refers to a mixture of a compound described herein with other chemical components, such as carriers, stabilizers, diluents, dispersing agents, suspending agents, thickening agents, and/or excipients.

### A. Liver Fibrosis and Ishak Fibrosis Stage

The present invention provides methods of reducing liver fibrosis in a human patient with a lysosomal acid lipase (LAL) deficiency comprising administering sebelipase alfa to the patient, wherein the patient has been determined to have at least a one point reduction (*e.g.,* a ≥ 1 point reduction or a ≥ 2 point reduction) in Ishak fibrosis stage after administration compared to a baseline Ishak fibrosis stage obtained from the patient prior to administration. Also provided are methods of treating a human patient with a lysosomal acid lipase (LAL) deficiency comprising administering sebelipase alfa to the patient, wherein the patient has been determined to have at least a one point reduction (*e.g.,* a ≥ 1 point reduction or a ≥ 2 point reduction) in Ishak fibrosis stage after administration compared to a baseline Ishak fibrosis stage obtained from the patient prior to administration.

In one embodiment, the Ishak fibrosis stage is assessed via liver biopsy. Liver biopsy is an important part of the evaluation of patients with a variety of liver diseases (see, *e.g.,* Goodman, Journal of Hepatology 47 (2007) 598-607). Besides establishing the diagnosis, the biopsy is often used to assess the severity of the disease in terms of both grade and stage. The stage in most chronic liver diseases relates to the degree of scarring with the end stage being cirrhosis with its clinical complications. Specifically, the stage of a disease is a measure of how far it has progressed in its natural history, with the end stage resulting in death of the patient or failure of the organ. The grade relates to the severity of the underlying disease process (with features that vary with the pathogenetic mechanisms) and reflects how quickly the disease is progressing to the end stage. In most forms of chronic liver disease, the end stage is cirrhosis with clinical decompensation, whereas earlier stages have lesser degrees of fibrosis or cirrhosis. The grade can be considered to relate to the severity of the underlying liver disease, with features that vary with the type and pattern of injury. Ideally, both grade and stage should predict prognosis and guide therapeutic intervention.

One of the commonly used systems for assessing liver fibrosis is the Ishak staging (or scoring), which is set forth below in Table 1 (see, *e.g.,* Ishak et al., Journal or Hepatology 22 (1995) 696-699) and Table 7 of Example 1 (see, *e.g.,* R A Standish, et al., Gut 2006;55:569-578 . The stages describe the architectural changes associated with different degrees of scar formation and are easy to comprehend. The stages describe the architectural changes associated with different degrees of scar formation and are easy to comprehend. An advantage of this system is its simplicity of staging from 0 to 6 and its stages can readily be translated into other types of assessment stages, as described by Goodman (Journal of Hepatology 47 (2007) 598-607).

**Table 1: Ishak Stage/Score**

| | |
|---|---|
| No fibrosis | 0 |
| Fibrous expansion of some portal areas, with or | 1 |
| without short fibrous septa | |
| Fibrous expansion of most portal areas, with or without short fibrous septa | 2 |
| Fibrous expansion of most portal areas with occasional portal to portal bridging | 3 |
| Fibrous expansion of portal areas with marked bridging (portal to portal as well as portal to central) | 4 |
| Marked bridging (portal-portal and/or portal-central) with occasional nodules (incomplete cirrhosis) | 5 |
| Cirrhosis, probable or definite | 6 |

The methods described herein result in an at least one point reduction in Ishak fibrosis stage (score). For example, in one embodiment, the methods result in a reduction from Ishak fibrosis stage 6 to Ishak fibrosis stage 5. In another embodiment, the methods result in a reduction from Ishak fibrosis stage 5 to Ishak fibrosis stage 4. In another embodiment, the methods result in a reduction from Ishak fibrosis stage 4 to Ishak fibrosis stage 3. In another embodiment, the methods result in a reduction from Ishak fibrosis stage 3 to Ishak fibrosis stage 2. In another embodiment, the methods result in a reduction from Ishak fibrosis stage 2 to Ishak fibrosis stage 1. In another embodiment, the methods result in a reduction from Ishak fibrosis stage 0 to Ishak fibrosis stage 0. In another embodiment, the reduction is a ≥ 1 point reduction.

In another embodiment, the reduction is a two point reduction. For example, in one embodiment, the methods result in a reduction from Ishak fibrosis stage 6 to Ishak fibrosis stage 4. In another embodiment, the methods result in a reduction from Ishak fibrosis stage 5 to Ishak fibrosis stage 3. In another embodiment, the methods result in a reduction from Ishak fibrosis stage 4 to Ishak fibrosis stage 2. In another embodiment, the methods result in a reduction from Ishak fibrosis stage 3 to Ishak fibrosis stage 1. In another embodiment, the methods result in a reduction from Ishak fibrosis stage 2 to Ishak fibrosis stage 0. In another embodiment, the reduction is a ≥ 2 point reduction.

In another embodiment, the reduction is a three point reduction. For example, in one embodiment, the methods result in a reduction from Ishak fibrosis stage 6 to Ishak fibrosis stage 3. In another embodiment, the methods result in a reduction from Ishak fibrosis stage 5 to Ishak fibrosis stage 2. In another embodiment, the methods result in a reduction from Ishak fibrosis stage 4 to Ishak fibrosis stage 1. In another embodiment, the methods result in a reduction from Ishak fibrosis stage 3 to Ishak fibrosis stage 0. In another embodiment, the reduction is a ≥ 3 point reduction.

In one embodiment, the at least one point reduction occurs on or by week 20. In another embodiment, the at least one point reduction occurs on or by week 30. In another embodiment, the at least one point reduction occurs on or by week 52. In another embodiment, a ≥ 2 point reduction occurs on or by week 52.

### B. Patients with Insufficient LAL Activity

Without wishing to limit the disclosure to the treatment of any particular condition or group of conditions, the disclosure includes the treatment of lysosomal acid lipase (LAL) deficiencies in patients. As used herein, a patient with a LAL deficiency is any patient that has insufficient LAL activity. The insufficient LAL activity in the patient can, for example, be the result of low RNA levels, low protein levels, or low protein activity. The insufficient LAL activity can result from a mutation in the LAL coding sequence, a LAL regulatory sequence, or in another gene (*e.g.,* a gene that regulates LAL). Insufficient LAL activity can also be the result of environmental factors.

One embodiment focuses on the treatment of lysosomal storages diseases (LSDs) that result from a deficiency in lysosomal acid lipase, specifically Wolman Disease (WD) and Cholesteryl Ester Storage Disease (CESD). Without wishing to limit the disclosure to any particular theory or mechanism of operation, both WD and CESD can be due to mutations at the LAL locus and result in a massive accumulation of lipid material in the lysosomes in a number of tissues and a profound disturbance in cholesterol and lipid homeostatic mechanisms which can be treated by administration of exogenous LAL (*e.g*., sebelipase alfa) in accordance with the methods of the disclosure. Thus, in one embodiment, the LAL deficiency treated WD. In another embodiment, the LAL deficiency is CESD. In some embodiments, a diagnosis of WD or CESD is based on genetic analysis (*e.g*., identification of a functional mutation in a LAL-encoding sequence). In other embodiments, a diagnosis of WD or CESD is based on clinical findings (*e.g.,* physical examination and/or laboratory tests).

In some embodiments, exogenous LAL (*e.g*., sebelipase alfa) can be used to treat complications in a variety of conditions such as Non-Alcoholic Fatty Liver Disease (NAFLD) and Non-Alcoholic Steatohepatitis (NASH). NAFLD refers to a disease of the liver which has similar histopathology to liver disease that is due to excessive intake of alcohol. It is characterized by macrovesicular steatosis which causes enlargement of the liver. NAFLD can progress into NASH which refers to liver disease that is similar to NAFLD with the addition of inflammation and damage to the liver which can lead to fibrosis and cirrhosis.

In some embodiments, exogenous LAL (*e.g.,* sebelipase alfa) can be used to treat conditions including pancreatitis, for example, chronic pancreatitis and/or acute pancreatitis as well as alcohol induced pancreatic injury such as alcohol induced pancreatitis.

Exogenous LAL (*e.g.,* sebelipase alfa) produced by any useful method can be used to treat diseases due to alcohol induced cell injury including, but not limited to, those alcohol induced cell injuries that result in accumulation of lipid esters in body tissue such as, but not limited to, liver, spleen, gut, and cardiovascular tissue. According to the disclosure, malabsorption can also be treated by administering exogenous LAL (*e.g.,* sebelipase alfa). Exogenous LAL (*e.g.,* sebelipase alfa) is also useful for the treatment of patients with Tangier disease and familial hypoalphalipoproteinemia. Tangier disease/familial hypoalphalipoproteinemia is associated with the accumulation of cholesterol esters in macrophages accompanied by hepatosplenomegaly and/or lymphadenopathy along with low HDL levels which can be treated by the administration of exogenous LAL (*e.g.,* sebelipase alfa). For example, without wishing to limit the disclosure to any particular theory or mechanism of operation, impaired LAL activity can decrease ABCA1 expression and conversely an increased LAL activity obtained by the administration of exogenous LAL to a patient with Tangier disease/familial hypoalphalipoproteinemia will increase ABCA1 expression to overcome the effects of an ABCA1 gene with a reduced functional activity as a result of polymorphism.

In some embodiments, the level of LAL activity in a patient prior to treatment is about 1%, about 2%, about 3%, about 5%, about 10%, about 15%, about 20%, about 30%, about 40%, about 50%, about 60%, about 70%, or about 80% of normal levels of LAL activity. In one embodiment, the level of LAL activity in a patient prior to treatment is about 50% or less of normal levels of LAL activity. In one embodiment, the level of LAL activity in a patient prior to treatment is about 40 % or less of normal levels of LAL activity. In some embodiments, the level of LAL activity in a patient prior to treatment is about 30% or less of normal levels of LAL activity. In some embodiments, the level of LAL activity in a patient prior to treatment is about 30% or less of normal levels of LAL activity. In some embodiments, the level of LAL activity in a patient prior to treatment is about 20% or less of normal levels of LAL activity. In some embodiments, the level of LAL activity in a patient prior to treatment is about 10% or less of normal levels of LAL activity. In some embodiments, the level of LAL activity in a patient prior to treatment is about 5% or less of normal levels of LAL activity. In some embodiments, a patient shows no measurable LAL activity prior to treatment.

In some embodiments, the level of LAL activity is measured in cultured fibroblast obtained from a human patient suffering from LAL deficiency. In some embodiments, the level of LAL activity is measured in lymphocytes (*e.g.,* leukocytes) of a human patient suffering from LAL deficiency. The lymphocytes include, but are not limited to, peripheral blood mononuclear cells (PMBC). Methods for the measurement are described, for example, in Burton et al., (1980) Clinica Chimica Acta 101: 25-32, and in Anderson et al., (1999) Mol. Genet. & Metab., 66: 333-345. LAL deficient patients who are to be treated with exogenous LAL (*e.g.,* sebelipase alfa) can exhibit fibroblast LAL enzymatic activity that is less than about 30, about 20, about 10, about 5, about 4, about 3, about 2 or about 1 pmol/mg/min as measured using triolein as a substrate. LAL deficient patients who are to be treated with exogenous LAL (*e.g.,* sebelipase alfa) can exhibit leukocyte LAL enzymatic activity that is less than about 30, about 20, about 10, about 5, about 4, about 3, about 2 or about 1 pmol/mg/min as measured by triolein as a substrate. LAL deficient patients who are to be treated with exogenous LAL (*e.g.,* sebelipase alfa) can exhibit fibroblast LAL enzymatic activity that is less than about 30, about 20, about 10, about 5, about 4, about 3, about 2 or about 1 pmol/mg/min as measured using cholesteryl oleate as a substrate. LAL deficient patients who are to be treated with exogenous LAL (*e.g.,* sebelipase alfa) can exhibit leukocyte LAL enzymatic activity that is less than about 30, about 20, about 10, about 5, about 4, about 3, about 2 or about 1 pmol/mg/min as measured using cholesteryl oleate as a substrate.

### C. Administration of Exogenous LAL

For the treatment of a condition, generally, the amount of exogenous LAL (*e.g.,* sebelipase alfa) administered can vary depending on known factors such as age, health, and weight of the recipient, type of concurrent treatment, frequency of treatment, and the like. Usually a dosage of active ingredient can be about 0.01 to about 50 mg per kilogram of body weight. In one embodiment, dosage of exogenous LAL in accordance with the invention is about 0.1 to 0.5 mg per kilogram of body weight. In one embodiment, the dose is about 0.1 mg to about 5.0 mg per kilogram. In one embodiment, the dose is about 0.1 mg to about 5.0 mg per kilogram. In one embodiment the dose is about 0.1, about 0.2, about 0.25, about 0.30, about 0.35, about 0.40, about 0.45, about 0.50 mg per kilogram. In one embodiment, the dose is about 1 mg to about 5 mg per kilogram. In one embodiment, the dose is about 1 mg per kilogram. In one embodiment, the dose is about 3 mg per kilogram. For example, 0.1 mg per kilogram of body weight, 0.2 mg per kilogram of body weight, 0.3 mg per kilogram of body weight, 0.4 mg per kilogram of body weight, 0.5 mg per kilogram of body weight, 1 mg per kilogram of body weight, 2 mg per kilogram of body weight, 3 mg per kilogram of body weight, 4 mg per kilogram of body weight, or 5 mg per kilogram of body weight can be administered. In one embodiment, the dose is about 1 mg to about 20 mg per kilogram of body weight.

The invention also includes other dosages when employing a dosing schedule of the invention. For example in accordance with a dosing schedule of the invention, between about 0.1 mg and about 50 mg per kilogram of body weight is administered to a patient.

In some embodiments, about 0.5 to about 50 mg of exogenous LAL (*e.g.,* sebelipase alfa) are administered, *e.g.* to a patient with Wolman disease at the age between 1 month and 24 months. In one embodiment the patient is less than 1 year of age. In another embodiment, the patient is less than 2 years of age. In some embodiments, about 0.1 mg, about 0.2 mg, about 0.3 mg, about 0.4 mg, about 0.5 mg, about 1 mg, about 2 mg, about 3 mg, about 5 mg, about 10 mg, about 15 mg, about 20 mg, about 25 mg, about 30 mg, about 35 mg, about 40 mg, or about 45 mg of exogenous LAL is administered to the patient with Wolman disease. In some embodiments, about 0.5 to about 30 mg, about 0.5 to about 20 mg, about 0.5 to about 10 mg, or about 0.5 to about 5 mg are administered to the patient with Wolman disease. In some embodiments, about 1 to about 30 mg, about 1 to about 20 mg, about 1 to about 10 mg, or about 1 to about 5 mg are administered.

In some embodiments, about 1 mg to about 350 mg of exogenous LAL (*e.g.,* sebelipase alfa) are administered, *e.g.* to a patient diagnosed with CESD. Thus, in some embodiments, about 1, 5, 10, 25, 50, 75, 100, 125, 150, 175, 200, 225, 250, 275, 300, 325, or 350 mg of exogenous LAL (*e.g.,* sebelipase alfa) is administered to the patient with CESD. In some embodiments, about 5 to about 350 mg, about 5 to about 300 mg, about 5 to about 250 mg, or about 5 to about 200 mg are administered to the patient with CESD. In some embodiments, about 10 to about 350 mg, about 10 to about 300, about 10 to about 250, or about 10 to about 200 mg are administered to the patient with CESD.

In one embodiment, sebelipase alfa is administered to the patient at a dose of 1 mg/kg once every other week. In a particular embodiment wherein sebelipase alfa is administered to the patient at a dose of 1 mg/kg once every other week, sebelipase alfa is administered at a total infusion volume of: (a) 10 mL for a 1 to 10.9 kg patient, (b) 25 mL for a 11 to 24.9 kg patient, (c) 50 mL for a 25 to 49.9 kg patient, (d) 100 mL for a 50 to 99.9 kg patient, or (e) 250 mL for a 100 to 120.9 kg patient. In another embodiment, sebelipase alfa is administered to the patient at a dose of 3 mg/kg once weekly. In a particular embodiment, wherein sebelipase alfa is administered to the patient at a dose of 3 mg/kg once weekly, sebelipase alfa is administered at a total infusion volume of: (a) 25 mL for a 1 to 10.9 kg patient, (b) 50 mL for a 11 to 24.9 kg patient, (c) 100 mL for a 25 to 49.9 kg patient, (d) 250 mL for a 50 to 99.9 kg patient or (e) 500 mL for a 100 to 120.9 kg patient. In another embodiment, sebelipase alfa is administered to the patient at a dose of 0.35 mg/kg once every week or every other week (*e.g.,* to a pediatric patient and/or in the event of tolerance issues).

### D. Combination Treatments

The therapeutic proteins disclosed herein can be used in combination with other therapeutic agents. The disclosure provides for a pretreatment procedure to minimize or prevent any potential anaphylactic reactions that can be incurred by administration of exogenous LAL (*e.g.,* sebelipase alfa). In one embodiment, to pretreat a potential anaphylactic reaction, an H-1 receptor antagonist, also known as an antihistamine (*e.g.,* diphenhydramine) is administered to the patient. In one embodiment, the H-1 receptor antagonist is administered in a dose of about 1 mg to about 10 mg per kilogram of body weight. For example, an antihistamine can be administered in a dose of about 5 mg per kilogram. Administration of the antihistamine can be prior to the administration of exogenous LAL (*e.g.,* sebelipase alfa) in accordance with the invention. In one embodiment, the H-1 receptor antagonist is administered about 10 to about 90 minutes, for example, about 30 to about 60 minutes prior to the administration of exogenous LAL (*e.g.,* sebelipase alfa). The H-1 receptor antagonist can be administered using an ambulatory system connected to a vascular access port. In one embodiment, the antihistamine is administered about 90 minutes prior to the administration of exogenous LAL. In one embodiment, the antihistamine is administered between about 10 and about 60 minutes prior to the administration of exogenous LAL (*e.g.,* sebelipase alfa). In another embodiment, the antihistamine is administered between about 20 and about 40 minutes prior to administering exogenous LAL (*e.g.,* sebelipase alfa). For example, the antihistamine can be administered 20, 25, 30, 35, or 40 minutes prior to the administration of exogenous LAL (*e.g.,* sebelipase alfa). In one embodiment, the antihistamine administered is diphenhydramine. Any useful antihistamine can be used. Such antihistamines include, without limitation, clemastine, doxylamine, loratidine, desloratidine, fexofenadine, pheniramine, cetirizine, ebastine, promethazine, chlorpheniramine, levocetirizine, olopatadine, quetiapine, meclizine, dimenhydrinate, embramine, dimethidene, and dexchloropheniramine.

In one embodiment, the antihistamine is administered in a dose of between about 0.1 mg and about 10 mg per kilogram of body weight. In one embodiment, the antihistamine is administered in a dose between about 1 mg and about 5 mg per kilogram of body weight. For example the dose can be 1 mg, 2 mg, 3 mg, 4 mg, or 5 mg per kilogram of body weight. The antihistamine can be administered by any useful method. In one embodiment, the antihistamine is administered intravenously. In another embodiment, the antihistamine is administered in pharmaceutically acceptable capsules.

In another embodiment, with reference to intravenous infusion, the potential for anaphylactic reactions can be reduced by administering the infusions using a ramp-up protocol. In this context, a ramp-up protocol refers to slowly increasing the rate of the infusion over the course of the infusion in order to desensitize the patient to the infusion of the medication.

Immunosuppresants such as, but not limited to, antihistamines, corticosteroids, sirolimus, voclosporin, ciclosporin, methotrexate, IL-2 receptor directed antibodies, T-cell receptor directed antibodies, TNF-alpha directed antibodies or fusion proteins (*e.g.,* infliximab, etanercept, or adalimumab), CTLA-4-Ig (*e.g.,* abatacept), anti-OX-40 antibodies can also be administered before, during, or after exogenous LAL administration, for example, if an anaphylactic reaction or adverse immune response is expected or experienced by a patient.

The disclosure also encompasses therapy involving administration of exogenous LAL-containing compositions in combination with one or more cholesterol lowering agents (e.g., HMG-CoA reductase inhibitors). Non-limiting examples of such agents include: atorvastatin (Lipitor® and Torvast®), fluvastatin (Lescol®), lovastatin (Mevacor®, Altocor®, Altoprev®), pitavastatin (Livalo®, Pitava®), pravastatin (Pravachol®, Selektine®, Lipostat®), rosuvastatin (Crestor®), and simvastatin (Zocor®, Lipex®).

### E. Effects of Exogenous LAL

The present invention provides for a correction or normalization of disease-related symptoms following treatment. The clinical progression (*i.e.,* improvement of the condition) in response to exogenous LAL (*e.g.,* sebelipase alfa) can be monitored by any useful method or procedure.

In some embodiments, administration of exogenous LAL (*e.g.,* sebelipase alfa) is sufficient to achieve a Cmax of about 200 ng/mL to about 1,500 ng/mL. In some embodiments, administration of exogenous LAL is sufficient to achieve a Cmax of about 200 ng/mL to about 1,000 ng/mL. In some embodiments, administration of exogenous LAL is sufficient to achieve a Cmax of about 200 ng/mL to about 800 ng/mL. In some embodiments, administration of exogenous LAL is sufficient to achieve a Cmax of about 200 ng/mL, about 300 ng/mL, about 400 ng/mL, about 500 ng/mL, about 600 ng/mL, about 700 ng/mL, about 800 ng/mL, about 900 ng/mL, about 1,000 ng/mL, about 1,250 ng/mL, or about 1,500 ng/mL. In some embodiments, Cmax is reached during infusion.

In some embodiments, administration of exogenous LAL (*e.g.,* sebelipase alfa) is sufficient to achieve a LAL half-life (t_{1/2}) that is less than 40 minutes. In some embodiments, administration of exogenous LAL is sufficient to achieve a LAL half-life (t_{1/2}) that is less than 30 minutes. In some embodiments, administration of exogenous LAL is sufficient to achieve a LAL half-life (t_{1/2}) that is less than 20 minutes. In some embodiments, administration of exogenous LAL is sufficient to achieve a LAL half-life (t_{1/2}) that is less than 15 minutes. In some embodiments, administration of exogenous LAL is sufficient to achieve a LAL half-life (t_{1/2}) that is less than 10 minutes. In some embodiments, administration of exogenous LAL is sufficient to achieve a LAL half-life (t_{1/2}) of about 6, 7, 8, 9, 10, 11, 12, 13, 14, 15, 16, 17, 18, 19, 20, 25, 30, 35, or 40 minutes.

In some embodiments, exogenous LAL (*e.g.,* sebelipase alfa) increases LAL activity in a patient. LAL activity can be increased, for example, in liver, spleen, lymph nodes, aorta, peripheral blood leukocytes, and/or skin fibroblasts. In some embodiments, LAL activity is measured in extracts of lymphocytes isolated from blood samples.

Exogenous LAL (*e.g.,* sebelipase alfa) can increase LAL activity to at least about 1.5, about 2, about 2.5, about 3, about 4, about 5, about 6, about 7, about 8, about 9, about 10, about 15, or about 20 times the activity prior to LAL administration. Exogenous LAL can increase LAL activity to at least about 10, about 11, about 12, about 13, about 14, about 15, about 16, about 17, about 18, about 19, about 20 times the activity prior to LAL administration. LAL activity can be assessed using methods known in the art including, for example, assays using cholesteryl [1-¹⁴C]oleate, triolein (glycerol tri [l-¹⁴C]oleate), *p*-nitropheny myristate or 4-MUO (4-methylumbelliferyl oleate) substrates.

In one embodiment, organ and tissue volume and characterization is employed to determine the improvement of the condition following administration of exogenous LAL (*e.g.,* sebelipase alfa) in accordance with the invention.

In one embodiment, clinical progression in liver function/injury following administration of exogenous LAL (*e.g.,* sebelipase alfa) is monitored by the quantification of blood transaminases such as aspartic acid aminotransferase (AST) and/or alanine transaminase (ALT), and/or other biomarkers, such as albumin, alkaline phosphatase, and bilirubin (direct and total), over time.

In one embodiment, clinical progression is monitored using imaging technology. For example, and without limitation, the imaging technology used can be ultrasound, CT scanning, magnetic resonance imaging, and nuclear magnetic resonance spectroscopy.

In some embodiments, administration of exogenous LAL (*e.g.,* sebelipase alfa) with the doses described herein is sufficient to restore growth and/or increase body weight in human patients. Administration of exogenous LAL can also increase the rate of growth (i.e., body weight increase) in an infant or child patient suffering from early onset LAL deficiency. For example, administration of exogenous LAL can increase the rate of body weight increase by at least about 10%, about 20%, about 30%, about 40%, about 50%, about 60%, about 70%, about 80%, about 90%, about 100%, about 200%, about 300%, about 400%, or about 500% of the growth rate/velocity seen prior to the administration. In some embodiments, administration of exogenous LAL restores normal growth rate in a child patient suffering from early onset LAL deficiency (*e.g.,* Wolman Disease) whose age is between about 1 month and about 24 months. "Normal" in this context refers to normal growth rate for the patient being treated as determined by a practitioner of ordinary skill in the art of medical sciences.

In one embodiment, the methods described herein result in a shift toward normal levels of alanine aminotransferase (ALT), low density lipoprotein cholesterol (LDL-C), collagen, and/or liver fat content. In another embodiment, the methods result in reduction of alanine aminotransferase (ALT), low density lipoprotein cholesterol (LDL-C), collagen, portal inflammation, lobular inflammation, macrovesicular steatosis, microvesicular steatosis, macrophages and/or overall liver fat content levels compared to baseline. In another embodiment, the method results in reduction of: alanine aminotransferase (ALT) by about 60% or more, low density lipoprotein cholesterol (LDL-C) by about 40% or more, and/or liver fat content by about 30% or more, compared to baseline. In one embodiment, levels are assessed by magnetic resonance imaging (MRI).

In another embodiment, the methods described herein result in a shift toward normal levels of low density lipoprotein cholesterol (LDL-C) (*e.g.,* a decrease in LDL-C levels compared to baseline). In another embodiment, the methods described herein result in at least about a 5%, 10%, 15%, 20%, 25%, 30%, or 35% decrease in LDL-C levels compared to baseline (*e.g.,* after 20 or more weeks of treatment with SA). In a particular embodiment, the methods described herein result in at least about a 26%, 27%, 28%, 29%, or 30% decrease in LDL-C levels compared to baseline (*e.g.,* after 20, 52, or 76 weeks of treatment with SA).

In another embodiment, the methods described herein result in a shift toward normal levels of high density lipoprotein cholesterol (HDL-C) (*e.g.,* an increase in HDL-C levels compared to baseline). In another embodiment, the methods described herein result in at least about a 5%, 10%, 15%, 20%, 25%, 30%, or 35% increase in HDL-C levels compared to baseline (*e.g.,* after 20 or more weeks of treatment with SA). In a particular embodiment, the methods described herein result in at least about a 18%, 19%, 20%, 21%, 22%, 23%, 24%, or 25% increase in HDL-C levels compared to baseline (*e.g.,* after 20, 52, or 76 weeks of treatment with SA).

In another embodiment, the methods described herein result in a shift toward normal levels of non-high density lipoprotein cholesterol (non-HDL-C) (*e.g.,* a reduction in non-HDL-C levels compared to baseline). In another embodiment, the methods described herein result in at least about a 5%, 10%, 15%, 20%, 25%, 30%, or 35% decrease in non-HDL-C levels compared to baseline (*e.g.,* after 20 or more weeks of treatment with SA). In a particular embodiment, the methods described herein result in at least about a 25%, 26%, 27%, 28%, 29%, or 30% decrease in non-HDL-C levels compared to baseline (*e.g.,* after 20, 52, or 76 weeks of treatment with SA).

In another embodiment, the methods result in reduction of (ALT), LDL-C, collagen, portal inflammation, lobular inflammation, macrovesicular steatosis, microvesicular steatosis, macrophages and/or overall liver fat content levels compared to baseline.

In one embodiment, for example with reference to WD and CESD or other LAL deficiencies, hepatomegaly is reversed significantly with liver size returning to a size of which is within about 1% to about 60% larger than that of normal. "Normal" in this context refers to a liver of normal size for the patient being treated as determined by a practitioner of ordinary skill in the art of medical sciences. In one embodiment, the methods described herein are sufficient to minimize hepatomegaly. In another embodiment, the methods described herein are sufficient to decrease liver size of the patient. In another embodiment, the methods described herein result in a shift toward normal liver volume (*e.g.,* a reduction in liver volume compared to baseline). In one embodiment, liver size is reduced to between about 1% and about 50% greater than normal. In another embodiment, liver size is reduced to between about 1% and about 40% greater than normal. In one embodiment, liver size is reduced to between about 1% and about 30% greater than normal. In another embodiment, liver size is reduced to between about 1% and about 20% greater than normal. In another embodiment, liver size is reduced to between about 10% and about 20% greater than normal. For example, the liver can be 10%, 11%, 12% 13% 14%, 15%, 16%, 17%, 18%, 19%, or 20% larger than the normal size. In still another embodiment, liver size is reduced to between about 0% and about 10% greater than normal. For example, the liver can be 0%, 1%, 2%, 3%, 4%, 5%, 6%, 7%, 8%, 9%, or 10% larger than the normal size of the liver. In another embodiment, the methods described herein result in at least about a 5%, 10%, 15%, or 20% decrease in liver volume compared to baseline (*e.g.,* after 20 or more weeks of treatment with SA). In a particular embodiment, the methods described herein result in at least about a 11%, 12%, or 13% decrease in liver volume compared to baseline (*e.g.,* after 20, 30, 52, or 76 weeks of treatment with SA).

In another embodiment, the methods described herein result in a shift toward normal levels of liver fat content (hepatic fat fraction) (*e.g.,* a reduction in liver fat content compared to baseline). In another embodiment, the methods described herein result in at least about a 10%, 15%, 20%, or 25% decrease in hepatic fat fraction compared to baseline (*e.g.,* after 20 or more weeks of treatment with SA). In a particular embodiment, the methods described herein result in at least about a 21%, 22%, 23%, 24%, 25%, 26%, 27%, 28%, or 29% decrease in hepatic fat fraction compared to baseline (*e.g.,* after 20, 30, 52, or 76 weeks of treatment with SA). In one embodiment, levels of liver fat content are assessed by magnetic resonance imaging (MRI).

In another embodiment, the methods described herein are sufficient to decrease serum ferritin levels. In another embodiment, the methods described herein are sufficient to decrease serum lipid levels, including, for example, cholesteryl ester (CE) and/or triglycerides (TG) levels. In another embodiment, the methods described herein result in a shift toward normal levels of TGs (*e.g.,* a reduction in TG levels compared to baseline). In another embodiment, the methods described herein result in at least about a 5%, 10%, 15%, 20%, 25%, 30%, or 35% decrease in TG levels compared to baseline (*e.g.,* after 20 or more weeks of treatment with SA). In a particular embodiment, the methods described herein result in at least about a 16%, 17%, 18%, 19%, 20%, 21%, 22%, 23%, 24%, or 25% decrease in TG levels compared to baseline (*e.g.,* after 20, 52, or 76 weeks of treatment with SA).

Treatment with exogenous LAL (*e.g.,* sebelipase alfa) can also improve liver function. Thus, in some embodiments, treatment with exogenous LAL is sufficient to restore normal liver function and/or normalize liver tests. In another embodiment, the methods described herein result in a shift toward normal serum levels of liver transaminases, such as alanine aminotransferase (ALT) and/or serum aspartate transaminase (AST). In another embodiment, the methods described herein are sufficient to decrease AST and/or ALT. In some embodiments, treatment with exogenous LAL is sufficient to decrease serum levels of liver transaminases, *e.g*., by at least about 20%, about 30%, about 40%, about 50%, about 60%, about 70%, about 80%, and/or to at least about 90%. In one embodiment, treatment with exogenous LAL is sufficient to decrease serum levels of liver transaminases by at least about 40%. In one embodiment, treatment with exogenous LAL is sufficient to decrease serum levels of liver transaminases by at least about 50%. In one embodiment, treatment with exogenous LAL is sufficient to decrease serum levels of liver transaminases by at least about 60%. In one embodiment, treatment with exogenous LAL is sufficient to decrease serum levels of liver transaminases by at least about 70%. In one embodiment, treatment with exogenous LAL is sufficient to decrease serum levels of liver transaminases by at least about 80%. In one embodiment, treatment with exogenous LAL is sufficient to decrease serum levels of liver transaminases by at least about 90%.

In some embodiments, the liver transaminase is alanine aminotransferase (ALT). In one embodiment, administration of exogenous LAL (*e.g.,* sebelipase alfa) is sufficient to reduce serum ALT. For example, administration of exogenous LAL can reduce serum ALT, *e.g.,* by at least about 50%, 60%, 70%, 80% or 90%. Serum ALT level can serve an indication of liver injury. Thus, the present invention also contemplates methods of reducing liver injury in a human patient suffering from LAL deficiency by administering an effective amount of exogenous LAL to reduce serum ALT. In another embodiment, the methods described herein result in at least about a 35%, 40%, 45%, 50%, 55%, or 60% decrease in ALT levels compared to baseline (*e.g.,* after 20 or more weeks of treatment with SA). In a particular embodiment, the methods described herein result in at least about a 51%, 52%, 53%, 54% 55%, 56%, or 57% decrease in ALT levels compared to baseline (*e.g.,* after 20, 52, or 76 weeks of treatment with SA).

In some embodiments, the liver transaminase is serum aspartate transaminase (AST). In one embodiment, administration of exogenous LAL (*e.g.,* sebelipase alfa) is sufficient to reduce serum AST. For example, administration of exogenous LAL can reduce serum AST, *e.g.,* by at least about 50%, 60%, 70%, 80% or 90%. Serum AST level can serve an indication of liver injury. Accordingly, the present disclosure also contemplates a method of reducing liver injury in a patient suffering from LAL deficiency by administering an effective amount of exogenous LAL to reduce serum AST. In another embodiment, the methods described herein result in at least about a 35%, 40%, 45%, 50%, 55%, or 60% decrease in AST levels compared to baseline (*e.g.,* after 20 or more weeks of treatment with SA). In a particular embodiment, the methods described herein result in at least about a 42%, 43%, 44%, 45%, 46%, 47%, 48%, 49%, 50%, or 51% decrease in AST levels compared to baseline (*e.g.,* after 20, 52, or 76 weeks of treatment with SA).

In some embodiments, treatment with exogenous LAL (*e.g.,* sebelipase alfa) can decrease serum ferritin levels. Thus, in some embodiments, treatment with exogenous LAL is sufficient to decrease serum ferritin, *e.g.,* by at least about 20%, 30%, 40%, 50%, 60%, 70%, 80%, 90%, or 95% as compared to pretreatment levels. In one embodiment, treatment with exogenous LAL is sufficient to decrease serum levels of ferritin by at least 50%. In yet another embodiment, treatment with exogenous LAL is sufficient to decrease serum levels of ferritin by at least about 60%. In one embodiment, treatment with exogenous LAL is sufficient to decrease serum levels of ferritin by at least about 70%. In one embodiment, treatment with exogenous LAL is sufficient to decrease serum levels of ferritin by at least about 80%. In one embodiment, treatment with exogenous LAL is sufficient to decrease serum levels of ferritin by at least about 90%. In one embodiment, treatment with exogenous LAL is sufficient to decrease serum levels of ferritin by at least about 95%.

In one embodiment, for example, with reference to Wolman Disease and CESD or other LAL deficiencies, splenomegaly is reversed significantly with spleen size returning to a size of which is within about 1% to about 60% larger than that of normal. "Normal" in this context refers to a spleen of normal size for the patient being studied as determined by a practitioner of ordinary skill in the art of medical sciences. In one embodiment, spleen size is reduced to between about 1% and about 50% greater than normal. In another embodiment, spleen size is reduced to between about 1% and about 40% greater than normal. In one embodiment, spleen size is reduced to between about 1% and about 30% greater than normal. In another embodiment, spleen size is reduced to between about 1% and about 20% greater than normal. In another embodiment, spleen size is reduced to between about 10% and about 20% greater than normal. For example, the spleen can be 10%, 11%, 12% 13% 14%, 15%, 16%, 17%, 18%, 19%, or 20% larger than the normal size. In still another embodiment, spleen size is reduced to between about 0% and about 10% greater than normal. For example, the spleen can be 0%, 1%, 2%, 3%, 4%, 5%, 6%, 7%, 8%, 9%, or 10% larger than the normal size of the spleen.

In one embodiment, administration of exogenous LAL (*e.g.,* sebelipase alfa) is sufficient to decrease lymphadenopathy (i.e., enlarged lymph nodes). Thus, in some embodiments, lymph nodes are reduced to about a size of which is within about 1% to about 60% larger than that of normal. "Normal" in this context refers to lymph nodes of normal size for the patient being studied as determined by a practitioner of ordinary skill in the art of medical sciences. In one embodiment, lymph node size is reduced to about 1% to about 50% greater than normal. In another embodiment, lymph node size is reduced to about 1% to about 40% greater than normal. In one embodiment, lymph node size is reduced to about 1% to about 30% greater than normal. In another embodiment, lymph node size is reduced to about 1% to about 20% greater than normal. In another embodiment, lymph node size is reduced to about 10% to about 20% greater than normal. For example, the lymph nodes can be 10%, 11%, 12% 13% 14%, 15%, 16%, 17%, 18%, 19%, or 20% larger than the normal size. In still another embodiment, lymph node size is reduced to about 0% to about 10% greater than normal. For example, the lymph node can be 0%, 1%, 2%, 3%, 4%, 5%, 6%, 7%, 8%, 9%, or 10% larger than the normal size of the lymph nodes. In another embodiment, lipid analysis is performed to monitor improvement of the condition. For example, lipid analysis can be done to evaluate the therapeutic effect of the exogenous LAL (*e.g.,* sebelipase alfa). The lipid analysis can be conducted on a tissue sample of a patient (*e.g.,* a blood sample, liver biopsy sample) by any useful method such as, but not limited to high-performance liquid chromatography, gas chromatography, mass spectroscopy, or thin-layer chromatography, or any combination thereof as deemed appropriate by one skilled in the art. In one embodiment, lipid analyses performed in accordance with the disclosure, demonstrate the levels of total cholesterol, triglycerides, low-density lipoproteins, high-density lipoproteins and/or cholestryl ester.

In one embodiment, for example, with reference to Wolman Disease and CESD or other LAL deficiencies, lipid analysis of a patient treated in accordance with the invention shows a normalization of lipid concentrations in the liver, spleen, intestine, lymph nodes, and/or aorta as can determined by a practitioner of ordinary skill in the field of medical sciences.

Lipid levels can be assessed using plasma lipid analyses or tissue lipid analysis. In plasma lipid analysis, blood plasma can be collected, and total plasma free cholesterol levels can be measured using, for example colormetric assays with a COD-PAP kit (Wako Chemicals), total plasma triglycerides can be measured using, for example, a Triglycerides/GB kit (Boehringer Mannheim), and/or total plasma cholesterol can be determined using a Cholesterol/HP kit (Boehringer Mannheim). In tissue lipid analysis, lipids can be extracted, for example, from liver, spleen, and/or small intestine samples (*e.g.,* using the Folch method provided in Folch et al. J. Biol. Chem 226: 497-505 (1957)). Total tissue cholesterol concentrations can be measured, for example, using O-phthalaldehyde.

In some embodiments, administration of exogenous LAL (*e.g.,* sebelipase alfa) is sufficient to increase nutrient absorption. In one embodiment, administration of exogenous LAL increases nutrient absorption as measured by levels of serum alpha tocopherol, 25OH vitamin D, serum retinol, didehydroretinol, or transthyretin.

In some embodiments, for example with reference to WD and CESD or other LAL deficiencies, administration of exogenous LAL (*e.g.,* sebelipase alfa) is sufficient to increase serum hemoglobin levels (Hb). In one embodiment, the hemoglobin level is increased at least about 10% or about 20% as compared to that observed prior to administration with exogenous LAL.

In some embodiments, the exogenous LAL (*e.g.,* sebelipase alfa) can be administered using methods to minimize side effects. For example, the administration of exogenous LAL can minimize immune responses to the exogenous LAL.

In another embodiment, the patient is a pediatric patient. In another embodiment, the patient is less than 8 months of age upon starting treatment with SA. In another embodiment, SA is administered to the pediatric patient at a dose of 1 mg/kg weekly or once every other week. In another embodiment, SA is administered to the pediatric patient at a dose of 3 mg/kg or 5 mg/kg once every other week. In another embodiment, sebelipase alfa is administered to the patient at a dose of 0.35 mg/kg once every week or every other week. In another embodiment, the methods described herein result in a life expectancy of 40 months or greater (*e.g.,* 41, 42, 43, 44, 45, 46, 47, 48, 49, 50, 51, 52, 53, 54, 55, 56, 57, 58, 59, 60, 61, 62, 63, 64, 65, 66, 67, 68, 69, 70 months, or greater) for a pediatric patient. In another embodiment, the methods described herein result in an improvement in weight gain in a pediatric patient. For example, in one embodiment, the pediatric patient is in the 45% (*e.g.,* 50%, 55%, 60%, 65%, 70%, 75%, 80%, or 85%) or greater percentile on the weight-for-age growth curve after treatment with SA. In another embodiment, the methods described herein result in an improvement in gastrointestinal symptoms (*e.g.,* a decrease in vomiting and diarrhea) in a pediatric patient. In another embodiment, the methods described herein result in normal levels of albumin in a pediatric patient. In another embodiment, the methods described herein result in normal liver function in a pediatric patient. In another embodiment, the methods described herein result in a shift toward normal levels of ALT in a pediatric patient (*e.g.,* a reduction in ALT levels compared to baseline). In another embodiment, the methods described herein result in a shift toward normal levels of AST in a pediatric patient (*e.g.,* a reduction in AST levels compared to baseline). In another embodiment, the methods described herein result in a shift toward normal levels of hemoglobin in a pediatric patient (*e.g.,* an increase in hemoglobin levels compared to baseline). In another embodiment, the methods described herein result in a shift toward normal levels of albumin in a pediatric patient (*e.g.,* an increase in albumin levels compared to baseline). In another embodiments, the methods described herein result in normal development in a pediatric patient.

### F. LAL and Pharmaceutical Compositions Comprising Exogenous LAL

The present disclosure encompasses treating any of the LAL deficiency-related conditions described herein and other conditions not previously mentioned, but which would benefit from the treatment. Exogenous LAL (*e.g.,* sebelipase alfa) employed in accordance with the invention includes recombinant LAL which can be produced in any useful protein expression system including, without limitation, cell culture (*e.g.,* CHO cells, COS cells), bacteria such as *E. coli,* transgenic animals such as mammals and avians (*e.g.,* chickens, duck, and turkey) and in plant systems (*e.g.,* duck weed and tobacco plants). One aspect of the invention relates to recombinant LAL produced in accordance with US Patent No. 7,524,626, issued October 3, 2006; US Patent Application Nos. 11/973,853, filed October 10, 2007; 11/978,360, filed October 29, 2007; and 12/319,396, filed January 7, 2009. One aspect of the invention relates to recombinant LAL produced as described in Du et al., (2005) Am. J. Hum. Genet. 77: 1061-1074, and Du et al., (2008) J. Lipid Res., 49: 1646-1657. In one useful embodiment, the exogenous LAL is produced in the oviduct of a transgenic avian (*e.g.,* a transgenic chicken), for example, according to a method described in WO 2011/133960 (PCT/US2011/033699), filed April 23, 2011. In some embodiments, the recombinant LAL is produced in an avian cell line. In some embodiments, the recombinant LAL is produced in a mammalian (*e.g.,* a human) cell line.

In one embodiment, exogenous lysosomal acid lipase used in accordance with the invention contains glycans having substantial N-acetylglucosamine (GlcNAc) and mannose terminated N-linked structures. GlcNAc and mannose terminated glycans on exogenous LAL can be specifically recognized and internalized by macrophages and fibroblast. Mannose-6-phosphate (M6P), which can target proteins to the GlcNAc/mannose receptors which are expressed on cells implicated in conditions treatable by exogenous LAL administration, is also typically present on exogenous LAL used in accordance with the invention.

Typically, the exogenous LAL of the invention discussed and disclosed herein is human LAL. In one embodiment, the exogenous LAL has the amino acid sequence provided in Genbank RefSeq NM_000235.2). In one embodiment, the mature exogenous LAL has the amino acid sequence:

In some embodiments, the exogenous LAL comprises amino acids 1-378 of SEQ ID NO:1, amino acids 3-378 of SEQ ID NO:1, amino acids 6-378 of SEQ ID NO:1, or amino acids 7-378 of SEQ ID NO:1. In some embodiments, the exogenous LAL comprises a mixture of at least two polypeptides selected from the group consisting of amino acids 1-378 of SEQ ID NO:1, amino acids 3-378 of SEQ ID NO:1, amino acids 6-378 of SEQ ID NO:1, and amino acids 7-378 of SEQ ID NO:1. In some embodiments, the exogenous LAL comprises a mixture of a polypeptide comprising amino acids 1-378 of SEQ ID NO:1, a polypeptide comprising amino acids 3-378 of SEQ ID NO:1, and a polypeptide comprising amino acids 6-378 of SEQ ID NO:1. In some embodiments, the exogenous LAL comprises a polypeptide that is identical to amino acids 1-378 of SEQ ID NO:1, amino acids 3-378 of SEQ ID NO:1, amino acids 6-378 of SEQ ID NO:1, or amino acids 7-378 of SEQ ID NO:1. In other embodiments, the exogenous LAL comprises a polypeptide that is at least about 70%, about 75%, about 80%, about 85%, about 90%, about 95%, about 96%, about 97%, about 98%, or about 99% identical to amino acids 1-378 of SEQ ID NO:1, amino acids 3-378 of SEQ ID NO:1, amino acids 6-378 of SEQ ID NO:1, or amino acids 7-378 of SEQ ID NO:1. In some embodiments, the exogenous LAL comprises a polypeptide that is a functional fragment of SEQ ID NO:1 or is at least about 70%, about 75%, about 80%, about 85%, about 90%, about 95%, about 96%, about 97%, about 98%, or about 99% identical a functional fragment of SEQ ID NO:1.

In some embodiments the exogenous LAL is a recombinant LAL protein described in WO 2011/133960 (PCT/US2011/033699), filed April 23, 2011.

It is recognized that amino acid positions that are not identical often differ by conservative amino acid substitutions, where amino acid residues are substituted for other amino acid residues with similar chemical properties (*e.g.,* charge or hydrophobicity) and therefore do not change the functional properties of the molecule. Where sequences differ in conservative substitutions, the percent sequence identity can be adjusted upwards to correct for the conservative nature of the substitution. Means for making this adjustment are well known to those of skill in the art. The scoring of conservative substitutions can be calculated according to, for example, the algorithm of Meyers & Millers, Computer Applic. Biol. Sci. 4:11-17 (1988).

A "comparison window" refers to a segment of contiguous positions, such as between about 25 and about 400 positions, or between about 50 to 200 positions, or between about 100 and 150 positions, over which a sequence may be compared to a reference sequence of the same number of contiguous positions after the two sequences are optimally aligned. Methods of alignment of sequences for comparison are well known in the art. Optimal alignment of sequences for comparison can be conducted, for example, by a local homology algorithm (Smith & Waterman, Adv. Appl. Math. 2:482 (1981), by a global alignment algorithm (Needleman & Wunsch, J. Mol. Biol. 48:443 (1970), by search for similarity methods (Pearson & Lipman, Proc. Natl. Acad. Sci. U.S.A. 85:2444 (1988); Altschul et al., Nucl. Acids Res. 25:3389-402 (1997), by computerized implementations of these algorithms (*e.g.,* GAP, BESTFIT, FASTA, and BLAST in the Wisconsin Genetics Software Package, Genetics Computer Group, 575 Science Dr., Madison, Wis.), typically using the default settings, or by manual alignment and visual inspection (see, *e.g.,* Current Protocols in Molecular Biology, Ausubel et al. (eds.), 1994). For example, BLAST protein searches can be performed using the XBLAST program, score=50, wordlength=3 to obtain amino acid sequences that are more than 80% identical to the amino acid sequence of SEQ ID NO:1 or a fragment thereof.

One example of a useful algorithm implementation is PILEUP. PILEUP creates a multiple sequence alignment from a group of related sequences using progressive pairwise alignments. It can also plot a dendrogram showing the clustering relationships used to create the alignment. PILEUP uses a simplification of the progressive alignment method of Feng & Doolittle, J. Mol. Evol. 35:351-360 (1987). The method used is similar to the method described by Higgins & Sharp, CABIOS 5:151-3 (1989). The multiple alignment procedure begins with the pairwise alignment of the two most similar sequences, producing a cluster of two aligned sequences. This cluster can then be aligned to the next most related sequence or cluster of aligned sequences. Two clusters of sequences can be aligned by a simple extension of the pairwise alignment of two individual sequences. A series of such pairwise alignments that includes increasingly dissimilar sequences and clusters of sequences at each iteration produces the final alignment.

In some embodiments, exogenous LAL polypeptides of the invention include variants of the wild-type sequences. These variants fall into one or more of three classes: substitutional, insertional, or deletional variants. These variants can be naturally occurring allelic or interspecies variants or they can be prepared by site-specific mutagenesis of nucleotides in the DNA encoding protein. Site-specific mutagenesis can be performed using cassette or PCR mutagenesis or other techniques well known in the art to produce DNA encoding the variant and, thereafter, expressing the DNA in recombinant cell culture. Variant target protein fragments having up to about 100-150 amino acid residues can be prepared by *in vitro* synthesis using established techniques. Conservative substitution tables providing functionally similar amino acids are well known in the art (Henikoff & Henikoff, Proc. Natl. Acad. Sci. U.S.A. 89:10915-10919 (1992)).

Amino acid substitutions are typically of single residues. Insertions usually will be on the order of from about 1 to about 20 amino acids, although considerably longer insertions can be tolerated. Deletions range from about 1 to about 20 residues, although in some cases, deletions can be much longer. Substitutions, deletions, and insertions or any combinations thereof can be used to arrive at a final derivative.

In some embodiments, the exogenous LAL (*e.g.,* sebelipase alfa) has a specific activity of at least about 100 U/mg. In some embodiments, the exogenous LAL has a specific activity of at least about 200 U/mg. In some embodiments, the exogenous LAL has a specific activity of at least about 250 U/mg. In some embodiments, the exogenous LAL has a specific activity of about 100 to about 1,000 U/mg. In some embodiments, the exogenous LAL has a specific activity of about 100 to about 500 U/mg. In some embodiments, the exogenous LAL has a specific activity of about 100 to about 350 U/mg. In some embodiments, the exogenous LAL has a specific activity of about 200 to about 350 U/mg. In some embodiments, the exogenous LAL has a specific activity of about 250 to about 350 U/mg. In some embodiments, the exogenous LAL has a specific activity of about 250 U/mg. In some embodiments, the exogenous LAL has a specific activity of about 275 U/mg. In some embodiments, the exogenous LAL has a specific activity of about 300 U/mg.

Human LAL has 6 potential sites in its amino acid sequence for N-linked glycosylation: Asn36, Asn72, Asn101, Asn161, Asn273, and Asn321 as set forth in SEQ ID NO:1. In some embodiments, at least 1, 2, 3, 4, or 5 of the N-linked glycosylation sites are glycosylated. In some embodiments all six glycosylation sites are glycosylated. In some embodiments, Asn36, Asn101, Asn161, Asn273, and Asn321 are glycosylated. In some embodiments, Asn36, Asn101, Asn161, Asn273, and Asn321 are glycosylated, and Asn72 is not glycosylated. In some embodiments, the N-glycan structures comprise bi, tri-, and tetraantennary structures with N-acetylglucosamine (GlcNAc), mannose, and/or mannose-6-phosphate (M6P). In some embodiments, the exogenous LAL comprises M6P-modified N-glycans at Asn101, Asn161, and Asn273. In some embodiments, the exogenous LAL does not comprise O-linked glycans. In some embodiments, the exogenous LAL does not comprise sialic acid. In some embodiments, the exogenous LAL has a glycosylation pattern as described in PCT/US2011/033699, filed April 23, 2011.

In some embodiments, the molecular weight of the exogenous LAL is about 55 kD.

In certain embodiments, a subject may be treated with a nucleic acid molecule encoding exogenous LAL, *e.g.,* in a vector. Doses for nucleic acids encoding polypeptides range from about 10 ng to 1 g, 100 ng to 100 mg, 1 µg to 10 mg, or 30-300 µg DNA per patient. Doses for infectious viral vectors vary from 10-100, or more, virions per dose.

While it is possible for the therapeutic protein provided for in this invention, recombinant LAL, to be administered in raw form, it is preferable to administer the therapeutic protein as part of a pharmaceutical formulation.

Pharmaceutical formulations include those suitable for oral, rectal, nasal, topical (including buccal and sub-lingual), vaginal or parenteral. The pharmaceutical formulations include those suitable for administration by injection including intramuscular, sub-cutaneous and intravenous administration. The pharmaceutical formulations also include those for administration by inhalation or insufflation. The formulations can, where appropriate, be conveniently presented in discrete dosage units and can be prepared by any of the methods well known in the art of pharmacy. The methods of producing the pharmaceutical formulations typically include the step of bringing the therapeutic proteins into association with liquid carriers or finely divided solid carriers or both and then, if necessary, shaping the product into the desired formulation.

Pharmaceutical formulations suitable for oral administration can conveniently be presented as discrete units such as capsules, cachets or tablets each containing a predetermined amount of the active ingredient; as a powder or granules; as a solution; as a suspension; or as an emulsion. The active ingredient can also be presented as a bolus, electuary or paste. Tablets and capsules for oral administration can contain conventional excipients such as binding agents, fillers, lubricants, disintegrants, or wetting agents. The tablets can be coated according to methods well known in the art. Oral liquid preparations can be in the form of, for example, aqueous or oily suspensions, solutions, emulsions, syrups or elixirs, or can be presented as a dry product for constitution with water or other suitable vehicle before use. Such liquid preparations can contain conventional additives such as suspending agents, emulsifying agents, non-aqueous vehicles (which can include edible oils) or preservatives.

Therapeutic proteins of the invention can also be formulated for parenteral administration (*e.g.,* by injection, for example bolus injection or continuous infusion) and can be presented in unit dose form in ampoules, pre-filled syringes, small volume infusion or in multi-dose containers with an added preservative. The therapeutic proteins can be injected by, for example, subcutaneous injections, intramuscular injections, and intravenous (IV) infusions or injections. In one embodiment, the exogenous LAL (*e.g.,* sebelipase alfa) is administered intravenously by IV infusion by any useful method. In one example, the exogenous LAL can be administered by intravenous infusion through a peripheral line. In another example, the exogenous LAL can be administered by intravenous infusion through a peripherally inserted central catheter. In another example, the exogenous LAL can be administered by intravenous infusion facilitated by an ambulatory infusion machine attached to a venous vascular access port. In one embodiment, of intravenous infusion, the medication is administered over a period of 1 to 8 hours depending on the amount of medication to be infused and the patient's previous infusion-related reaction history, as determined by a physician skilled in the art. In another embodiment, the exogenous LAL is administered intravenously by IV injection. In another embodiment, the exogenous LAL can be administered via intraperitoneal injection. In still another embodiment, the exogenous LAL is administered via a pharmaceutically acceptable capsule of the therapeutic protein. For example, the capsule can be an enteric-coated gelatin capsule.

In some embodiments, the exogenous LAL (*e.g.,* sebelipase alfa) is administered by infusion, and the infusion can occur over an extended time period, for example, 30 minutes to 10 hours. Thus, the infusion can occur, for example, over a period of about 1 hour, about 2 hours, about 3 hours, about 4 hours, or about 5 hours. The infusion can also occur at various rates. Thus, for example, the infusion rate can be about 1 mL per hour to about 20 mL per hour. In another embodiment, the infusion rate is about 125 mL per hour. In some embodiments, the infusion rate is 5 mL to 10 mL per hour. In one embodiment, the infusion rate is 1, 2, 3, 4, 5, 6, 7, 8, 9, 10, 11, 12, 13, 14, 15, 16, 17, 18, 19 or 20 mL per hour. In one embodiment, the infusion rate is 0.1 to 5 mg/kg/hr. In one embodiment, the infusion rate is about 0.1, about 0.2, about 0.3, about 0.5, about 1.0, about 1.5, about 2.0, or about 3 mg/kg/hr,

The exogenous LAL (*e.g.,* sebelipase alfa) can take such forms as suspensions, solutions, or emulsions in oily or aqueous vehicles, and can contain formulatory agents such as suspending, stabilizing and/or dispersing agents. The exogenous LAL can be in powder form, obtained by aseptic isolation of sterile solid or by lyophilization from solution, for constitution with a suitable vehicle, *e.g.,* sterile, pyrogen-free water, before use.

For topical administration to the epidermis, the exogenous LAL can be formulated as ointments, creams or lotions, or as a transdermal patch. Ointments and creams can, for example, be formulated with an aqueous or oily base with the addition of suitable thickening and/or gelling agents. Lotions can be formulated with an aqueous or oily base and will in general also contain one or more emulsifying agents, stabilizing agents, dispersing agents, suspending agents, thickening agents or coloring agents.

Formulations suitable for topical administration in the mouth include lozenges comprising active ingredient in a flavored base, usually sucrose and acacia or tragacanth; pastilles comprising the active ingredient in an inert base such as gelatin and glycerin or sucrose and acacia; and mouthwashes comprising the active ingredient in a suitable liquid carrier. Pharmaceutical formulations suitable for rectal administration wherein the carrier is a solid are most preferably represented as unit dose suppositories. Suitable carriers include cocoa butter and other materials commonly used in the art, and the suppositories can be conveniently formed by a mixture of the active compound with the softened or melted carrier(s) followed by chilling and shaping in molds.

Formulations suitable for vaginal administration can be presented as pessaries, tampons, creams, gels, pastes, foams or sprays containing in addition to the active ingredient, such carriers as are known in the art to be appropriate.

For intra-nasal administration the exogenous LAL can be used as a liquid spray or dispersible powder or in the form of drops.

Drops can be formulated with an aqueous or non-aqueous base also comprising one or more dispersing agents, solubilizing agents or suspending agents. Liquid sprays are conveniently delivered from pressurized packs.

For administration by inhalation, therapeutic proteins according to the invention can be conveniently delivered from an insufflator, nebulizer or a pressurized pack or other convenient means of delivering an aerosol spray. Pressurized packs can comprise a suitable propellant such as dichlorodifluoromethane, trichlorofluoromethane, dichlorotetrafluoroethane, carbon dioxide or other suitable gas. In the case of a pressurized aerosol, the dosage unit can be determined by providing a valve to deliver a metered amount.

For administration by inhalation or insufflation, the exogenous LAL can take the form of a dry powder composition, for example a powder mix of the compound and a suitable powder base such as lactose or starch. The powder composition can be presented in unit dosage form in, for example, capsules or cartridges or, *e.g.,* gelatin or blister packs from which the powder can be administered with the aid of an inhalator or insufflator. When desired, the above described formulations adapted to give sustained release of the active ingredient, can be employed.

The pharmaceutical compositions described herein can also contain other active ingredients such as antimicrobial agents, or preservatives.

In some embodiments, a pharmaceutical composition comprising exogenous LAL further comprises a buffer. Exemplary buffers include acetate, phosphate, citrate and glutamate buffers. Exemplary buffers also include lithium citrate, sodium citrate, potassium citrate, calcium citrate, lithium lactate, sodium lactate, potassium lactate, calcium lactate, lithium phosphate, sodium phosphate, potassium phosphate, calcium phosphate, lithium maleate, sodium maleate, potassium maleate, calcium maleate, lithium tartarate, sodium tartarate, potassium tartarate, calcium tartarate, lithium succinate, sodium succinate, potassium succinate, calcium succinate, lithium acetate, sodium acetate, potassium acetate, calcium acetate, and mixtures thereof. In some embodiments, the buffer is trisodium citrate dihydrate. In some embodiments, the buffer is citric acid monohydrate. In some embodiments, a pharmaceutical composition comprises trisodium citrate dehydrate and citric acid monohydrate.

In some embodiments, a pharmaceutical composition comprising exogenous LAL further comprises a stabilizer. Exemplary stabilizers include albumin, trehalose, sugars, amino acids, polyols, cyclodextrins, salts such as sodium chloride, magnesium chloride, and calcium chloride, lyoprotectants, and mixtures thereof. In some embodiments, a pharmaceutical composition comprises human serum albumin.

The present invention encompasses any route of administration which facilitates the uptake of the exogenous LAL into the lysosomes of pertinent organs and tissues.

### F. Kits and Unit Dosage Forms

Also provided herein are kits which include sebelipase alfa in a therapeutically effective amount adapted for use in the preceding methods. The kits optionally also can include instructions, *e.g.,* comprising administration schedules, to allow a practitioner (*e.g.,* a physician, nurse, or patient) to administer sebelipase alfa to a patient. The kit also can include a syringe.

In one embodiment, the present disclosure provides a kit for reducing liver fibrosis in a human patient with a lysosomal acid lipase (LAL) deficiency, the kit comprising: (a) a dose of sebelipase alfa; and (b) instructions for using sebelipase alfa in the methods described herein.

### EXAMPLES

### Example 1

### Change in Liver Fibrosis in Children and Adults with Lysosomal Acid Lipase Deficiency After 52 Weeks of Sebelipase alfa (ARISE Trial)

### A. Protocol

Lysosomal Acid Lipase Deficiency (LAL-D) is a rare genetic, progressive disease that frequently leads to fibrosis, micronodular cirrhosis, and ultimately liver failure. In an animal model of LAL-D, sebelipase alfa (SA) improved liver pathology with resolution of hepatomegaly, liver fibrosis and restoration of normal architecture.

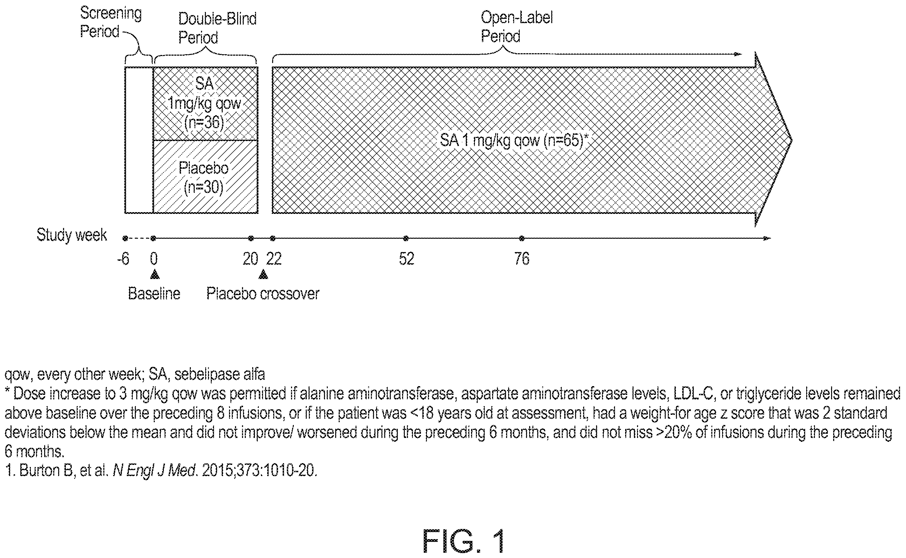

Study LAL-CL02 (also known as "ARISE" (NCT01757184)) is a phase 3 multicenter, randomized, placebo-controlled study designed to evaluate the safety and efficacy of sebelipase alfa (SA) in children and adults with Lysosomal Acid Lipase (LAL) Deficiency. The study consists of a screening period of up to 6 weeks, a 20 week double-blind treatment period, an open-label period of up to 130 weeks (extended to an additional 104 weeks for subjects receiving drug in a region where SA is not registered or not available), and a follow-up phone call at least 4 weeks after the last dose of study drug. The overall study design is depicted in Figure 1. Subjects in the placebo (PBO) group receive SA upon entry into the open-label period.

Subjects were required to be ≥4 years of age at the time of informed consent, have a deficiency of LAL enzyme activity confirmed by dried blood spot (DBS), and an alanine aminotransferase (ALT) ≥ 1.5 x the upper limit of normal (ULN) on 2 consecutive screening measures obtained at least 1 week apart.

Eligible subjects were randomized to double-blind treatment with SA or PBO. Subjects randomized to active treatment received every other week (QOW) intravenous (IV) infusions of SA at a dose of 1 mg/kg, for a total of 11 infusions over the 20-week double-blind treatment period. No dose modifications were permitted during the double-blind period. Subjects who demonstrated evidence of significant clinical progression on blinded study drug were permitted to discontinue from the double-blind treatment period and transition to open-label treatment with SA at a dose of 1 mg/kg QOW.

During the open-label period (beginning at Week 22), all subjects received QOW IV infusions of SA. Dose modifications were permitted during the open-label period. A dose increase to 3 mg/kg QOW was permitted if the subject met protocol defined dose escalation criteria, and a dose reduction to 0.35 mg/kg QOW was permitted in the event of poor tolerability. Liver biopsy is the accepted standard for histologic assessment of liver disease activity and fibrosis, despite such limitations as sampling variability, potential complications of an invasive technique, and subjective scoring. Per the study protocol, liver biopsies were to be obtained at Baseline, Week 20 (end of double-blind treatment period), and Week 52 (open-label period). Subjects could also have an optional liver biopsy anytime between Week 104 and Week 152. Liver biopsies were to be obtained in adult subjects unless medically contraindicated. Liver biopsies were optional in pediatric subjects, with the appropriate consent and where permitted by local regulations and each site's IRB/IEC. Liver biopsies were obtained in 18 of 19 adult subjects (≥ 18 years of age) and in 15 of 47 pediatric subjects. Of these 33 subjects who had at least 1 liver biopsy, 30 subjects had both valid Baseline data and at least 1 valid post-dose liver biopsy.

An independent pathologist at a central facility who was blinded to assessment time point and treatment assignment during the double-blind treatment period evaluated all biopsies semiquantiatively for histologic features such as Ishak Stage, portal inflammation, lobular inflammation, macrovesicular steatosis, and microvesicular steatosis. Computer-assisted morphometry was used to quantify percent steatosis, collagen, fibrosis, and macrophages.

### B. Disposition and Demographics of Subjects with Liver Biopsy Data

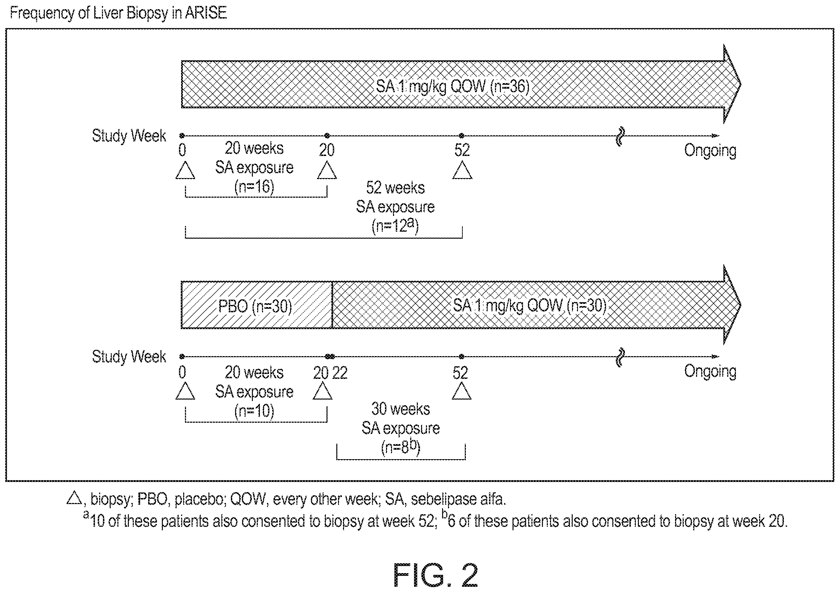

Liver biopsies were obtained at Baseline, Week 20 (end of double-blind treatment period) and Week 52 (open-label period). Optional liver biopsies were also obtained from Weeks 104-152. Figure 2 depicts the frequency of liver biopsy for patients in the SA and PBO arms. Liver biopsies were obtained in subjects ≥ 18 years of age unless medically contraindicated, and on an optional basis in subjects < 18 years of age with consent from a parent or legal guardian (and assent from the subject, if applicable).

A total of 66 subjects were randomized in Study LAL-CL02 ARISE. Thirty-six (36) subjects were randomized to sebelipase alfa (SA) and 30 subjects were randomized to PBO. Of 19 adult subjects, biopsy was medically contraindicated in 1 subject. Of 47 pediatric subjects, biopsy was medically contraindicated in 1 subject. Consent for biopsies was obtained for 15 pediatric subjects. Therefore, 33 subjects (18 adults and 15 pediatric subjects) had at least 1 liver biopsy.

Thirty of the 33 subjects with liver biopsy had paired data (both a valid Baseline biopsy and at least 1 valid post-dose biopsy. The remaining 3 subjects were not included in the paired analyses.

The disposition by treatment group a randomization was as follows:
- 33 subjects had at least 1 liver biopsy
- 32 subjects had liver biopsy data at Baseline
- 31 subjects had valid biopsy data at Baseline (liver biopsy data from one subject was excluded from the analyses because Week 20 liver biopsy was taken 1 day after Week 22 dose of study drug):
   ∘ 18 subjects randomized to SA
   ∘ 13 subjects randomized to PBO
- 30 subjects had valid biopsy data at Baseline and at least 1 valid post-dose liver biopsy (see Table 2):
   ∘ 18 subjects randomized to SA
   ∘ 12 subjects randomized to PBO
- Of the subjects randomized to the SA/SA arm:
   ∘ 10 subjects had biopsy data at Baseline, Week 20, and Week 52
   ∘ 6 subjects had biopsy data at Baseline and Week 20 only
   ∘ 2 subjects had biopsy data at Baseline and Week 52 only
- Of the subjects randomized to the PBO/SA arm:
   ∘ 6 subjects had biopsy data at Baseline, Week 20, and Week 52
   ∘ 4 subjects had biopsy data at Baseline and Week 20 only
   ∘ 2 subjects had biopsy data at Baseline and Week 52 only

The disposition by time period was as follows:
- 26 subjects had paired liver biopsy data for the double-blind period (Baseline and Week 20):
   ∘ 16 subjects in the SA arm
   ∘ 10 subjects in the PBO arm
- 20 subjects had paired liver biopsy data at Baseline and Week 52:
   ∘ 12 subjects in the SA/SA arm
   ∘ 8 subjects in the PBO/SA arm

**Table 2: Subjects with Valid Liver Biopsy Data at Baseline and Post-Dose**

| **Patient** | **Treatment Arm at Randomization** | **Baseline Biopsy** | **Week 20 Biopsy** | **Week 52 Biopsy** |
|---|---|---|---|---|
| 1 | SA | X | X | X |
| 2 | SA | X | X | X |
| 3 | SA | X | X | X |
| 4 | SA | X | X | X |
| 5 | SA | X | X | X |
| 6 | SA | X | X | X |
| 7 | SA | X | X | X |
| 8 | SA | X | X | X |
| 9 | SA | X | X | X |
| 10 | SA | X | X | X |
| 11 | PBO | X | X | X |
| 12 | PBO | X | X | X |
| 13 | PBO | X | X | X |
| 14 | PBO | X | X | X |
| 15 | PBO | X | X | X |
| 16 | PBO | X | X | X |
| 17 | SA | X | X | |
| 18 | SA | X | X | |
| 19 | SA | X | X | |
| 20 | SA | X | X | |
| 21 | SA | X | X | |
| 22 | SA | X | X | |
| 23 | PBO | X | X | |
| 24 | PBO | X | X | |
| 25 | PBO | X | X | |
| 26 | PBO | X | X | |
| 27 | SA | X | | X |
| 28 | SA | X | | X |
| 29 | PBO | X | | X |
| 30 | PBO | X | | X |
| | | **30** | **26** | **20** |

Age is subject age at Baseline, when informed consent was signed. There were 9 subjects who had dose escalations per protocol-defined criteria (from 1 mg/kg QOW to 3 mg/kg QOW). No subjects with liver biopsy data had a dose escalation before Week 52. In other words, all subjects with liver biopsy data through Week 52 were exposed only to PBO and/or 1 mg/kg SA.

Liver biopsies were obtained in 15 of 47 pediatric subjects. Two of these 15 pediatric subjects each had only 1 liver biopsy, and so are not included in the cohort of 13 pediatric subjects with paired liver biopsy data.

### C. Liver Biopsy Evaluation

The following assessments were made using liver histopathology samples:
1. Percent steatosis by morphometry (H&E Stain)
2. Ishak stage (scored 0-6) (H&E Stain)
3. Percent collagen (Sirius Red Stain)
4. Percent fibrosis (SMA Stain)
5. Macrophages (CD68 Stain)
6. Portal inflammation (scored 0-4) (H&E Stain)
7. Lobular inflammation (scored 0-4) (H&E Stain)
8. Macrovesicular steatosis (scored 0-4) (H&E Stain)
9. Microvesicular steatosis (scored 0-4) (H&E Stain)

Evaluation of liver tissue is largely based on a thorough examination of sections stained with hematoxylin and eosin (H&E). H&E staining is the most common staining technique used in histology. It is the primary technique for evaluation of morphology. Additional stains, such as those described below, were used to identify features not easily seen on an H&E stain.

Smooth muscle actin (SMA): The alpha isotype of actin expressed by hepatic stellate cells reflects their activation to myofibroblast-like cell and has been directly related to experimental liver fibrogenesis, and indirectly to human fibrosis in chronic liver disease. In vivo, alpha-smooth muscle actin expression is a reliable marker of hepatic stellate cells activation which precedes fibrous tissue deposition and it could be useful to identify the earliest stages of hepatic fibrosis and monitoring the efficacy of the therapy.

CD68: CD68 (Cluster of Differentiation 68) is a lysosomal glycoprotein expressed in macrophages, such as Kupffer cells found in the liver. Immunohistochemistry for CD68 is used to identify Kupffer cells and other macrophages.

Sirius red is a polyazo dye used for selective staining of collagen when quantitative measurement of fibrosis by morphometry is required. Sirius red has affinity for most hepatic collagens, including fibril-forming types (types I and III,) so that quantitative assessment of fibrosis can be performed in a reliable and reproducible way.

### 1. Percent Steatosis (H&E Stain)

A quantitative morphometry-based assessment of liver histopathology was utilized for hepatic steatosis (measured in the H&E stained section). Liver biopsy parameters at baseline are summarized in Table 3.

**Table 3. Liver Biopsy Parameters at Baseline**

| **Liver Biopsy Assessment at the Baseline Visit** | **Patients (n=32)** |
|---|---|
| Percentage of steatosis, mean (SD) | 31.6 (21.8) |
| Macrovesicular steatosis, n (%)* | 5 (16%) |
| Microvesicular steatosis, n (%)^{‡} | 31 (97%) |
| Percentage of fibrinogenic cells, mean (SD) | 6.7 (7.7) |
| Percentage of collagen, mean (SD) | 11.9 (13.4) |
| Percentage of microphages (CD68+ cells), mean (SD) | 7.9 (6.3) |

| | |
|---|---|
| *All 5 samples had fat vacuoles replacing <33% of hepatocyte area; ^{≠}28 (88%) samples had fat vacuoles replacing >66% of hepatocyte area. | |

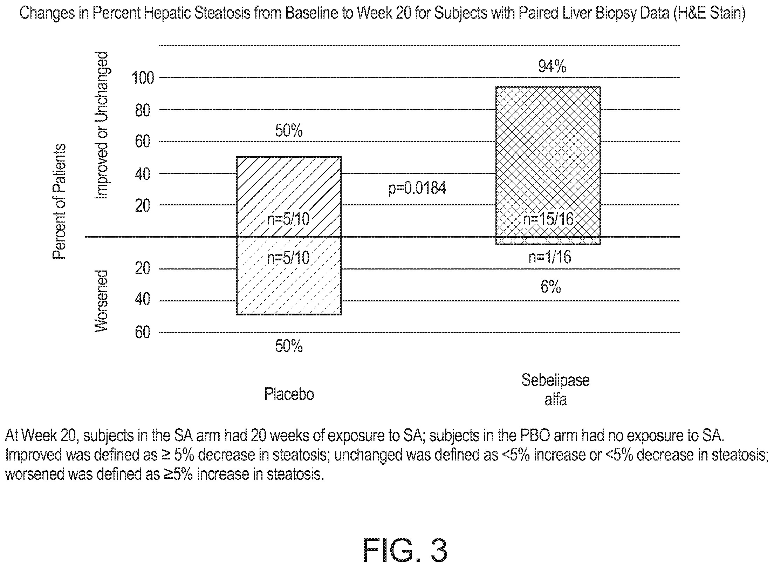

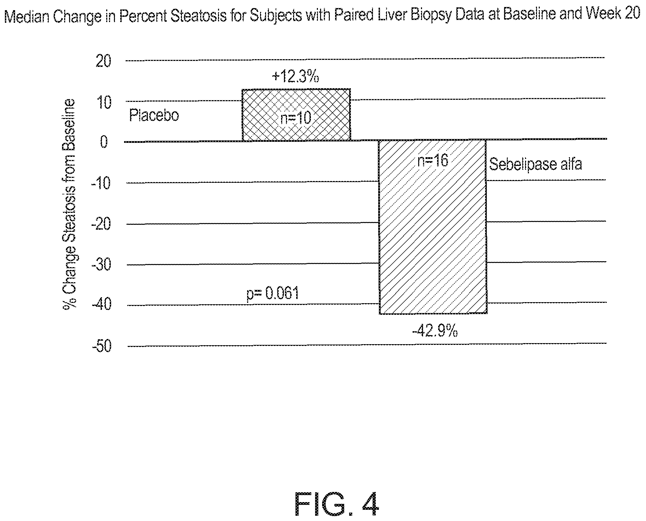

The change from Baseline to Week 20 in hepatic steatosis is presented in Table 4 and Figure 3. Post-hoc analysis showed that when responders were defined as subjects who improved or were unchanged from Baseline (with improved or unchanged defined as <5% increase in steatosis), at Week 20 there were significantly more subjects randomized to the SA arm who responded (15/16 subjects, 94%) compared with subjects randomized to the PBO arm (5/10 subjects, 50%; p=0.0184). Patients who received SA showed greater median percentage improvement in steatosis than those who received PBO (-42.9% versus 12.3%, p = 0.061) (Figure 4). In the cohort that received 52 weeks of SA (n=12), median percentage of steatosis decreased by 37% from baseline.

**Table 4: Changes in Hepatic Steatosis from Baseline to Week 20 for Subjects with Paired Liver Biopsy Data (H&E Stain)**

| **Result at Week 20 (end of double-blind treatment period)** | **SA N=16 n (%)** | **PBO N=10 n (%)** | **Differenc e (%)** | **p-value** |
|---|---|---|---|---|
| Improved or unchanged from baseline | 15 (94) | 5 (50) | 44 | 0.0184 |
| Improved from baseline | 10 (63) | 4 (40) | 23 | 0.4216 |
| Unchanged from baseline | 5 (31) | 1 (10) | 21 | 0.3524 |
| Worsened from baseline | 1 (6) | 5 (50) | -44 | 0.0184 |

| | | | | |
|---|---|---|---|---|
| SA= sebelipase alfa; PBO=placebo; H&E stain= hematoxylin and eosin. At Week 20, subjects in the SA arm had 20 weeks of exposure to SA; subjects in the PBO arm had no exposure to SA. | | | | |

When the median percent change in steatosis of each individual subject's morphometrically determined values from Baseline to Week 20 was analyzed, the trend was for subjects treated with SA to show more aggregate improvement in percentage steatosis compared with subjects who received PBO (Table 5 and Figure 4), although the differences did not reach statistical significance (p=0.0613).

**Table 5: Summary Statistics for Percent Hepatic Steatosis by Timepoint and Treatment Group (Baseline and Week 20)**

| **Morphometric analysis of Hepatic Steatosis (H&E Stain)** | **Parameter** | **SA N=19** | **PBO N=13** | **p-value** |
|---|---|---|---|---|
| Baseline Percent Steatosis | n (%) | 18 (95) | 13 (100) | |
| | Mean (SD) | 29.7 (20.6) | 34.3 (24.8) | |
| | Median | 23.9 | 24.8 | |
| | Min, Max | 6.2, 79.8 | 5.4, 81.2 | |
| Week 20 Percent Steatosis | n (%) | 16 (84) | 10 (77) | |
| | Mean (SD) | 16.9 (9.8) | 28.4 (12.3) | |
| | Median | 15.6 | 25.5 | |
| | Min, Max | 4.5,45.1 | 11.6,44.8 | |
| Change in Percent Steatosis at Week 20 | n (%) | 16 (84) | 10 (77) | |
| | Mean (SD) | -28.3 (50.9) | 28.6 (90.9) | |
| | Median | -42.9 | 12.3 | |
| | Min, Max | -81.6, 131.6 | -85.7, 200.0 | 0.0613 |

| | | | | |
|---|---|---|---|---|
| * Wilcoxon rank sum test comparing the percent change between the treatment groups SA= sebelipase alfa; PBO=placebo; H&E stain= hematoxylin and eosin. At Week 20, subjects in the SA arm had 20 weeks of exposure to SA; subjects in the PBO arm had no exposure to SA. | | | | |

Summary statistics for percent steatosis are presented for Baseline, Week 20, and Week 52 for subjects randomized to SA (Table 6) and for subjects randomized to PBO (Table 7). At Baseline, the morphometric scores for percent steatosis assessed by H&E were comparable between the 2 treatment groups (29.7% in the SA group versus 28.4% in the PBO group).

At Week 20, SA/SA subjects showed a mean decrease (improvement) in percent steatosis of 14.8% and a median decrease (improvement) of 13.05%. SA/PBO subjects showed a mean decrease (improvement) of 6.1%, and a median increase (worsening) of 3.6%.

At Week 52, SA/SA subjects showed a mean decrease (improvement) in percent steatosis of 11.2%, a median decrease (improvement) of 15.4%, and a mean percent change from Baseline of 1.1%. SA/PBO subjects showed a mean decrease (improvement) in percent steatosis of 3.1%, a median decrease (improvement) of 10.4%, and a mean percent change from Baseline of 15.4%. The trend for improvement from Baseline in percent steatosis in subjects treated with SA continued through Week 52. This is apparent for both subjects treated with SA for 52 weeks (subjects randomized to SA) and for 30 weeks (subjects randomized to PBO).

**Table 6: Percent Steatosis by Timepoint: Baseline, Week 20, and Week 52 for Subjects Randomized to SA (H&E Stain)**

| **Statistic** | **Baseline Value** | **Week 20 Value** | **Week 20 Change from Baseline** | **Week 52 Value** | **Week 52 Change from Baseline** | **Week 52 Percentage Change from Baseline** |
|---|---|---|---|---|---|---|
| n (%) | 18 (95) | 16 (84) | 16 (84) | 13 (68) | 12 (63) | 12 (63) |
| Mean (SD) | 29.73 (20.574) | 16.86 (9.815) | -14.83 (15.079) | 18.25 (12.666) | -11.22 (19.891) | 1.13 (106.101) |
| Median | 23.90 | 15.55 | -13.05 | 14.30 | -15.40 | -36.79 |
| Min, Max | 6.2, 79.8 | 4.5,45.1 | -38.7, 10.0 | 4.0, 40.5 | -40.8,18.1 | -90.0, 238.2 |

| | | | | | | |
|---|---|---|---|---|---|---|
| SA= sebelipase alfa. Subjects in the SA/SA arm had 20 weeks of exposure to SA at Week 20 and 52 weeks of exposure at Week 52. | | | | | | |

**Table 7: Percent Steatosis by Timepoint: Baseline, Week 20, and Week 52 for Subjects Randomized to PBO (H&E Stain)**

| **Statistic** | **Baseline Value** | **Week 20 Value** | **Week 20 Change from Baseline** | **Week 52 Value** | **Week 52 Change from Baseline** | **Week 52 Percentage Change from Baseline** |
|---|---|---|---|---|---|---|
| n (%) | 13 (100) | 10 (77) | 10 (77) | 8 (62) | 6 (46) | 6 (46) |
| Mean (SD) | 34.30 (24.840) | 28.41 (12.345) | -6.10 (27.312) | 28.68 (17.806) | -3.05 (21.046) | 15.37 (112.239) |
| Median | 24.80 | 25.45 | 3.60 | 27.85 | -10.40 | -45.47 |
| Min, Max | 5.4, 81.2 | 11.6, 44.8 | -69.6, 26.9 | 6.8, 56.6 | -22.5, 23.4 | -63.8, 199.1 |

| | | | | | | |
|---|---|---|---|---|---|---|
| PBO=placebo. Subjects in the PBO/SA arm had no exposure to SA at Week 20 and 30 weeks of exposure at Week 52. | | | | | | |

### 2. Ishak Stage (H&E Stain)

Ishak stage scores range from 0 to 6, and were determined based on histopatholgical assessment of the liver biopsies (Table 8).

Other staging systems described in the literature (Knodell histologic activity index, Batts-Ludwig stage, Scheuter, and METAVIR) are scored on scales that range from 0 to 4. The Ishak scale has greater granularity (stages range from 0 to 6), with each Ishak fibrosis stage reflecting more scarring than the preceding stage. In recent years, the Ishak staging system has become widely used in clinical trials, especially in the United States.

Note that there is not a linear relationship between Ishak stages and fibrosis. For example, fibrosis changes from stages 1 to 2 are much smaller (3.0% to 3.6%) than changes from stages 4 to 5 (13.7% to 24.3%).

**Table 8: Description of Ishak Stages**

| Ishak stage: Categorical Description | Ishak stage: Categorical Assignment | Mean Fibrosis Measurement |
|---|---|---|
| No fibrosis | 0 | |
| Fibrous expansion of some portal areas +/- short fibrous septa | 1 | 3.0% |
| Fibrous expansion of most portal areas +/- short fibrous septa | 2 | 3.6% |
| Fibrous expansion of most portal areas with occasional portal to portal bridging | 3 | 6.5% |
| Fibrous expansion of portal areas with marked bridging (portal to portal as well as portal to central) | 4 | 13.7% |
| Marked bridging (portal to portal and/or portal to central), with occasional nodules (incomplete cirrhosis) | 5 | 24.3% |
| Cirrhosis, probable or definite | 6 | 27.8% |

| | | |
|---|---|---|
| Source: R A Standish, E Cholongitas, A Dhillon, A K Burroughs, A P Dhillon. An appraisal of the histopatholgical assessment of liver fibrosis. Gut 2006;55:569-578 | | |

The changes from Baseline to Week 20 and the changes from Baseline to Week 52 in Ishak scores are presented in Table 9 (subjects randomized to SA) and Table 10 (subjects randomized to PBO).

At baseline, 32 patients with biopsies had fibrosis (stage ≥ 1). 47% had bridging fibrosis (stage 3-4) and 31% has cirrhosis (stage 5-6). Of the 20 patients with paired biopsy data from baseline and study week 52, 8 patients were Ishak stage 5 or 6 at baseline, indicative of early or established cirrhosis.

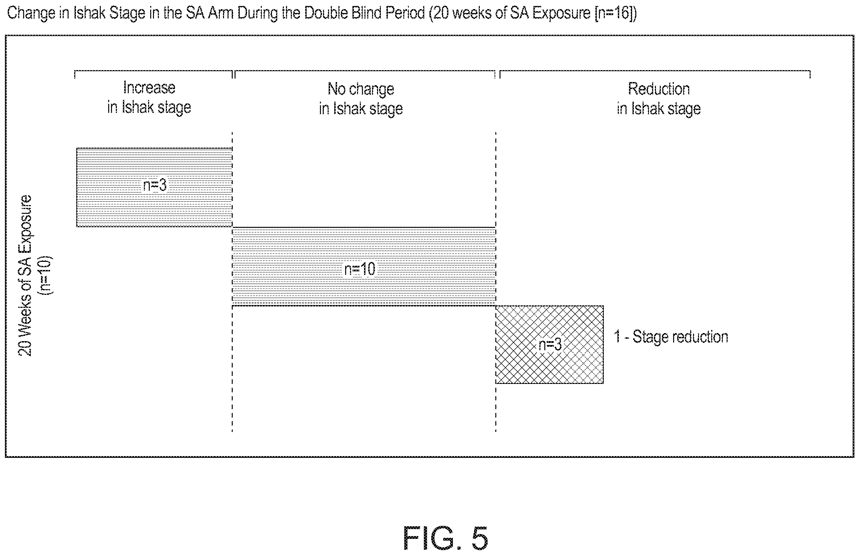

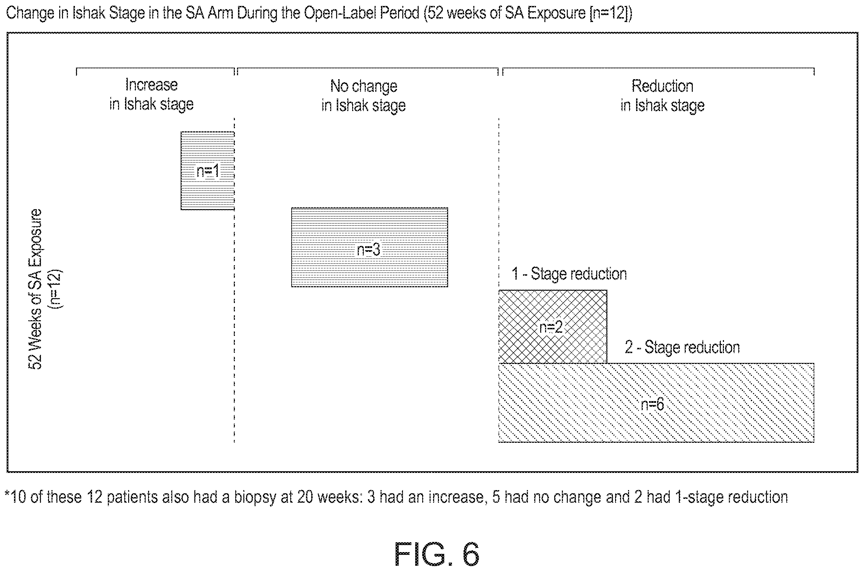

Figure 5 depicts the change in Ishak Stage in the SA Arm during the double-blind period (20 weeks of SA Exposure [n=16]). Figure 6 depicts the change in Ishak Stage in the SA Arm during the open-label period (52 weeks of SA Exposure [n=12]). In the 12 patient cohort receiving 52 weeks of SA, 8 had a reduction in fibrosis stage (6 had a 2-point reduction and 2 had a 1-point reduction) and 3 had no change. One patient had an increase.

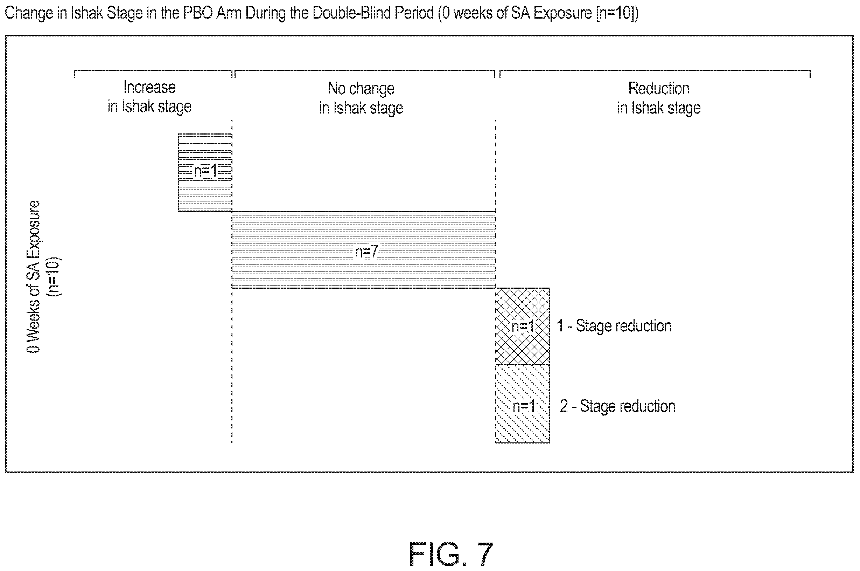

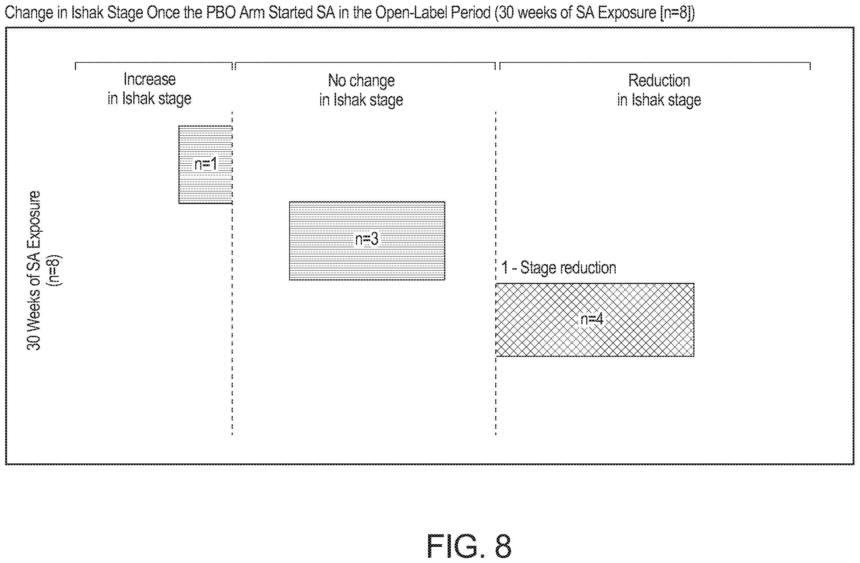

Figure 7 depicts the change in Ishak Stage in the PBO arm during the double-blind period (0 weeks of SA Exposure [n=10]). Figure 8 depicts the change in Ishak Stage once the PBO arm started SA in the open-label period (30 weeks of SA Exposure [n=8]). In the 8 patient cohort who received 30 weeks of SA, 4 had a 1-point reduction in fibrosis stage, 3 had no change, and 1 had an increase.

At Week 20, the majority of subjects in both the SA and PBO arms (17 of 26) had no change from Baseline in Ishak scores.

As noted above, at Week 52, 6 subjects had a ≥2 point reduction in Ishak scores from Baseline (see Figure 6). All 6 subjects had 52 weeks of SA exposure, emphasizing greater benefit with longer exposure to SA. Five of the 6 subjects with a ≥2 point reduction in Ishak stage scores from Baseline to Week 52 had an Ishak stage of 3 at Baseline, suggesting that when treatment is started before fibrosis is well established, there is a greater treatment benefit. Overall, at Week 52, 18 of 20 subjects (90%) with paired liver biopsy data had Ishak scores that had improved or did not progress.

More than half of the patients who had a 1 point reduction in Ishak stage, had cirrhosis at baseline. Of the six (6) patients who had a 2 point reduction in stage, one (1) had cirrhosis at baseline. Five (5) of the six (6) had stage 3 at baseline and experienced a mean percentage change at 52 weeks of -60.5% in ALT, -40.3% in LDL-C and -31.6% in liver fat content as assessed by MRI. During 52 weeks of the study, sixty-two (62) patients had ≥1 adverse event (AE), most of which were unrelated. Ten (10) patients had infusion associated reactions (IARs). Five (5) patients had Serious Adverse Events (AEs), one of which was considered related to treatment (an IAR). SA was stopped and the patient was reintroduced to SA following a brief desensitization protocol and remains on study drug. No patient discontinued the study due to an AE.

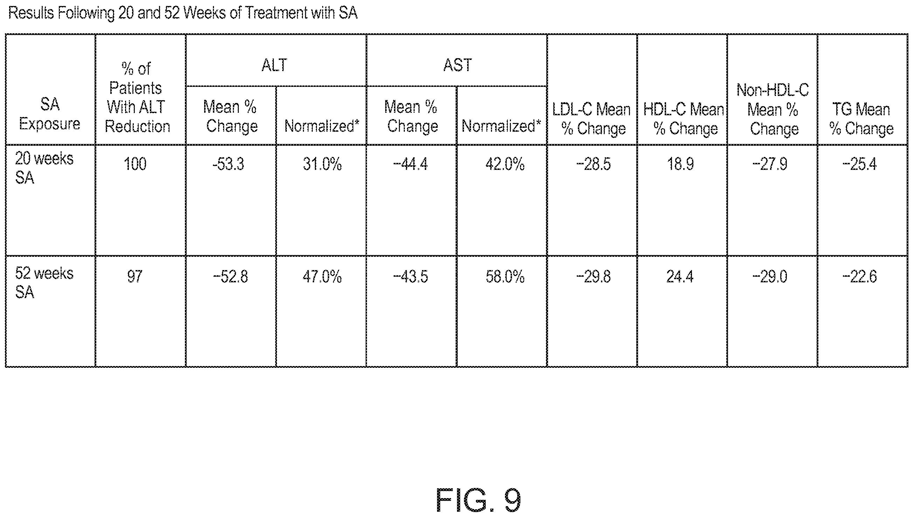

Of the twelve (12) patients treated with SA for 52 weeks, 8 had demonstrated reduction in fibrosis stage, three (3) had no change, and one (1) had an increase. Longer duration of treatment tended to show greater reductions in fibrosis. These reductions were accompanied by reductions in liver fat, ALT, and LDL-C. A summary of the ALT, AST, LDL-C, HDL-C, Non-HDL-C, and TG data is set forth in Figure 9. These data support the value of early and long term SA treatment in children and adults with LAL-D.

**Table 9: Changes from Baseline to Week 20 and to Week 52 in Ishak Scores for Subjects Randomized to SA (H&E Stain)**

| **Change from Baseline in Ishak Score** | **Week 20 SA/SA (N=16)** n **(%)** | **Week 52 SA/SA (N=12)** n **(%)** |
|---|---|---|
| 2+ point reduction | 0 (0) | 6 (50) |
| 1 point reduction | 3 (19) | 2 (17) |
| No change | 10 (63) | 3 (25) |
| 1 point increase | 1 (6) | 1 (8) |
| 2+ point increase | 2 (13) | 0 |

| | | |
|---|---|---|
| SA= sebelipase alfa. Subjects in the SA/SA arm had 20 weeks of exposure to SA at Week 20 and 52 weeks of exposure at Week 52. | | |

**Table 10: Changes from Baseline to Week 20 and to Week 52 in Ishak Scores for Subjects Randomized to PBO (H&E Stain)**

| **Change from Baseline in Ishak Score** | **Week 20 PBO/SA (N=10) n (%)** | **Week 52 PBO/SA (N=8) n (%)** |
|---|---|---|
| 2+ point reduction | 1 (10) | 0 |
| 1 point reduction | 1 (10) | 4 (50) |
| No change | 7 (70) | 3 (38) |
| 1 point increase | 1 (1-) | 1 (13) |
| 2+ point increase | 0 | 0 |

| | | |
|---|---|---|
| SA= sebelipase alfa; PBO=placebo. Subjects in the PBO/SA arm had no exposure to SA at Week 20 and 30 weeks of exposure at Week 52. | | |

All 31 subjects with Baseline biopsies had some fibrosis at Baseline (Table 11 and Table 12). Ten subjects had Ishak scores of 5 or 6 at Baseline, indicative of early or established cirrhosis. For subjects randomized to SA (Table 9), Ishak scores improved by Week 20, and continued to improve by Week 52 (when 21% of subjects had no fibrosis), suggesting that the longer the treatment, the more improvement was evident.

**Table 11: Ishak Stage Scores by Timepoint for Subjects Randomized to SA**

| **Ishak Stage** | Baseline n (%) | **Week** 20 n (%) | Week 52 n (%) |
|---|---|---|---|
| Subjects with liver biopsy data | 18 (95) | 16 (84) | 13 (68) |
| Stage 0: No fibrosis | 0 | 0 | 4 (21) |
| Stage 1: Fibrous expansion of some portal areas +/- short fibrous septa | 1 (5) | 0 | 3 (16) |
| Stage 2: Fibrous expansion of most portal areas +/- short fibrous septa | 2(11) | 4 (21) | 0 |
| Stage 3: Fibrous expansion of most portal areas with occasional portal to portal bridging | 9 (47) | 5 (26) | 2 (11) |
| Stage 4: Fibrous expansion of portal areas with marked bridging (portal to portal as well as portal to central) | 1 (5) | 1 (5) | 0 |
| Stage 5: Marked bridging (portal to portal and/or portal to central), with occasional nodules (incomplete cirrhosis) | 1 (5) | 1 (5) | 2 (11) |
| Stage 6: Cirrhosis, probable or definite | 4 (21) | 5 (26) | 2 (11) |

| | | | |
|---|---|---|---|
| SA= sebelipase alfa. Subjects in the SA/SA arm had 20 weeks of exposure to SA at Week 20 and 52 weeks of exposure at Week 52. | | | |

**Table 12: Ishak Stage Scores by Timepoint for Subjects Randomized to PBO**

| **Ishak Stage** | **Baseline n (%)** | **Week 20 n (%)** | **Week 52 n (%)** |
|---|---|---|---|
| Subjects with liver biopsy data | 13 (100) | 10 (77) | 8 (62) |
| Stage 0: No fibrosis | 0 | 1 (8) | 0 |
| Stage 1: Fibrous expansion of some portal areas +/- short fibrous septa | 1 (8) | 2 (15) | 1 (8) |
| Stage 2: Fibrous expansion of most portal areas +/- short fibrous septa | 2 (15) | 0 | 0 |
| Stage 3: Fibrous expansion of most portal areas with occasional portal to portal bridging | 4 (31) | 3 (23) | 3 (23) |
| Stage 4: Fibrous expansion of portal areas with marked bridging (portal to portal as well as portal to central) | 1 (8) | 1 (8) | 1 (8) |
| Stage 5: Marked bridging (portal to portal and/or portal to central), with occasional nodules (incomplete cirrhosis) | 1 (8) | 0 | 2 (15) |
| Stage 6: Cirrhosis, probable or definite | 4 (31) | 3 (23) | 1 (8) |

| | | | |
|---|---|---|---|
| SA= sebelipase alfa ; PBO=placebo. Subjects in the PBO/SA arm had no exposure to SA at Week 20 and 30 weeks of exposure at Week 52. | | | |

### 3. Collagen (Sirius Red Stain)

Summary statistics for percent collagen are presented for Baseline and Week 52 for subjects randomized to SA (Table 13) and for subjects randomized to PBO (Table 14). At Baseline, the morphometric scores for percent collagen assessed by Sirius red were mean of 9.2% (median of 8.1%) in the SA group and mean of 16.4% (median of 10.8%) in the PBO group.

At Week 20, both treatment groups showed a small median increase (3.85% for SA subjects and 3.35% for PBO subjects).

By Week 52, subjects in the SA/SA treatment group showed improvement in percent collagen: the mean change from Baseline was a decrease (improvement) of 3.2% for SA/SA subjects (median decrease of 2.5%). Subjects in the PBO/SA treatment group had a mean increase (worsening) from Baseline of 0.6% and a median increase (worsening) of 2.7%.

**Table 13: Percent Collagen: Liver Biopsy Data by Timepoint for Subjects Randomized to SA (Sirius Red Stain)**

| **Statistic** | **Baseline Value** | **Week 20 Value** | **Week 20 Change from Baseline** | **Week 52 Value** | **Week 52 Change from Baseline** | **Week 52 Percentage Change from Baseline** |
|---|---|---|---|---|---|---|
| n (%) | 18 (95) | 16 (84) | 16 (84) | 13 (68) | 11 (58) | 11 (58) |
| Mean (SD) | 9.17 (4.936) | 16.54 (11.095) | 7.60 (10.277) | 6.50 (7.199) | -3.23 (6.037) | -21.28 (80.622) |
| Median | 8.05 | 14.35 | 3.85 | 5.60 | -2.50 | -30.12 |
| Min, Max | 2.1,21.9 | 4.1,43.2 | -6.0,35.1 | 0.4, 26.2 | -11.9,4.3 | -95.9, 152.4 |

| | | | | | | |
|---|---|---|---|---|---|---|
| SA= sebelipase alfa; Subjects in the SA/SA arm had 20 weeks of exposure to SA at Week 20 and 52 weeks of exposure at Week 52. | | | | | | |

**Table 14: Percent Collagen: Liver Biopsy Data by Timepoint for Subjects Randomized to PBO (Sirius Red Stain)**

| **Statistic** | **Baseline Value** | **Week 20 Value** | **Week 20 Change from Baseline** | **Week 52 Value** | **Week 52 Change from Baseline** | **Week 52 Percentage Change from Baseline** |
|---|---|---|---|---|---|---|
| n (%) | 13 (100) | 10 (77) | 10 (77) | 8 (62) | 6 (46) | 6 (46) |
| Mean (SD) | 16.38 (19.753) | 16.06 (13.936) | 0.05 (11.228) | 10.79 (6.179) | 0.58 (5.723) | 9.75 (65.017) |
| Median | 10.80 | 10.65 | 3.35 | 10.35 | 2.70 | 18.25 |
| Min, Max | 0.7, 77.0 | 5.0, 53 | -24.0, 10.3 | 2.3, 21.6 | -6.5,7.1 | -73.9, 78.0 |

| | | | | | | |
|---|---|---|---|---|---|---|
| SA= sebelipase alfa; PBO=placebo. Subjects in the PBO/SA arm had no exposure to SA at Week 20 and 30 weeks of exposure at Week 52. | | | | | | |

### 4. Fibrosis (SMA stain)

Summary statistics for percent fibrosis are presented for Baseline and Week 52 for subjects randomized to SA (Table 15) and for subjects randomized to PBO (Table 16). At Baseline, the morphometric scores for percent fibrosis assessed by SMA stain were a mean of 6.1% (median 4.05%) in the SA group and a mean of 7.9% (median 4.00%) in the PBO group. At Week 20, the percent fibrosis assessments were 5.9% (median 6.05%) in the SA group and 7.7% (median 6.6%) in the PBO group.

By Week 52, subjects in both treatment groups showed improvement in percent fibrosis: the mean decrease (improvement) from Baseline was 4.4% for SA/SA subjects (median was 3.1%) and 2.4% for PBO/SA subjects (median was 2.9%).

**Table 15: Percent Fibrosis: Liver Biopsy Data by Timepoint for Subjects Randomized to SA (SMA Stain)**

| **Statistic** | **Baseline Value** | **Week 20 Value** | **Week 20 Change from Baseline** | **Week 52 Value** | **Week 52 Change from Baseline** | **Week 52 Percentage Change from Baseline** |
|---|---|---|---|---|---|---|
| n (%) | 18 (95) | 16 (84) | 16 (84) | 13 (68) | 12 (63) | 12 (63) |
| Mean (SD) | 6.11 (5.944) | 5.90 (3.754) | -0.02 (4.858) | 2.65 (2.296) | -4.37 (6.273) | -30.63 (79.999) |
| Median | 4.05 | 6.05 | -0.25 | 1.70 | -3.10 | -69.32 |
| Min, Max | 1.4, 26.1 | 0.9, 13.5 | -12.6, 8.0 | 0.5, 7.8 | -18.3,3.0 | -93.2, 176.5 |

| | | | | | | |
|---|---|---|---|---|---|---|
| SA= sebelipase alfa. Subjects in the SA/SA arm had 20 weeks of exposure to SA at Week 20 and 52 weeks of exposure at Week 52. | | | | | | |

**Table 16: Percent Fibrosis: Liver Biopsy Data by Timepoint for Subjects Randomized to PBO (SMA Stain)**

| **Statistic** | **Baseline Value** | **Week 20 Value** | **Week 20 Change from Baseline** | **Week 52 Value** | **Week 52 Change from Baseline** | **Week 52 Percentage Change from Baseline** |
|---|---|---|---|---|---|---|
| n (%) | 13 (100) | 10 (77) | 10 (77) | 8 (62) | 6 (46) | 6 (46) |
| Mean (SD) | 7.90 (9.902) | 7.66 (5.768) | -1.29 (8.052) | 3.56 (1.671) | -2.42 (3.475) | -17.78 (63.428) |
| Median | 4.00 | 6.60 | 0.95 | 3.40 | -2.95 | -39.94 |
| Min, Max | 1.2,35.5 | 1.8, 22.4 | -16.9, 8.1 | 1.4,6.7 | -6.4, 2.2 | -67.6, 95.7 |

| | | | | | | |
|---|---|---|---|---|---|---|
| SA= sebelipase alfa; PBO=placebo. Subjects in the PBO/SA arm had no exposure to SA at Week 20 and 30 weeks of exposure at Week 52. | | | | | | |

### 5. Macrophages (CD68 Immunostain)

Summary statistics for percent macrophages are presented for Baseline and Week 52 for subjects randomized to SA (Table 17) and for subjects randomized to PBO (Table 18). At Baseline, the morphometric scores for percent CD68+ cells assessed by CD68+ immunostain were a mean of 9.1% (median 7.4%) in the SA group and a mean of 4.6% (median of 6.0%) in the PBO group.

At Week 20, the SA group had improved to a mean of 6.4% (median of 4.6%). The PBO group had a mean of 7.1% (median of 6.2%).

At Week 52, subjects in the SA/SA group continued to show improvement in percent macrophages: the mean decrease (improvement) from Baseline was 4.4% (median decrease of 1.6%). Subjects in the PBO/SA group had a mean increase (worsening) from Baseline of 1.75% (median increase of 1.8%).

**Table 17: Percent Macrophages: Liver Biopsy Data by Timepoint for Subjects Randomized to SA (CD68+ Immunostain)**

| **Statistic** | **Baseline Value** | **Week 20 Value** | **Week 20 Change from Baseline** | **Week 52 Value** | **Week 52 Change from Baseline** | **Week 52 Percentage Change from Baseline** |
|---|---|---|---|---|---|---|
| n (%) | 18 (96) | 16 (84) | 16 (84) | 13 (68) | 12 (63) | 12 (63) |
| Mean (SD) | 9.14 (7.056) | 6.44 (5.42) | -2.90 (4.944) | 6.36 (5.173) | -4.40 (6.325) | -31.46 (34.034) |
| Median | 7.40 | 4.55 | -1.65 | 6.00 | -1.55 | -34.67 |
| Min, Max | 0.8,23.9 | 0.5, 19.0 | -14.9, 2.6 | 1.1, 19.9 | -19.3,0.4 | -80.8, 37.5 |

| | | | | | | |
|---|---|---|---|---|---|---|
| SA= sebelipase alfa. Subjects in the SA/SA arm had 20 weeks of exposure to SA at Week 20 and 52 weeks of exposure at Week 52. | | | | | | |

**Table 18: Percent Macrophages: Liver Biopsy Data by Timepoint for Subjects Randomized to PBO (CD68+ Immunostain)**

| **Statistic** | **Baseline Value** | **Week 20 Value** | **Week 20 Change from Baseline** | **Week 52 Value** | **Week 52 Change from Baseline** | **Week 52 Percentage Change from Baseline** |
|---|---|---|---|---|---|---|
| n (%) | 13 (100) | 10 (77) | 10 (77) | 8 (62) | 6 (46) | 6 (46) |
| Mean (SD) | 4.60 (4.982) | 7.08 (4.880) | -0.17 (3.630) | 8.41 (5.309) | 1.75 (3.409) | 33.01 (51.093) |
| Median | 6.00 | 6.15 | -1.45 | 5.10 | 1.75 | 40.26 |
| Min, Max | 1.3, 19.6 | 1.4, 15.0 | -4.6, 5.0 | 4.3, 17.4 | -3.8,6.2 | -46.3, 104.8 |

| | | | | | | |
|---|---|---|---|---|---|---|
| SA= sebelipase alfa; PBO=placebo. Subjects in the PBO/SA arm had no exposure to SA at Week 20 and 30 weeks of exposure at Week 52 | | | | | | |

### 6. Portal Inflammation

**Portal inflammation is scored from 0 to 4; scores are presented by timepoint (Baseline, Week 20, and Week 52) in Table 19 for subjects randomized to SA and in Table 20 for subjects randomized to PBO. Portal inflammation was present in almost all subjects at Baseline (scores of 1 or 2, mild or moderate, for 30 of 31 subjects). By Week 20, 2 subjects (both in the PBO group) had progressed to scores of 3 (moderate/marked portal inflammation); none of the SA subjects had progressed to a score of 3. By Week 52, the majority of subjects remained in the mild or moderate categories for portal inflammation.**

**Table 19: Portal Inflammation: Liver Biopsy Data by Timepoint for Subjects Randomized to SA) (H&E Stain)**

| **Portal Inflammation** | **Score** | **Baseline n (%)** | **Week 20 n (%)** | **Week 52 n (%)** |
|---|---|---|---|---|
| Subjects with liver biopsy data | | 18 (95) | 16 (84) | 13 (68) |
| None | 0 | 0 | 1 (5) | 1 (5) |
| Mild, some or all portal areas | 1 | 12 (63) | 6 (32) | 4 (21) |
| Moderate, some or all portal areas | 2 | 6 (32) | 9 (47) | 8 (42) |
| Moderate/marked, all portal areas | 3 | 0 | 0 | 0 |
| Marked, all portal areas | 4 | 0 | 0 | 0 |

| | | | | |
|---|---|---|---|---|
| SA= sebelipase alfa. Subjects in the SA/SA arm had 20 weeks of exposure to SA at Week 20 and 52 weeks of exposure at Week 52. | | | | |

**Table 20: Portal Inflammation: Liver Biopsy Data by Timepoint for Subjects Randomized to PBO (H&E Stain)**

| **Portal Inflammation** | **Score** | **Baseline n (%)** | **Week 20 n (%)** | **Week 52 n (%)** |
|---|---|---|---|---|
| Subjects with liver biopsy data | | 13 (100) | 10 (77) | 8 (62) |
| None | 0 | 1 (8) | 0 | 0 |
| Mild, some or all portal areas | 1 | 7 (54) | 5 (38) | 1 (8) |
| Moderate, some or all portal areas | 2 | 5 (38) | 3 (23) | 6 (46) |
| Moderate/marked, all portal areas | 3 | 0 | 2 (15) | 1 (8) |
| Marked, all portal areas | 4 | 0 | 0 | 0 |

| | | | | |
|---|---|---|---|---|
| SA= sebelipase alfa; PBO=placebo. Subjects in the PBO/SA arm had no exposure to SA at Week 20 and 30 weeks of exposure at Week 52. | | | | |

### 7. Lobular Inflammation

Lobular inflammation is scored from 0 to 4. Scores are presented by timepoint (Baseline, Week 20, and Week 52) in Table 21 for subjects randomized to SA and in Table 22 for subjects randomized to PBO. Lobular inflammation was present in all subjects at Baseline. The trend was for a decrease over time in lobular inflammation following treatment with SA. By Week 52, 2 subjects (both in the SA/SA group) had improved to no lobular inflammation (score of 0).

**Table 21: Lobular Inflammation: Liver Biopsy Data by Timepoint for Subjects Randomized to SA (H&E Stain)**

| **Lobular Inflammation** | **Score** | **Baseline n (%)** | **Week 20 n (%)** | **Week 52 n (%)** |
|---|---|---|---|---|
| Subjects with liver biopsy data | | 18 (95) | 16 (84) | 13 (68) |
| None | 0 | 0 | 1 (5) | 2 (11) |
| One focus or less per 10X objective | 1 | 10 (53) | 9 (47) | 8 (42) |
| Two to four foci per 10X objective | 2 | 8 (42) | 6 (32) | 3 (16) |
| Five to ten foci per 10X objective | 3 | 0 | 0 | 0 |
| More than ten foci per 10X objective | 4 | 0 | 0 | 0 |

| | | | | |
|---|---|---|---|---|
| SA= sebelipase alfa. Subjects in the SA/SA arm had 20 weeks of exposure to SA at Week 20 and 52 weeks of exposure at Week 52. | | | | |

**Table 22: Lobular Inflammation: Liver Biopsy Data by Timepoint for Subjects Randomized to PBO (H&E Stain)**

| **Lobular Inflammation** | **Score** | **Baseline n (%)** | **Week 20 n (%)** | **Week 52 n (%)** |
|---|---|---|---|---|
| Subjects with liver biopsy data | | 13 (100) | 10 (77) | 8 (62) |
| None | 0 | 0 | 0 | 0 |
| One focus or less per 10X objective | 1 | 8 (62) | 7 (54) | 6 (46) |
| Two to four foci per 10X objective | 2 | 5 (38) | 3 (23) | 2 (15) |
| Five to ten foci per 10X objective | 3 | 0 | 0 | 0 |
| More than ten foci per 10X objective | 4 | 0 | 0 | 0 |

| | | | | |
|---|---|---|---|---|
| SA= sebelipase alfa; PBO=placebo. Subjects in the PBO/SA arm had no exposure to SA at Week 20 and 30 weeks of exposure at Week 52. | | | | |

### 8. Macrovesicular Steatosis

Macrovesicular steatosis is scored from 0 to 4. Scores are presented by timepoint (Baseline, Week 20, and Week 52) in Table 23 for subjects randomized to SA and in Table 24 for subjects randomized to PBO. The majority of subjects (27 of 31) had no macrovesicular steatosis at Baseline, which was an anticipated finding based on the documented pathology of the underlying disease. At Week 52, the majority of subjects (15 of 21) had no macrovesicular steatosis.

**Table 23: Macrovesicular Steatosis: Liver Biopsy Data by Timepoint for Subjects Randomized to SA (H&E Stain)**

| **Macrovesicular Steatosis** | **Score** | **Baseline n (%)** | **Week 20 n (%)** | **Week 52 n (%)** |
|---|---|---|---|---|
| Subjects with liver biopsy data | | 18 (95) | 16 (84) | 13 (68) |
| None | 0 | 16 (84) | 15 (79) | 9 (47) |
| Fat vacuoles replacing <5% of hepatocyte area | 1 | 1 (5) | 1 (5) | 3 (16) |
| Fat vacuoles replacing 5-33% of hepatocyte area | 2 | 1 (5) | 0 | 1 (5) |
| Fat vacuoles replacing 33-66% of hepatocyte area | 3 | 0 | 0 | 0 |
| Fat vacuoles replacing >66% of hepatocyte area | 4 | 0 | 0 | 0 |

| | | | | |
|---|---|---|---|---|
| SA= sebelipase alfa. Subjects in the SA/SA arm had 20 weeks of exposure to SA at Week 20 and 52 weeks of exposure at Week 52. | | | | |

**Table 24: Macrovesicular Steatosis: Liver Biopsy Data by Timepoint for Subjects Randomized to PBO (H&E Stain)**

| **Macrovesicular Steatosis** | **Score** | **Baseline n (%)** | **Week 20 n (%)** | **Week 52 n (%)** |
|---|---|---|---|---|
| Subjects with liver biopsy data | | 13 (100) | 10 (77) | 8 (62) |
| None | 0 | 11 (85) | 9 (69) | 6 (46) |
| Fat vacuoles replacing <5% of hepatocyte area | 1 | 2 (15) | 1 (8) | 2 (15) |
| Fat vacuoles replacing 5-33% of hepatocyte area | 2 | 0 | 0 | 0 |
| Fat vacuoles replacing 33-66% of hepatocyte area | 3 | 0 | 0 | 0 |
| Fat vacuoles replacing >66% of hepatocyte area | 4 | 0 | 0 | 0 |

| | | | | |
|---|---|---|---|---|
| SA= sebelipase alfa; PBO=placebo. Subjects in the PBO/SA arm had no exposure to SA at Week 20 and 30 weeks of exposure at Week 52. | | | | |

### 9. Microvesicular Steatosis

Microvesicular steatosis is scored from 0 to 4. Scores are presented by timepoint (Baseline, Week 20, and Week 52) in Table 25 for subjects randomized to SA and in Table 26 for subjects randomized to PBO. The majority of subjects had microvesicular steatosis at Baseline (30 of 31 subjects), as expected with the underlying disease. Furthermore, most (27 of 31 subjects) had >66% hepatocyte involvement/replacement, demonstrating and underlying the severity of disease. By Week 52, only 12 of 21 subjects had >66% hepatocyte involvement/replacement, reflecting the effect of SA on fat reduction in hepatocytes.

**Table 25: Microvesicular Steatosis: Liver Biopsy Data by Timepoint for Subjects Randomized to SA (H&E Stain)**

| **Microvesicular Steatosis** | **Score** | **Baseline n (%)** | **Week 20 n (%)** | **Week 52 n (%)** |
|---|---|---|---|---|
| Subjects with liver biopsy data | | 18 (95) | 16 (84) | 13 (68) |
| None | 0 | 0 | 0 | 0 |
| Fat vacuoles replacing <5% of hepatocyte area | 1 | 0 | 0 | 2 (11) |
| Fat vacuoles replacing 5-33% of hepatocyte area | 2 | 1 (5) | 0 | 2 (11) |
| Fat vacuoles replacing 33-66% of hepatocyte area | 3 | 0 | 4 (21) | 2 (11) |
| Fat vacuoles replacing >66% of hepatocyte area | 4 | 17 (89) | 12 (63) | 7 (37) |

| | | | | |
|---|---|---|---|---|
| SA= sebelipase alfa. Subjects in the SA/SA arm had 20 weeks of exposure to SA at Week 20 and 52 weeks of exposure at Week 52. | | | | |

**Table 26: Microvesicular Steatosis: Liver Biopsy Data by Timepoint for Subjects Randomized to PBO (H&E Stain)**

| **Microvesicular Steatosis** | **Score** | **Baseline n (%)** | **Week 20 n (%)** | **Week 52 n (%)** |
|---|---|---|---|---|
| Subjects with liver biopsy data | | 13 (100) | 10 (77) | 8 (62) |
| None | 0 | 1 (8) | 0 | 0 |
| Fat vacuoles replacing <5% of hepatocyte area | 1 | 1 (8) | 1 (8) | 1 (8) |
| Fat vacuoles replacing 5-33% of hepatocyte area | 2 | 1 (8) | 1 (8) | 1 (8) |
| Fat vacuoles replacing 33-66% of hepatocyte area | 3 | 0 | 2 (15) | 1 (8) |
| Fat vacuoles replacing >66% of hepatocyte area | 4 | 10 (77) | 6 (46) | 5 (38) |

| | | | | |
|---|---|---|---|---|
| SA= sebelipase alfa; PBO=placebo. Subjects in the PBO/SA arm had no exposure to SA at Week 20 and 30 weeks of exposure at Week 52. | | | | |

### C. Discussion and Conclusions

Liver biopsy is the accepted standard for histologic assessment of liver disease activity and fibrosis, despite such limitations as sampling variability, potential complications of an invasive technique, and subjective scoring.

Liver biopsies were obtained in 18 of 19 adult subjects (≥ 18 years of age) and in 15 of 47 pediatric subjects. Of these 33 subjects who had at least 1 liver biopsy, 30 subjects had both valid Baseline data and at least 1 valid post-dose liver biopsy (at Week 20 and/or Week 52). Sampling variability is a limitation of the study, because of the small numbers of subjects with paired liver biopsy data.

An independent pathologist at a central facility who was blinded to assessment time point and treatment assignment during the double-blind treatment period evaluated all biopsies semiquantitatively for histologic features such as Ishak Stage, portal inflammation, lobular inflammation, macrovesicular steatosis, and microvesicular steatosis. Computer-assisted morphometry was used to quantify percent steatosis, collagen, fibrosis, and macrophages.

A quantitative morphometry-based assessment of liver histopathology was utilized for percent steatosis. At Baseline, the morphometric scores for percent steatosis were comparable between the 2 treatment groups (29.7% in the SA group versus 28.4% in the PBO group). When responders were defined as subjects who improved or were unchanged from Baseline, at Week 20 there were significantly more subjects randomized to the SA arm who responded (15/16 subjects, 94%) compared with subjects randomized to the PBO arm (5/10 subjects, 50%; p=0.0184). When the median percent change in steatosis from Baseline to Week 20 was analyzed, the trend was for subjects treated with SA to show more improvement in percentage steatosis compared with subjects who received PBO, although the differences did not reach statistical significance (p=0.061). When considering subjects with paired liver biopsy data at Baseline and Week 52, the trend for improvement from Baseline in percent steatosis in subjects treated with SA continued through Week 52. This is apparent for both subjects treated with SA for 52 weeks (mean change from Baseline was 11.2%) and for 30 weeks (mean change from Baseline was 3.1%), with an apparent larger mean improvement in subjects treated for 52 weeks, suggesting a relationship between benefit and duration of treatment.

All 31 subjects with Baseline biopsies and at least 1 valid post-dose biopsy had some fibrosis at Baseline. Of the 20 subjects with paired data at Baseline and Week 52, 10 subjects had Ishak scores of 5 or 6 at Baseline, indicative of early or established cirrhosis. At Week 52, 18 of 20 subjects (90%) with paired liver biopsy data had Ishak stage scores that had improved or did not progress. In addition to improvement in liver markers and lipid parameters, long-term treatment with 52 weeks of SA showed the 92% of patients (11 of 12) had improved or stable Ishak fibrosis stage, with 67% having at least a 1-stage improvement (8 of 12) and 50% (6 of 12) having a 2 point reduction in Ishak stage scores from Baseline. All 6 subjects had 52 weeks of SA exposure, emphasizing greater benefit with longer exposure to SA. Five of the 6 subjects with a≥2 point reduction in Ishak stage scores from Baseline to Week 52 had an Ishak stage of 3 at Baseline, suggesting that when treatment is started before fibrosis is well established, there is a greater treatment benefit.

For the histologic features of collagen, fibrosis, macrophages, portal inflammation, and lobular inflammation, all subjects showed improvement over Baseline at Week 52, with subjects treated with SA for 52 weeks (those randomized to SA) showing more improvement than subjects randomized to PBO (and thus treated with SA for 30 weeks).

As expected based on the pathology of the underlying disease, few (5/20) subjects with paired liver biopsy data had any macrovesicular steatosis at Baseline, but most (19/20) subjects did have microvesicular steatosis. As with the other histologic parameters, microvesicular steatosis improved with SA treatment, with more improvement seen in subjects treated for 52 weeks than for 30 weeks.

The overall trend is supportive of improvement or halting of progression of liver damage in subjects treated with SA compared to Baseline. The results from subjects who were randomized to SA and who have biopsy data at all 3 time points (Baseline, 20 weeks, and 52 weeks) suggest increased improvement with increased time on SA. Similarly, when subjects were compared at Week 52, those treated with SA for 52 weeks (subjects randomized to SA) showed more improvement than those treated for 30 weeks (subjects randomized to PBO).

### Example 2: Further Interim Results from ARISE Trial

The following is a summary of interim data from the ongoing ARISE trial, which was conducted substantially according to the protocol described above and supplements the data in Example 1. The objective of this analysis was to evaluate the ongoing, open-label phase of the ARISE study for key clinical study parameters through 76 weeks of treatment with sebelipase alfa (SA).

Patients initially randomized to the SA group received SA for 76 weeks (study weeks 0-76). Patients initially randomized to the placebo group, who then entered the open-label period, received SA for 78 weeks (study weeks 22-100). Aggregate data are presented as 76 weeks of SA treatment.

The most common treatment-emergent adverse events (≥10% incidence) reported in the open-label extension period are set forth below in Table 27. 3951 infusions at 1 mg/kg and 248 infusions at 3 mg/kg were administered. Most adverse events (AEs) were mild or moderate in intensity. No patient discontinued due to AEs in the open-label period. Infusion-associated reactions (IARs) during the open-label extension period occurred in 13 patients (20%); all but 1 were mild or moderate in intensity. 7 patients had serious AEs. Of these, 1 was an IAR considered related to treatment. The patient stopped SA treatment for 86 weeks, underwent a desensitization protocol, and then restarted therapy. Anti-drug antibodies (ADAs) were detected in 7 patients (11%). Of these 7, 2 patients had neutralizing antibodies. The safety profile for patients who tested positive for ADAs was consistent with that of the overall study population.

**Table 27: Most Common Treatment-Emergent Adverse Events**

| **Adverse Event, n (%)** | **All Patients in Open-Label Period (N=65), n (%)** |
|---|---|
| Any AE during open-label period | 64 (98) |
| Nasopharyngitis | 29 (45) |
| Headache | 28 (43) |
| Pyrexia | 21 (32) |
| Cough | 20 (31) |
| Abdominal pain | 15 (23) |
| Vomiting | 15 (23) |
| Diarrhea | 14 (22) |
| Upper respiratory tract infection | 14 (22) |
| Abdominal pain, upper | 12 (18) |
| Gastroenteritis | 11 (17) |
| Rhinitis | 11 (17) |
| Rhinorrhea | 11 (17) |
| Oropharyngeal pain | 9 (14) |
| Epistaxis | 8 (12) |
| Dizziness | 7 (11) |
| Ligament sprain | 7 (11) |
| Nausea | 7 (11) |
| Respiratory tract infection | 7 (11) |
| Vitamin D deficiency | 7 (11) |

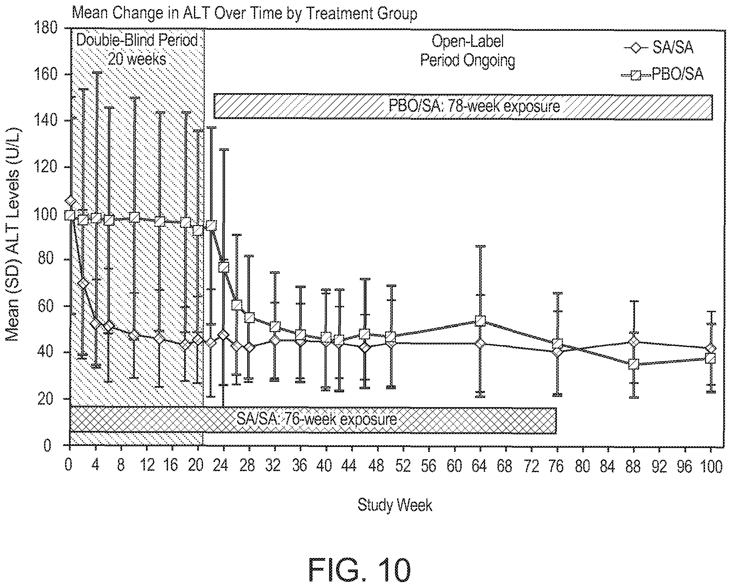

As shown in Figure 10, mean ALT levels decreased from 99.6 U/L at baseline to 39.6 U/L with 76 weeks of SA treatment. This represents a mean percentage change of -56.1%. Specifically, 87% of patients reached ALT ≤1.5 × ULN. At 76 weeks, 51% (31/61) had achieved ALT normalization.

In addition, mean serum AST levels decreased from 79.8 U/L at baseline to 36.3 U/L with 76 weeks of SA treatment, representing a mean percentage change of -50.7%. Specifically, 95% of patients reached AST ≤1.5 x ULN. At 76 weeks, 65% (37/57) of patients had achieved AST normalization with SA treatment.

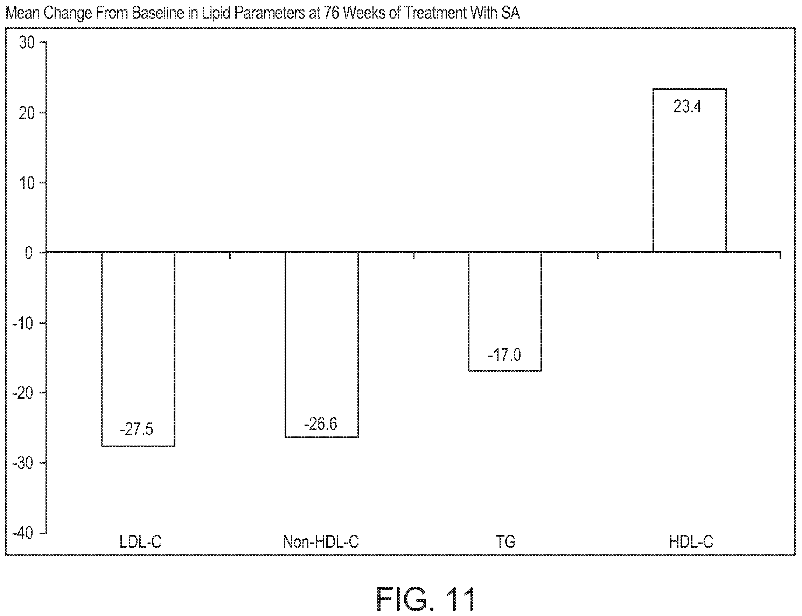

Figure 11 depicts the mean change from baseline in lipid parameters at 76 week of treatment with SA. As shown in Figure 11, mean LDL-C levels improved from 199 to 142 mg/dL. Mean non-HDL-C levels improved from 230 to 166 mg/dL. Mean TG levels improved from 155 to 123 mg/dL. Mean HDL-C level improved from 33 to 40 mg/dL.

Multi-echo gradient echo magnetic resonance imaging was done at baseline, study week 20, and study week 52 (representing 52 weeks of SA treatment in the SA/SA arm and 30 weeks of treatment in the PBO/SA arm), to assess hepatic fat fraction and liver volume. With 52 weeks of SA treatment, 88% of patients (28/32) had reduction in hepatic fat fraction (mean reduction -21%, n=32). 90% of patients (28/31) had a reduction in liver volume (mean reduction -13%, n=31). With 30 weeks of SA treatment, 88% of patients (21/24) had a reduction in hepatic fat fraction (mean reduction -28%, n=24). 96% of patients (25/26) had a reduction in liver volume (mean reduction -11%, n=26).

In sum, sustained improvements were observed in markers of liver injury, lipid abnormalities, liver volume, and hepatic fat content through 76 weeks of treatment with SA, highlighting the benefit of long-term therapy. Moreover, ongoing treatment with SA through 76 weeks was generally well tolerated by patients with LAL-D. Specifically, the long-term safety profile was similar to that during the double-blind period and most adverse events were mild to moderate in intensity.

### Example 3: Interim Results from Trial in Pediatric Patients

The following is a summary of a pediatric study (LAL-CL03 Study; clinicaltrials.gov/ct2/show/NCT01358370?term=LAL-1&rank=1),which was conducted substantially according to the protocol described above and supplements the data in Examples 1 and 2. The objective of this analysis was to evaluate the effect of SA on survival to 3 years of age and liver function in infants with rapidly progressive lysosomal acid lipase deficiency (LAL-D).

For this study, pediatric patients with LAL-D (i.e., infants through 3 years of age), received a starting dose of 0.35 mg/kg SA weekly, with escalation up to 1 mg/kg. Further escalation to 3.0 or 5.0 mg/kg was permitted based on protocol defined criteria. Key inclusion criteria included: (1) <8 months of age at the age of anticipated first infusion, (2) lab confirmed diagnosis of LAL-D, and (3) demonstrated growth failure or other evidence of rapidly progressive disease with onset before 6 months of age. Survival was the primary efficacy measure, but improvements in weight and functional development, hematological effects, and safety/tolerability over 3 years were also assessed.

Nine patients were enrolled over 31 months. The patient demographics are set forth below in Table 28. Eight of the nine had growth failure. All nine had diarrhea or vomiting, adrenal calcification, hepatomegaly and/or splenomegaly. All nine required a specialized diet (*e.g*., Monogen®) with three of the nine receiving a reduced fat diet and two of the nine requiring parenteral nutrition.

**Table 28: Pediatric Patient Demographics**

| **Parameter** | **Patients (N=9)** |
|---|---|
| Age at treatment initiation, months, median (range) | 3.0 (1.1-5.8) |
| Male, n (%) | 5 (56) |
| White, n (%) | 4 (44) |
| Growth failure/entry criteria met, n (%) | |
| Weight decreasing across ≥2 of the 11 major centiles | 7 (78) |
| Body weight <10^{th} centile and no weight increase during 2 weeks before screening | 1 (11) |
| Loss of >5% of birth weight after 2 weeks of age | 0 |
| Rapidly progressive course of LAL-D without meeting growth failure criteria | 1 (11) |

No patients have discontinued from the study to date. 1013 infusions have been administered. One patient (patient D) completed the study in May 2016 and continues to receive SA 3mg/kg every other week.

43 serious adverse events (SAE) occurred across all nine patients. Over the 1 year prior to this assessment there were two unrelated SAEs (croup and malabsorption [hypoalbuminemia] in two different patients) and one infusion-associated reaction (IAR) that was mild and resulted in no modification to therapy. The vast majority (95%) of adverse events (AE) were mild/moderate.

There were 54 total IARs across five patients, including four events also reported as SAE. 48 of the IARs were listed as related or possibly related. 51 of the IARs were mild or moderate (e.g. fever or vomiting). 3 severe IARs occurred in the same patient. All reactions were successfully managed and resolved with routine medical practice

Anti-drug antibody (ADA) positive results were obtained from 4/7 patients tested. Two of the positive patients have been consistently positive for ADA and neutralizing antibodies including most recent time tested. The other two patients have been less frequently ADA positive and only one with neutralizing antibodies. All ADA positive patients have experienced IAR without impacting treatment and with inconsistent temporal relationship to the presence of antibodies.

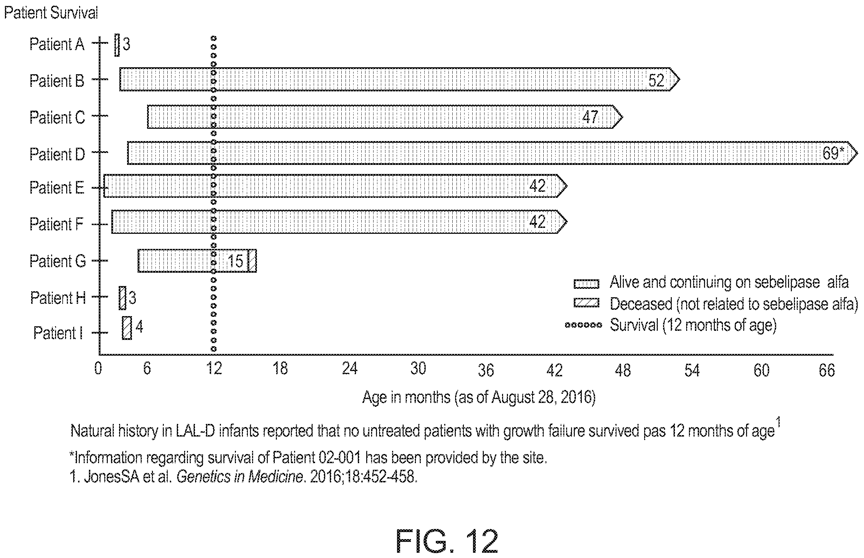

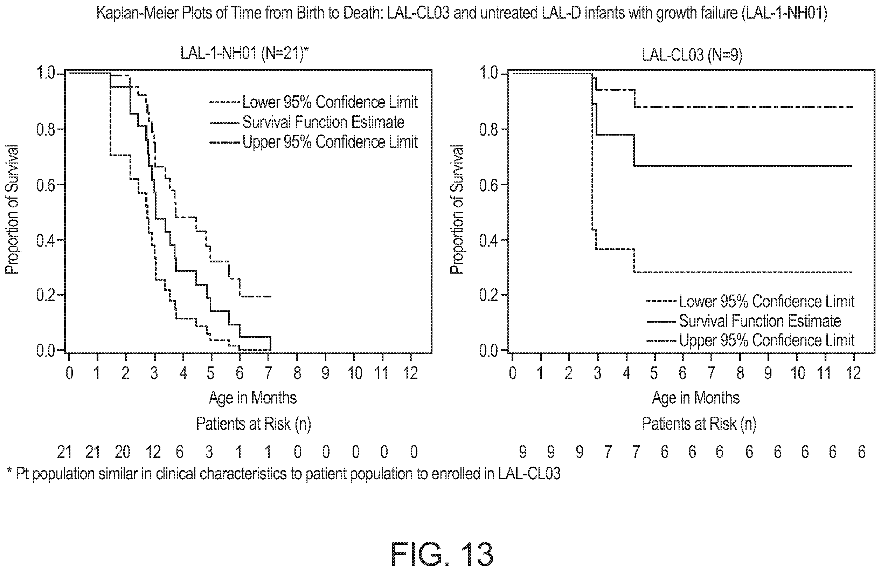

The survival statistics are set forth in Figure 12. Five patients have survived beyond 3 years of age as of Aug 28, 2016. The median age (range): 3 years, 11 months (3 years, 6 months to 5 years, and 9 months). The median time in the study was 3 year, 5 months. The oldest patient has been receiving SA for 5 years and 5 months. Four deaths were unrelated or likely unrelated to SA. Three patients died after receiving ≤4 doses of SA (one from cardiac arrest and one from massive intraperitoneal bleeding). One other patient died from liver failure as a result of LAL-D. One patient completed the study, but remains on treatment. Figure 13 sets forth the Kaplan-Meier plots of time from birth to death (LAL-CL03 Study and untreated LAL-D infants with growth failure (LAL--NH01 Study; clinicaltrials.gov/ct2/show/NCT01371825?term=LAL-CL03&rank=1).

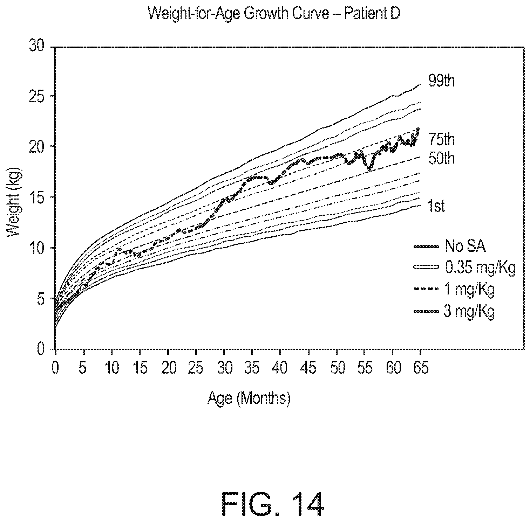

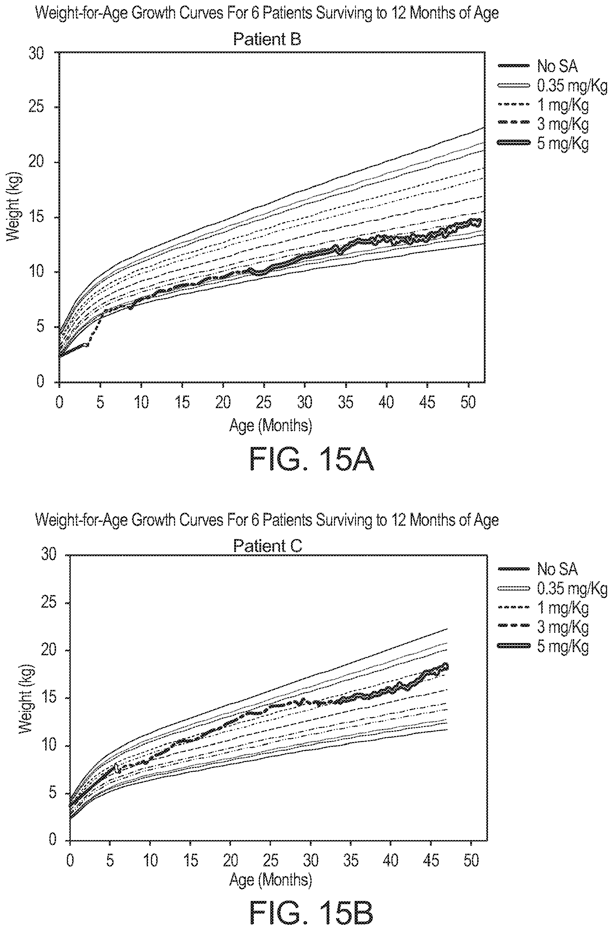

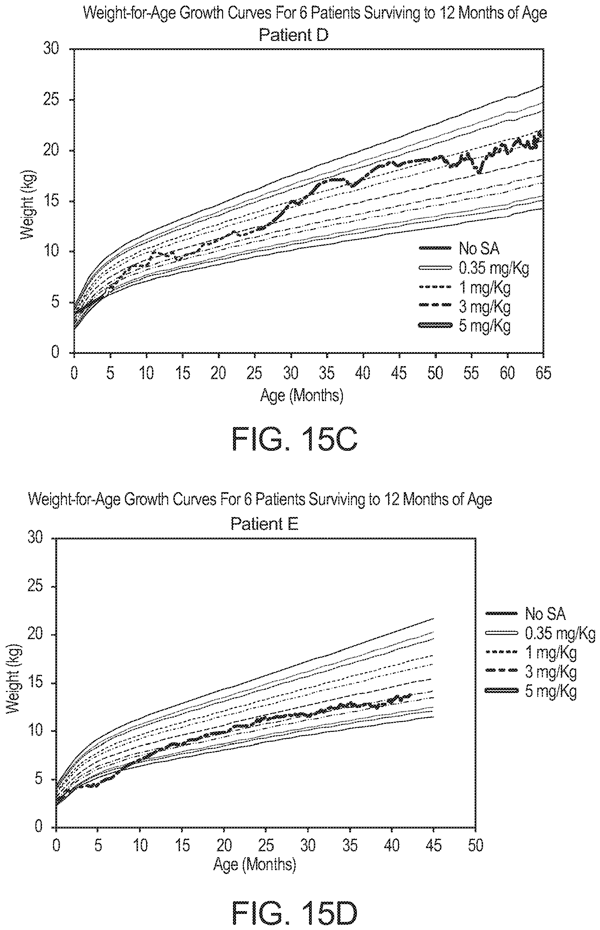

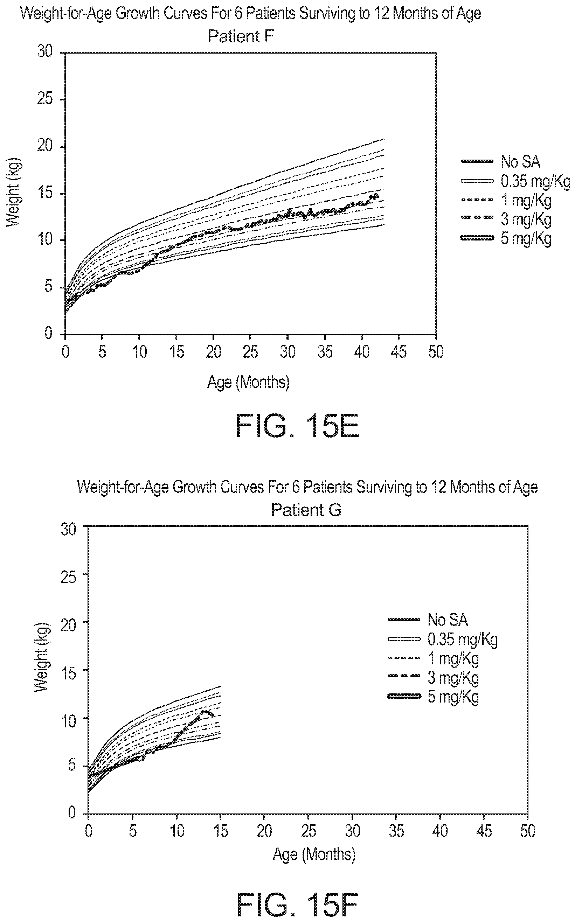

The weight-for-age growth curve for patient D is set forth in Figure 14. The weight-for-age growth curve for six patients surviving to 12 months of age (patients B, C, D, E, F, and G) is set forth in Figure 15.

The dosing status and dose escalation scheme is set forth below in Table 29.

**Table 29: Dosing Status and Dose Escalation**

| **Patient** | **Week of 1st infusion of 3mg/kg** | **Current Dose per kg** | **Anti-Drug Ab** | **Reason for Escalation to 3mg/kg** | **Current Status** |
|---|---|---|---|---|---|
| B | 23 | 5mg QW¹ | Enz & CU | WFA plateaued + hepatomegaly | WFA Improved, but remains low; liver function normal, palpable |
| C | 14 | 5mg QW² | CU | WFA plateaued persistently high transaminases and low albumin | WFA ∼85%, liver function normal, low albumin |
| D | 91 | 3mg QW | Y, No NAb | Mesenteric lymphadenopathy | WFA >75%, liver function normal |
| E | 12 | 3mg QW | No | Poor wt gain + LFA < -2 z score | WFA ∼25%, liver function normal |
| F | 6 | 3mg QW | Enz & CU | Poor wt gain + LFA < -2 z score | WFA ∼25%, liver function normal |
| G | 10 | Deceased | No | Poor wt gain + LFA < -2 z score | Died at 1.25 years |

| | | | | | |
|---|---|---|---|---|---|
| ¹Escalated to 5mg/kg qw at Week 88 due to poor weight gain; ²Escalated to 5mg/kg qw at Week 122 after a period of 3mg/kg qw. Y=Positive Anti-Drug Antibody prior to escalation; NAb=Neutralizing Antibody; Enz=Neutralizing antibody to enzyme; CU=Neutralizing antibody to cell uptake inhibition. | | | | | |

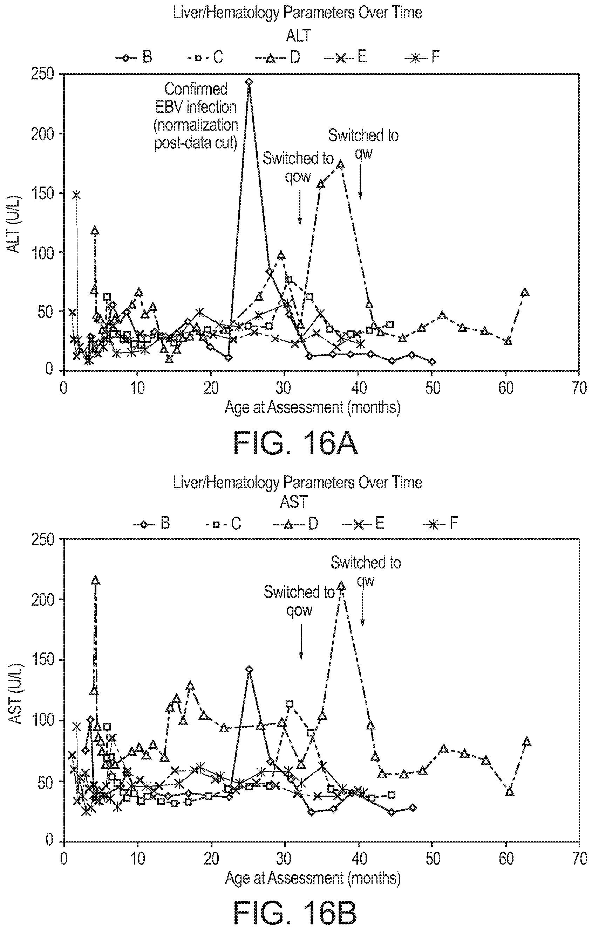

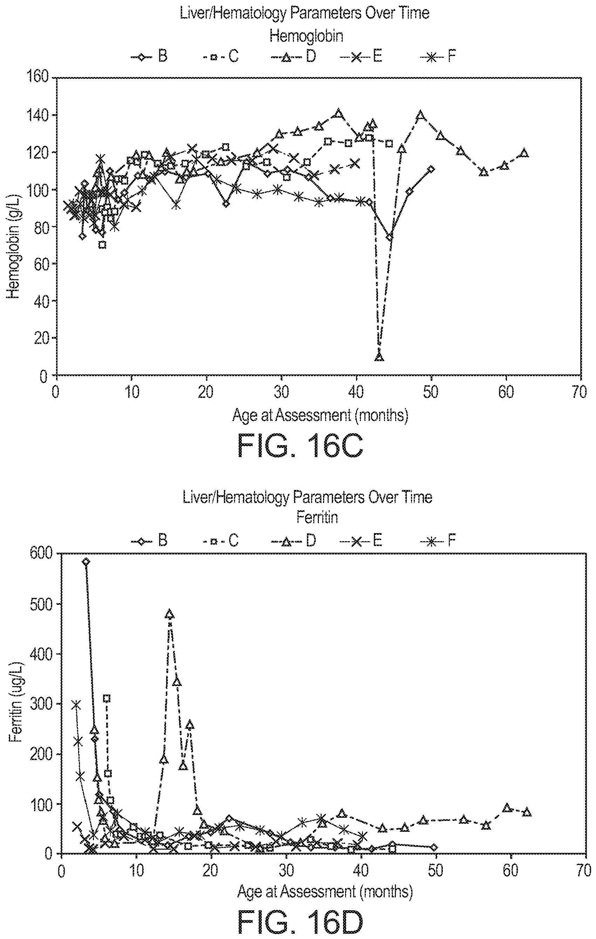

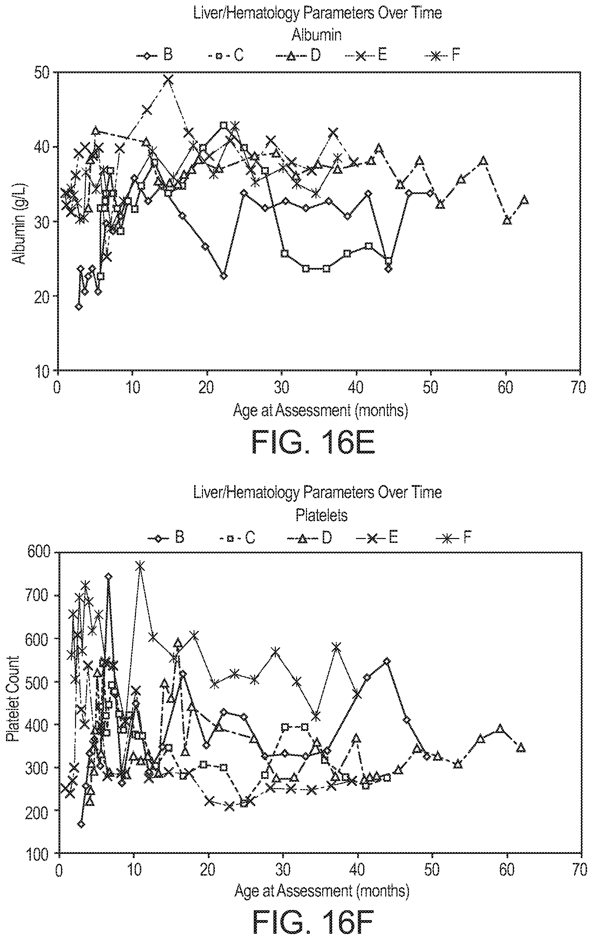

The weight, liver and hematological effects are set forth below in Table 30. The liver and hematology parameters (ALT, AST, hemoglobin, ferritin, albumin, and platelets) over time are set forth in Figure 16. The median weight percentile was 3.1% at baseline and increased to 37.0% at the most recent visit. The median length percentile was 1.8% percentile and increased to 30.6% at the most recent visit.

**Table 30: Dosing Status and Dose Escalation**

| **Parameter** | **B** | **C** | **D** | **E** | **F** |
|---|---|---|---|---|---|
| | Baseline value, last value (% change from baseline to last measurement) | | | | |
| ALT, U/L | 16, 8 **(-50%)** | 35, 28 **(-20%)** | 68, 67**(-1%)** | 50, 31 (**-38**%) | 149, 23 **(-85%)** |
| AST, U/L | 75, 27 **(-64%)** | 94, 27 **(-71%)** | 125, 83 **(-34%)** | 71, 42 **(-41%)** | 94, 39 (**-59**%) |
| Hemoglobin, g/L | 77, 112 **(45%)** | 72, 126 **(75%)** | 94, 121 **(29%)** | 93, 115 (**24%)** | 95, 95 **(No change**) |
| Albumin, g/L | 19, 34 **(79%)** | 23, 26 **(13%)** | 32, 33 **(3%)** | 34, 38 **(11%)** | 34, 39 **(13%)** |

In conclusion, 5 patients have survived to beyond 3 years of age. The median survival of infants in the natural history study was 3.7 months. All surviving patients required dose escalation to ≥ 3.0 mg/kg weekly. Improvements in weight gain, gastrointestinal symptoms, hepatosplenomegaly, ALT, AST, and hemoglobin have been sustained over time. The majority of patients have demonstrated normal development. SA was well tolerated.

## Claims

1. Sebelipase alfa for use in a method of reducing liver fibrosis in a human patient with a lysosomal acid lipase (LAL) deficiency, comprising administering sebelipase alfa to the patient, wherein the patient has been determined to have Ishak fibrosis stage of 1 or greater prior to administration, and wherein at least a one point reduction in Ishak fibrosis stage after administration compared to a baseline Ishak fibrosis stage obtained from the patient prior to administration is indicative of reduced liver fibrosis.

2. The sebelipase alfa for use according to claim 1, wherein the at least one point reduction occurs on or by week 20.

3. The sebelipase alfa for use according to claim 1 or claim 2, wherein the at least one point reduction occurs on or by week 30.

4. The sebelipase alfa for use according to any one of the preceding claims, wherein the patient has a ≥ 2 point reduction in Ishak fibrosis stage after administration compared to a baseline Ishak fibrosis stage obtained from the patient prior to administration, and the ≥ 2 point reduction occurs on or by week 52.

5. The sebelipase alfa for use according to any one of the preceding claims, wherein the Ishak fibrosis stage is assessed via liver biopsy.

6. The sebelipase alfa for use according to any one of the preceding claims, wherein sebelipase alfa is administered as an intravenous infusion, optionally wherein sebelipase alfa is infused over at least two hours.

7. The sebelipase alfa for use according to any one of the preceding claims, wherein sebelipase alfa is administered to the patient at a dose of 1 mg/kg once every other week.

8. The sebelipase alfa for use according to claim 7, wherein sebelipase alfa is administered at a total infusion volume of:
a) 10 mL for a 1 to 10.9 kg patient;
b) 25 mL for a 11 to 24.9 kg patient;
c) 50 mL for a 25 to 49.9 kg patient;
d) 100 mL for a 50 to 99.9 kg patient; or
e) 250 mL for a 100 to 120.9 kg patient.

9. The sebelipase alfa for use according to any one of claims 1-8, wherein sebelipase alfa is administered to the patient at a dose of 3 mg/kg once weekly.

10. The sebelipase alfa for use according to claim 9, wherein sebelipase alfa is administered at a total infusion volume of:
a) 25 mL for a 1 to 10.9 kg patient;
b) 50 mL for a 11 to 24.9 kg patient;
c) 100 mL for a 25 to 49.9 kg patient;
d) 250 mL for a 50 to 99.9 kg patient; or
e) 500 mL for a 100 to 120.9 kg patient.

11. The sebelipase alfa for use according to any one of the preceding claims, wherein the patient has cirrhosis at baseline.

12. The sebelipase alfa for use according to any one of the preceding claims, wherein the method results in a shift toward normal levels of alanine aminotransferase (ALT), collagen, macrophages, low density lipoprotein cholesterol (LDL-C), and/or liver fat content.

13. The sebelipase alfa for use according to any one of claims 1-12, wherein the method results in reduction of alanine aminotransferase (ALT), low density lipoprotein cholesterol (LDL-C), collagen, portal inflammation, lobular inflammation, macrovesicular steatosis, microvesicular steatosis, macrophages and/or overall liver fat content levels compared to baseline.

14. The sebelipase alfa for use according to claim 13, wherein the method results in reduction of: alanine aminotransferase (ALT) by about 60% or more, low density lipoprotein cholesterol (LDL-C) by about 40% or more, and/or liver fat content by about 30% or more, compared to baseline.

15. The sebelipase alfa for use according to any one of claims 12-14, wherein levels of liver fat content are assessed by magnetic resonance imaging (MRI).

## Patentansprüche

1. Sebelipase alfa zur Verwendung in einem Verfahren zur Verringerung der Leberfibrose bei einem menschlichen Patienten mit einem Mangel an lysosomaler saurer Lipase (Lysosomal Acid Lipase, LAL), umfassend die Verabreichung von Sebelipase alfa an den Patienten, wobei bestimmt wurde, dass der Patient vor der Verabreichung ein Ishak-Fibrosestadium von 1 oder mehr aufweist, und wobei mindestens eine Verringerung des Ishak-Fibrosestadiums nach der Verabreichung um mindestens einen Punkt im Vergleich zu einem vom Patienten vor der Verabreichung erhaltenen Ishak-Fibroseausgangsstadium auf eine verringerte Leberfibrose hinweist.

2. Sebelipase alfa zur Verwendung nach Anspruch 1, wobei die Reduktion um mindestens einen Punkt an oder bis Woche 20 erfolgt.

3. Sebelipase alfa zur Verwendung nach Anspruch 1 oder 2, wobei die Reduktion um mindestens einen Punkt an oder bis Woche 30 erfolgt.

4. Sebelipase alfa zur Verwendung nach einem der vorhergehenden Ansprüche, wobei der Patient nach der Verabreichung eine Verringerung des Ishak-Fibrosestadiums um ≤ 2 Punkte im Vergleich zu einem vom Patienten vor der Verabreichung erhaltenen Ishak-Fibrose-Ausgangsstadium aufweist und die Verringerung um ≤ 2 Punkte in oder bis Woche 52 auftritt.

5. Sebelipase alfa zur Verwendung nach einem der vorhergehenden Ansprüche, wobei das Ishak-Fibrosestadium durch Leberbiopsie bewertet wird.

6. Sebelipase alfa zur Verwendung nach einem der vorhergehenden Ansprüche, wobei Sebelipase alfa als intravenöse Infusion verabreicht wird, optional wobei Sebelipase alfa über mindestens zwei Stunden infundiert wird.

7. Sebelipase alfa zur Verwendung nach einem der vorhergehenden Ansprüche, wobei Sebelipase alfa dem Patienten einmal alle zwei Wochen in einer Dosis von 1 mg/kg verabreicht wird.

8. Sebelipase alfa zur Verwendung nach Anspruch 7, wobei Sebelipase alfa mit einem Gesamtinfusionsvolumen von
a) 10 ml für einen Patienten mit 1 bis 10,9 kg,
b) 25 ml für einen Patienten mit 11 bis 24,9 kg,
c) 50 ml für einen Patienten mit 25 bis 49,9 kg,
d) 100 ml für einen Patienten mit 50 bis 99,9 kg oder
e) 250 ml für einen Patienten mit 100 bis 120,9 kg verabreicht wird.

9. Sebelipase alfa zur Verwendung nach einem der Ansprüche 1 bis 8, wobei Sebelipase alfa dem Patienten einmal wöchentlich in einer Dosis von 3 mg/kg verabreicht wird.

10. Sebelipase alfa zur Verwendung nach Anspruch 9, wobei Sebelipase alfa mit einem Gesamtinfusionsvolumen von
a) 25 ml für einen Patienten mit 1 bis 10,9 kg,
b) 50 ml für einen Patienten mit 11 bis 24,9 kg,
c) 100 ml für einen Patienten mit 25 bis 49,9 kg,
d) 250 ml für einen Patienten mit 50 bis 99,9 kg oder
e) 500 ml für einen Patienten mit 100 bis 120,9 kg verabreicht wird.

11. Sebelipase alfa zur Verwendung nach einem der vorhergehenden Ansprüche, wobei der Patient zu Studienbeginn eine Zirrhose hat.

12. Sebelipase alfa zur Verwendung nach einem der vorhergehenden Ansprüche, wobei das Verfahren zu einer Verschiebung hin zu normalen Spiegeln von Alaninaminotransferase (ALT), Kollagen, Makrophagen, Lipoproteincholesterin niedriger Dichte (Low Density Lipoprotein Cholesterol, LDL-C) und/oder Leberfettgehalt führt.

13. Sebelipase alfa zur Verwendung nach einem der Ansprüche 1 bis 12, wobei das Verfahren zu einer Verringerung der Alaninaminotransferase (ALT), des Lipoproteincholesterins niedriger Dichte (LDL-C), des Kollagens, der Pfortaderentzündung, der lobulären Entzündung, der makrovesikulären Steatose, der mikrovesikulären Steatose, der Makrophagen und/oder des Gesamtfettgehalts im Vergleich zum Studienbeginn führt.

14. Sebelipase alfa zur Verwendung nach Anspruch 13, wobei das Verfahren zu einer Verringerung von Alaninaminotransferase (ALT) um etwa 60% oder mehr, Lipoproteincholesterin niedriger Dichte (LDL-C) um etwa 40% oder mehr und/oder Leberfettgehalt um etwa 30% oder mehr im Vergleich zum Studienbeginn führt.

15. Sebelipase alfa zur Verwendung nach einem der Ansprüche 12 bis 14, wobei der Leberfettgehalt durch Magnetresonanztomographie (MRT) bestimmt wird.

## Revendications

1. Sebelipase alfa à utiliser dans un procédé de réduction de la fibrose hépatique chez un patient humain présentant un déficit en lipase acide lysosomale (LAL), comprenant l'administration de la sébelipase alfa au patient, dans laquelle le patient a été déterminé comme ayant un stade de fibrose d'Ishak de 1 ou plus avant l'administration, et dans laquelle une réduction d'au moins un point du stade de fibrose d'Ishak après l'administration par rapport à un stade de fibrose d'Ishak de base obtenu du patient avant l'administration indique une réduction de la fibrose hépatique.

2. Sebelipase alfa à utiliser selon la revendication 1, dans laquelle la réduction d'au moins un point se produit avant ou à la semaine 20.

3. Sebelipase alfa à utiliser selon la revendication 1 ou la revendication 2, dans laquelle au moins un point de réduction se produit avant ou à la semaine 30.

4. Sebelipase alfa à utiliser selon l'une quelconque des revendications précédentes, dans laquelle le patient présente une réduction de ≥ 2 points du stade de fibrose d'Ishak après administration par rapport à un stade de fibrose d'Ishak de base obtenu du patient avant l'administration, et la réduction de ≥ 2 points se produit avant ou à la semaine 52.

5. Sebelipase alfa à utiliser selon l'une quelconque des revendications précédentes, dans laquelle le stade de fibrose d'Ishak est évalué par biopsie hépatique.

6. Sebelipase alfa à utiliser selon l'une quelconque des revendications précédentes, dans laquelle la sebelipase alfa est administrée sous forme d'une perfusion intraveineuse, éventuellement dans laquelle la sébelipase alfa est perfusée pendant au moins deux heures.

7. Sebelipase alfa à utiliser selon l'une quelconque des revendications précédentes, dans laquelle la sébelipase alfa est administrée au patient à une dose de 1 mg / kg une fois toutes les deux semaines.

8. Sebelipase alfa à utiliser selon la revendication 7, dans laquelle la sebelipase alfa est administrée à un volume de perfusion total de
a) 10 mL pour un patient de 1 à 10,9 kg ;
b) 25 mL pour un patient de 11 à 24,9 kg ;
c) 50 mL pour un patient de 25 à 49,9 kg ;
d) 100 ml pour un patient de 50 à 99,9 kg ; ou
e) 250 mL pour un patient de 100 à 120,9 kg.

9. Sebelipase alfa à utiliser selon l'une quelconque des revendications 1 à 8, dans laquelle la sébelipase alfa est administrée au patient à une dose de 3 mg / kg une fois par semaine.

10. Sebelipase alfa à utiliser selon la revendication 9, dans laquelle la sebelipase alfa est administrée à un volume de perfusion total de :
a) 25 mL pour un patient de 1 à 10,9 kg ;
b) 50 mL pour un patient de 11 à 24,9 kg ;
c) 100 mL pour un patient de 25 à 49,9 kg ;
d) 250 ml pour un patient de 50 à 99,9 kg ; ou
e) 500 mL pour un patient de 100 à 120,9 kg.

11. Sebelipase alfa à utiliser selon l'une quelconque des revendications précédentes, dans laquelle le patient a une cirrhose au départ.

12. Sebelipase alfa à utiliser selon l'une quelconque des revendications précédentes, dans laquelle le procédé entraîne une évolution vers des taux normaux d'alanine aminotransférase (ALT), de collagène, de macrophages, de cholestérol des lipoprotéines de basse densité (LDL-C), et / ou du taux de graisse hépatique.

13. Sebelipase alfa à utiliser selon l'une quelconque des revendications 1 à 12, dans laquelle le procédé conduit à une réduction de l'alanine aminotransférase (ALT), du cholestérol des lipoprotéines de basse densité (LDL-C), du collagène, de l'inflammation portale, de l'inflammation lobulaire, de la stéatose macrovésiculaire, de la stéatose microvésiculaire, des macrophages et / ou du taux de graisse hépatique globale par rapport à la valeur initiale.

14. Sebelipase alfa à utiliser selon la revendication 13, dans laquelle le procédé conduit à une réduction de : l'alanine aminotransférase (ALT) d'environ 60% ou plus, du cholestérol des lipoprotéines de basse densité (LDL-C) d'environ 40% ou plus, et / ou du taux de graisse hépatique d'environ 30% ou plus, par rapport à la valeur initiale.

15. Sebelipase alfa à utiliser selon l'une quelconque des revendications 12 à 14, dans laquelle les niveaux du taux graisse hépatique sont évalués par imagerie par résonance magnétique (IRM).
